# EUROPEAN PATENT SPECIFICATION

(11) **EP 0 345 318 B1**
(45) Date of publication and mention of the grant of the patent: **03.05.1995**
(21) Application number: 88910391.7
(22) Date of filing: 28.10.1988
(51) Int. Cl.: G01N 33/53, C12N 15/00, G01N 33/577, C07K 16/00, C07H 17/00

(54) **HUMAN GAMMA, DELTA T CELL ANTIGEN RECEPTOR POLYPEPTIDES AND NUCLEIC ACIDS**
MENSCHLICHE GAMMA, DELTA-T-ZELLEN-ANTIGEN-REZEPTOR-POLYPEPTIDE UND NUKLEINSÄUREN
POLYPEPTIDES RECEPTEURS D'ANTIGENES DE CELLULES T GAMMA, DELTA HUMAINES ET ACIDES NUCLEIQUES

(30) Priority: 29.10.1987 US 115256; 29.04.1988 US 187698
(43) Date of publication of application: 13.12.1989
(60) Divisional application: 94114134.3
(73) Proprietor: T CELL SCIENCES, INC., Cambridge, MA 02139-4135 (US); DANA-FARBER CANCER INSTITUTE, Boston, MA 02115 (US); THE PRESIDENT AND FELLOWS OF HARVARD COLLEGE, Cambridge, MA 02114 (US)
(72) Inventor: BRENNER, Michael, B., Ashland, MA 01721 (US); IP, Stephen, H., Sudbury, MA 01776 (US); SEIDMAN, Jonathan, Milton, MA 02186 (US); BAND, Hamid, Boston, MA 02215 (US)
(74) Representative: Silveston, Judith
(86) International application number: PCT/US88/03869
(87) International publication number: WO 89/03996

(56) References cited:
- EP-A- 0 200 350
- SCIENCE, vol. 238, 30th October 1987, pages 682-694; H. BAND et al.:"Immunochemical proof that a novel rearranging gene encodes the T cell receptordelta subunit"
- Scinece, vol. 231, 17 Jan. 1986 (Washington, D.C., USA), Quetermous et al, "Human-T-cell Gamma chain Genes, pages 252-255. See page 252, column 1, lines 5-11 and page 255, coumn 2, lines 1-8
- Science, vol. 227, 22 Febr. 1985 (Washington, D.C., USA), Kranz et al, "Chromosomal Locations of the Murine T-Cell Receptor Alpha-Chain Gene and the T-Cell Gamma Gene", pages 941-945. See abstract, and p, 941, coumn 2, lines 4-7 and lines 11-22
- Nature, vol. 316, 1 August 1985 (Tokyo, Japan) Lefrance et al, "Two tandemly organized human genes encoding the T-cell Gamma constant region sequences show multiple rearragement in different T-cell Types", pages 464-466. See Abstract
- Nature, vol. 316, 8 August 1985 (Tokyo, Japan), Murre et al, "Human Gamma-chain genes are rearranged in leukaemic T cells and map to the short arm of chromosome 7", pages 549-552. See page 549, lines 5-11
- Proc. Natl. Acad. Sci. USA, vol. 83, April
- J. Exp. Med., vol. 160, Sept. 1984 (The Rockefeller University Press), Royer et al. "Functional Isotypes Are not encoded by the constant region genes of the Beta Subunit of the T-cell receptor for antigen/major Histocompatibility complex, pages 5947-952.See page 947, lines 18-27 and page 948, lines 1-12.
- Proc. Natl. Acad. Sci. USA, vol. 78, No. 6., June 1981 (Washington D.C., USA), Hopp et al, "Prediction of protein antigenic determinants from amino acid sequences", pages 3824-3828. See Abstract and page 3824, column 1, lines 16-28, page 3825, column 2, lines 32-36, page 3827, column 2, lines 34-39.
- J.Mol. Biol. vol. 157, 1982 (Academic Press Inc. (London) Ltd.), Kyte et al, "A Simple Method for Displaying the Hydropathic Character of a Protein", pages 105-132. See Abstract.

## Description

### 1. INTRODUCTION

The present invention is directed to a form of the human y T cell antigen receptor polypeptide termed Form 2bc, which has a molecular weight of about 40,000 daltons, and a constant region which contains a sequence encoded by only two Cy2 CII exon copies. The invention also relates to T cell antigen receptor heterodimers comprising y Form 2bc, and to nucleic acid sequences encoding y Form 2bc and portions thereof. The invention also provides monoclonal antibodies specifically reactive with an epitope of the y or δ T cell antigen receptor polypeptides.

### 2. BACKGROUND OF THE INVENTION

The T cell antigen receptor (TCR) was shown to be a clone specific disulfide-linked heterodimeron T cells, composed of two glycosylated subunits, one of which is designated the a chain and the other of which is designated the β chain. The a and βTCR subunits have a relative molecular mass (Mᵣ) of approximately 50,000 and 40,000 daltons, respectively (Allison et al., 1982, Immunol.129:2293-2300; Meuer et al., 1983, J. Exp. Med. 157:705-719; Haskins et al., 1983, J. Exp. Med. 157:1149-1169). Genes that rearrange during T cell ontogeny and encode the βTCR (Yanagi et al., 1984, Nature 308:145-149; Hedrick et al., 1984, Nature 308:153-158) and aTCR (Chien et al., 1984, Nature 312:31-35; Saito et al., 1984, Nature 312:36-40, Sim et al., 1984, Nature 312:771-775) subunits were isolated either by subtractive hybridization or by probing with oligonucleotides.

The alpha and beta chains of the T cell antigen receptor of a T cell clone are each composed of a unique combination of domains designated variable (V), diversity (D), joining (J), and constant (C) (Siu et al., 1984, Cell 37:393; Yanagi et al., 1985, Proc. Natl. Acad. Sci. USA 82: 3430). Hypervariable regions have been identified (Patten et al., 1984, Nature 312:40; Becker et al., 1985, Nature 317:430). In each T cell clone, the combination of V, D and J domains of both the alpha and the beta chains participates in antigen recognition in a manner which is uniquely characteristic of that T cell clone and defines a unique binding site, also known as the idiotype of the T cell clone. In contrast, the C domain does not participate in antigen binding.

A unique feature of the human α,βTCR was the observed comodulation (Meuer et al., 1983, J. Exp. Med. 157:705-719), coimmunoprecipitation (PCT International Publication No. WO 88/00209, published January 14, 1988; Oettgen, et al., 1984, J. Biol. Chem. 259:12,039-12,048) and required coexpression (Weiss et al., 1984, J. Exp. Med. 160:1284-1299) of the α,βTCR molecules with a CD3 glycoprotein complex. Subsequently, the direct physical association of the two protein complexes was demonstrated by chemically cross-linking the a, βTCR molecules to the T3 glycoprotein and identifying the components of the cross-linked complex as the TCR subunit and the T3 glycoprotein (Mᵣ 28,000) subunit (Brenner et al., 1985, Cell 40:183-190). A T3 counterpart is similarly associated with murine α,βTCR (Allison et al., 1985, Nature 314:107-109; Samelson et al., 1984, Immunol. Rev. 81:131-144).

A third gene that rearranges in T cells, designated yTCR, was identified, first in mice (Saito et al, 1984, Nature 309:757-762; Kranz et al., 1985, Nature 313:762-755; Hayday et al., 1985, Cell 40:259-269) and then in humans (Lefranc et al., 1985, Nature 316:464-466; Murre et al., 1985, Nature 316:549-552). The human yTCR locus appears to consist of between five and ten variable, five joining, and two constant region genes (Dialynas et al., 1986, Proc. Natl. Acad. Sci. U.S.A. 83: 2619). Although the total number of functional variable and joining regions is limited, significant diversity is introduced during the process of V-J joining (Kranz et al., 1985, Nature 313:752-755; Lefranc et al., 1986, Cell 45:237-246; Quertermaus et al., 1986, Nature 322:184). The yTCR gene rearrangements occur in lymphocytes with suppressor-cytotoxic as well as helper phenotypes (Lefranc et al., 1985, Nature 316:464-466; Murre et al., 1985, Nature 316:549-552, Quertermaus et al., 1986, Science 231:252-255; Lefranc et al., 1986, Cell 45:237-246, Iwamoto et al., 1986, J. Exp. Med. 163:1203-1212; Zauderer et al., 1986, J. Exp. Med. 163:1314-1318).

The products of the yTCR gene have been identified in T3 coimmunoprecipitates from aβTCR⁻CD3⁺ T (Brenner et al., 1986, Nature 322:145-149; Bank et al., 1986, Nature 322:179-181; Borst et al., 1987, Nature 325, 683-688; Moingeon et al., 1987, Nature 325, 723-726, PCT International Publication No. WO 88/00209, published January 14,1988). The yTCR polypeptides were identified by use of monoclonal antibodies directed against yTCR peptide sequences; these polypeptides were found to be incorporated into heterodimers with another polypeptide called 8TCR (Brenner et al., 1986, Nature 322:145-149). The y,8 heterodimerwas reported to be associated noncovalently with CD3.

Use of antisera directed against yTCR-specific peptides has led to the identification of CD3-associated yTCR polypeptides on cells originating in peripheral blood, thymus, and a leukemic cell line (Brenner et al., 1986, Nature 322:145-149; Bank et al., 1986, Nature 322:179-181, Weiss et al., 1986, Proc. Natl. Acad. Sci. U.S.A. 83:6998-7002; Brenner et al., 1987, Nature 325:689-694; Lew et al., 1986, Science 234:1401-1405). Bank et al. (supra) disclosed a 44 kd y form which was associated with a 62,000 kD peptide and T3 on the surface of a human thymocyte clone. A similaryγTCR polypeptide was also identified on murine T lymphocytes, and the expression of this peptide during thymocyte differentiation is the subject of much current study (Roulet et al., 1985, Nature 314:103-107; Snodgrass et al., 1985, Nature 315:232-233; Lew et al., 1986, Science 234:1401-1408; Pardau et al., 1987, Nature 326:79-81; Bluestone et al., 1987, Nature 326:82-84).

With the study of γTCR⁺ human cell lines, two differentyTCR polypeptides have been identified that differ in their molecular weight and in their ability to form disulfide linkages (Borst et al., 1987, Nature 325:683-688; Brenner et al., 1987, Nature 325:689-694; Moingeon et al., 1987, Nature 325:723-726; Lanier et al., 1987, J. Exp. Med. 165:1076). Two different yTCR constant region gene segments, called Cy1 and Cy2, respectively, have been compared; a cysteine residue encoded by the second exon of Cy1 appears to be absent in Cy2 exon segments, and its absence has been suggested to explain the inability of some yTCR peptides to form disulfide bonds (Krangel, et al., 1987, Science, 237:1051-1055; Littman et al., Nature 326:85088).

In contrast to the multiple forms of yTCR, the δTCR molecule is relatively invariant and it appears that there is only one δTCR constant region (Hata et al., 1987, Science 238:678-682).

During T cell ontogeny, it has been shown that yTCR gene rearrangement precedes β and aTCR gene rearrangement (Roulet et al., 1985, Nature 314:103-107; Snodgrass et al., 1985, Nature 315:232-233; Sangoter et al., 1986, J. Exp. Med. 163:1491-1508).

Of mature, circulating T lymphocytes, a relatively small proportion are γδTCR⁺, and exhibit either CD3⁺4⁻8⁻ (double negative) or CD3⁺4-8⁺ surface antigens. CD3⁺4⁻8⁻ T cells constitute approximately two percent of mature CD3⁺ T cells. Unlike most mature CD3⁺4⁺ or CD3⁺8⁺ major histocompatibility locus (MHC) restricted cytotoxic T cells, but similar to CD3- natural killer cells, y8TCR⁺ CD3⁺4⁻8⁻ cloned lymphocytes have been shown to exhibit MHC-nonrestricted cytolytic activity; however, unlike natural killer cells, these y8⁺ CD3⁺ 4⁻8⁻ T cells did not consistently kill natural killer cell targets, such as K-562 (Borst et al., 1987, 325:683-688; Brenner et al., 1987, Nature 325:689-694; Moingeon et al., 1987, Nature 325:723-726; Bluestone et al., 1987, Nature 326:82-84).

### 3. SUMMARY OF THE INVENTION

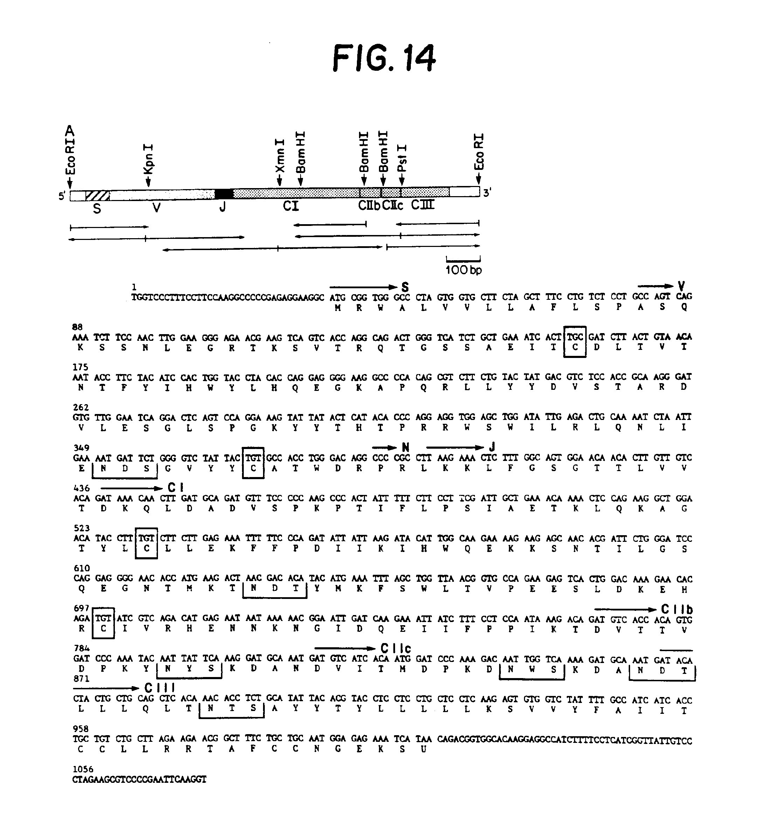

The present invention is directed to a form of the human yT cell antigen receptor (TCR) polypeptide termed Form 2bc. The Form 2bc yTCR chain has a primary amino acid sequence substantially as depicted in Figure 14. The Form 2bc yTCR chain has a molecular weight of about40,000 daltons, and comprises a constant region containing a sequence encoded by only two Cy2 CII exon copies. The invention also relates to TCR heterodimers comprising the yTCR polypeptide Form 2bc.

The present invention provides a purified polypeptide comprising an amino acid sequence of a constant region of a gamma T cell antigen receptor Form 2bc encoded by nucleotide numbers 439 through 1008 of Figure 14. The purified polypeptide has, in particular, a molecular weight of about 40,000 daltons and a sequence comprising a constant region consisting essentially of the amino acid sequence encoded by nucleotide numbers 439 through 1008 of Figure 14, and a variable region.

The present invention also provides a purified polypeptide comprising a gamma T cell antigen receptor Form 2bc having a primary amino acid sequence substantially as depicted in Figure 14 for the variable, N, joining, and constant regions.

The present invention provides nucleic acid sequences encoding the polypeptides of the present invention.

The present invention also provides a monoclonal antibody reactive with an epitope of a variable or rearranged variable-joining region of a δ chain of the human T cell antigen receptor, a monoclonal antibody reactive with an epitope of the constant region of the δ chain of the human T cell antigen receptor, and a monoclonal antibody reactive with an epitope of the constant region of the Form 2bc gamma chain of the human T cell antigen receptor, which epitope is characteristic of the Form 2bc gamma chain.

The invention also relates to monoclonal antibodies specifically reactive with an epitope of the gamma or δTCR polypeptides. Such antibodies can be identified by detecting their ability to co-modulate the CD3 antigen on a cell which expresses both the gamma,delta TCR and a CD3 antigen. In a specific embodiment, the invention provides a monoclonal antibody reactive with an epitope of a variable or rearranged variable-joining region of the δTCR chain. In a particular embodiment, such an antibody can be used to detect functional δTCR variable gene rearrangements in a cell. In another embodiment, the invention provides a monoclonal antibody reactive with an epitope of the constant region of the βTCR polypeptide. In yet another embodiment, the invention provides a monoclonal antibody reactive with an epitope of the constant region of the Form 2bc gamma chain of the human T cell antigen receptor, which epitope is characteristic of the Form 2bc gamma chain.

The present invention also provides a purified polypeptide complex comprising a T cell antigen receptor heterodimer consisting of the gamma T cell antigen receptor polypeptide of claim 17 and a second T cell antigen receptor polypeptide selected from the group consisting of the a, β, gamma, and δ T cell antigen receptor polypeptides.

The present invention further provides a method for producing a gamma,delta T cell antigen receptor heterodimer which comprises culturing a cell (a) capable of expressing a nucleic acid encoding a δ T cell antigen receptor polypeptide, and (b) which has been transfected with a nucleic acid encoding a gamma T cell antigen receptor polypeptide, under conditions such that both the gamma T cell antigen receptor polypeptide and the δ T cell antigen receptor polypeptide are expressed by the cell and assemble to form a heterodimer.

The present invention also provides a method for producing at least a portion of a gamma,delta T cell antigen receptor heterodimer, which comprises culturing a transfected cell (a) capable of expressing a nucleic acid encoding at least a region of a gamma T cell antigen receptor polypeptide selected from the group consisting of a constant region, a variable region, and a joining region; and (b) capable of expressing a nucleic acid encoding at least a region of a δ T cell antigen receptor polypeptide selected from the group consisting of a constant region, a variable region, a joining region, and a diversity region; and subjecting the cell to conditions such that both nucleic acid sequences are expressed by the cell.

The nucleic acid encoding at least a region of the gamma TCR may be transformed into the cell.

The present invention also provides cells that express at least a portion of the gamma,delta TCR.

The present invention, in addition, provides a method for determining the relative usage of a gamma T cell antigen receptor polypeptide in a sample from a human subject comprising:
(a) determining in a sample containing T cells from a subject the amount of a gamma Form 2bc T cell antigen receptor polypeptide;
(b) determining in a sample from a subject the amount of a gamma T cell antigen receptor polypeptide selected from the group consisting of a Form 1 gamma polypeptide, and Form 2abc gamma polypeptide;
   and
(c) comparing the amount determined in step (a) with the amount determined in step (b), thereby determining the relative usage.

### 3.1. DEFINITIONS

As used herein, the following terms will have the meanings indicated:

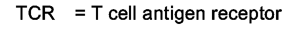

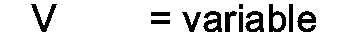

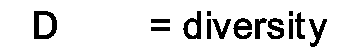

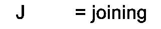

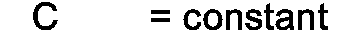

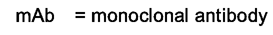

### 4. DESCRIPTION OF THE FIGURES

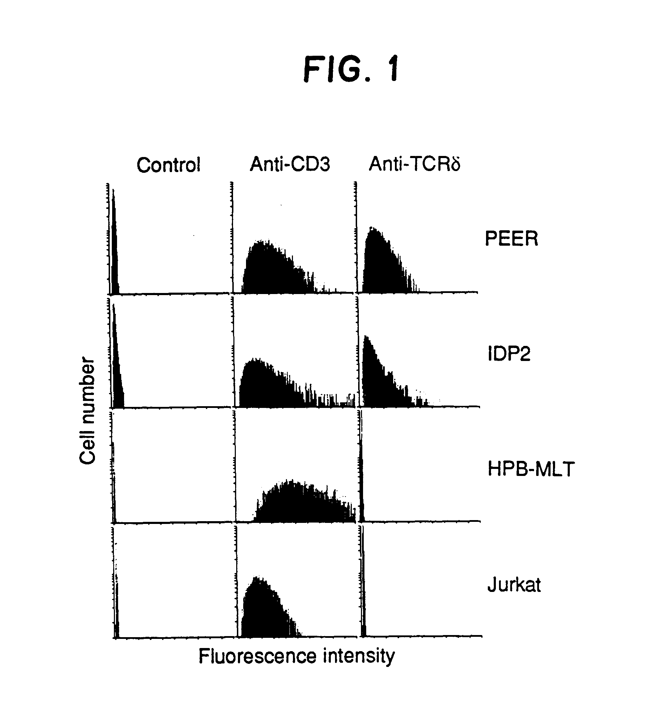

Figure 1. Cytofluorographic analysis of T cell lines with anti-TCR81.

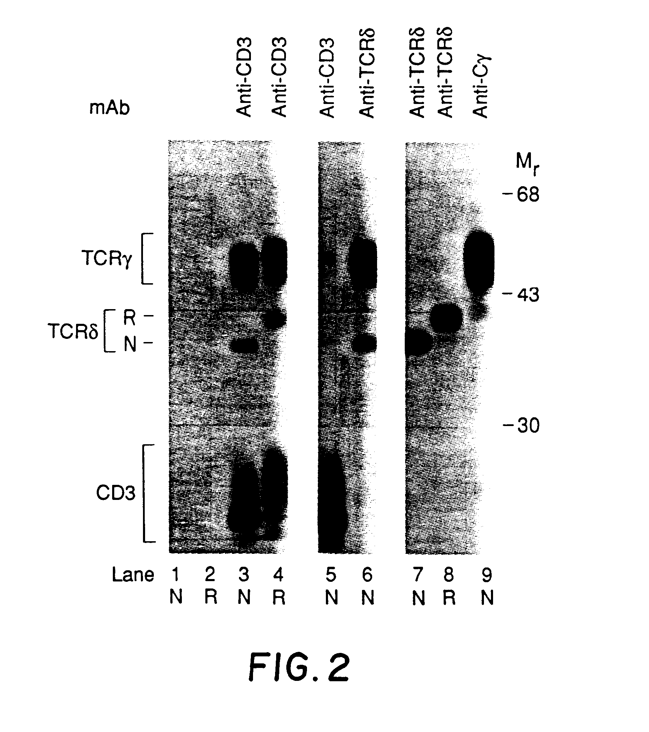

Figure 2. Immunochemical analysis of the specificity of mAb anti-TCRδ1. Surface ¹²⁵1-labeled IDP2 cells were immunoprecipitated using control mAb P3 (lanes 1 and 2), anti-leu 4 (lanes 3-5), anti-TCR81 (lanes 6-8), or anti-Cy serum (lane 9) and were then resolved by SDS-PAGE (polyacrylamide gel electrophoresis) and visualized by autoradiography. N = nonreducing conditions; R = reducing conditions.

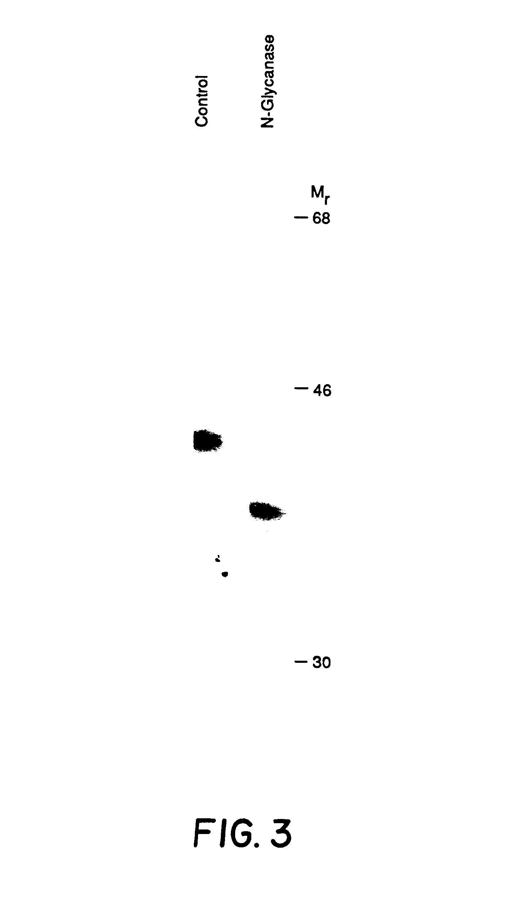

Figure 3. N-glycanase digestion of δTCR.

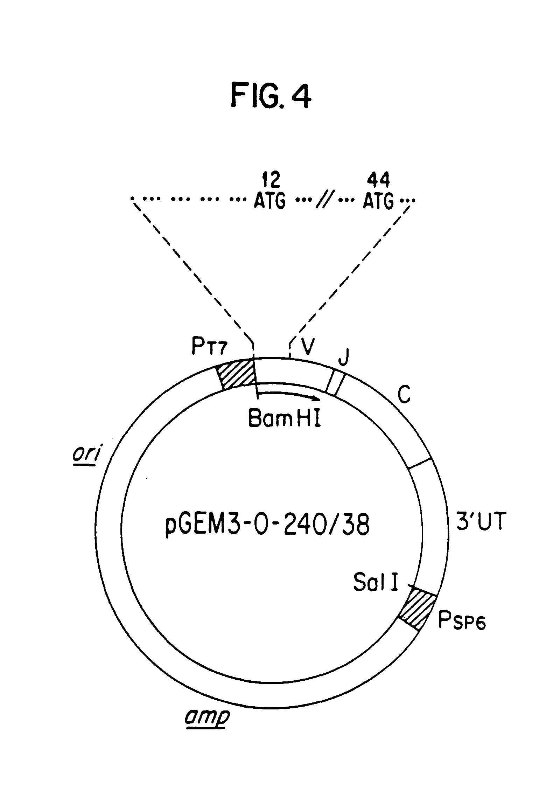

Figure 4. Map of pGEM3-0-240/38.

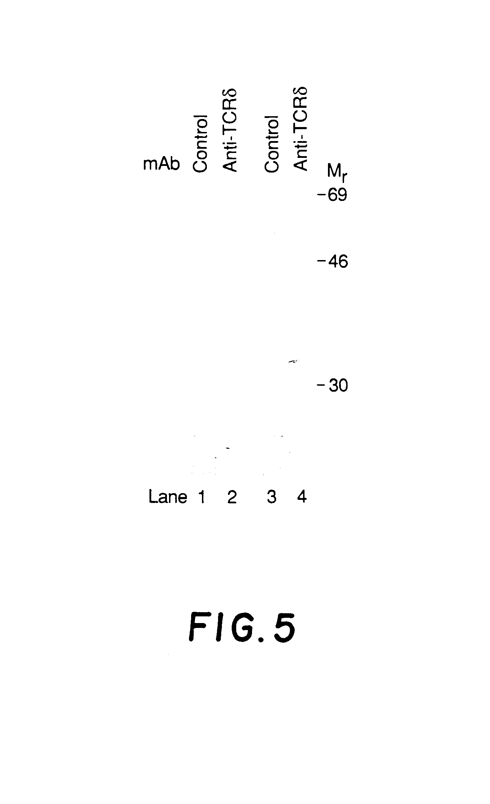

Figure 5. Immunoprecipitation of in vitro translation products of cDNA clone IDP2 0-240/38 by mAb anti-TCR81.

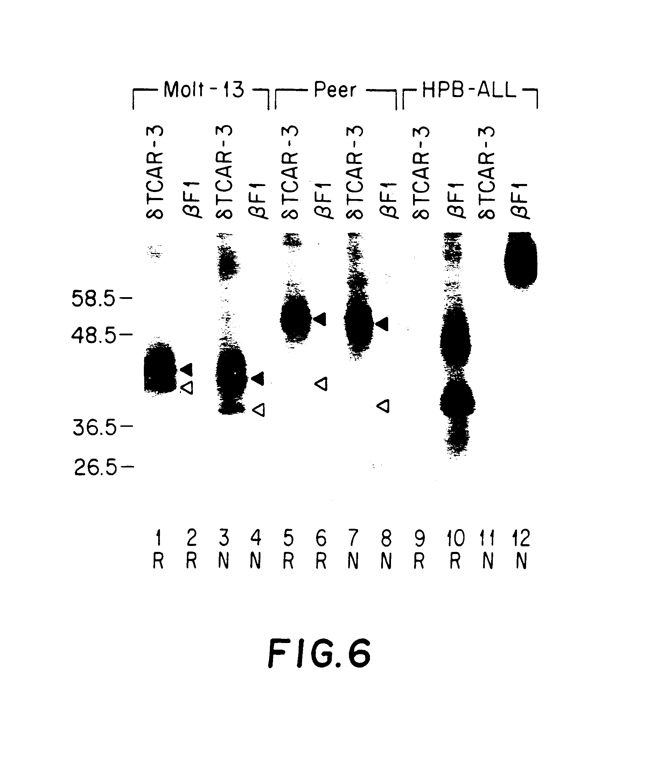

Figure 6. Immunoprecipitation and SDS-PAGE analysis of T cell antigen receptor. Open arrowheads indicate the position of the δ chains. The solid arrowheads indicate the position of the y chains. Lysates were immunoprecipitated using βTCAR-3 antibody (odd numbered lanes) or βF1 antibody (even numbered lanes).

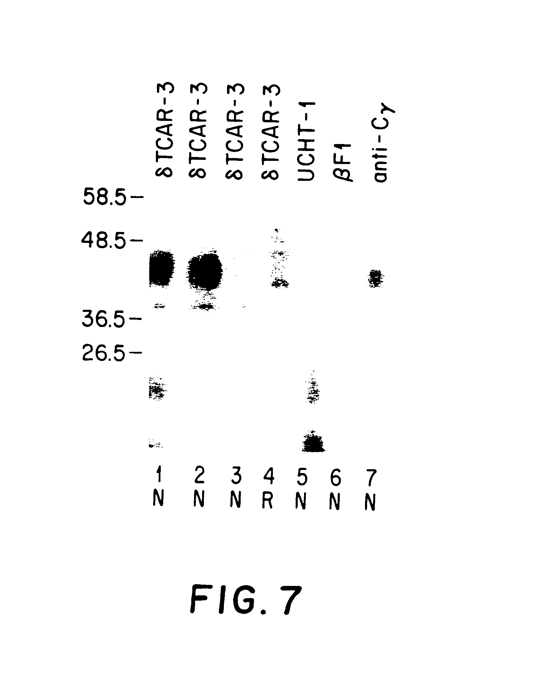

Figure 7. Immunoprecipitation of δ chain by δTCAR-3 antibody. Molt-13 cells solubilized in Tris-buffered saline (pH 8) containing 0.3% CHAPS (lane 1) or in 1% Triton X-100 (lanes 2-7). Lane 1, δTCAR-3 immunoprecipitates y,8TCR heterodimer with the CD3 proteins. Lane 2, δTCAR-3 immunoprecipitates γ,δTCR heterodimer without the CD3 proteins. Lanes 3 and 4, δTCAR-3 immunoprecipitates single δ chain from denatured lysates (N) and reducing (R) conditions, respectively. Lane 5, UCHT-1 immunoprecipitates the CD3 proteins. Lane 6, βF1 antibody does not immunoprecipitate a heterodimerfrom MOLT-13 cells. Lane 7, anti-Cy antiserum immunoprecipitates a single y chain.

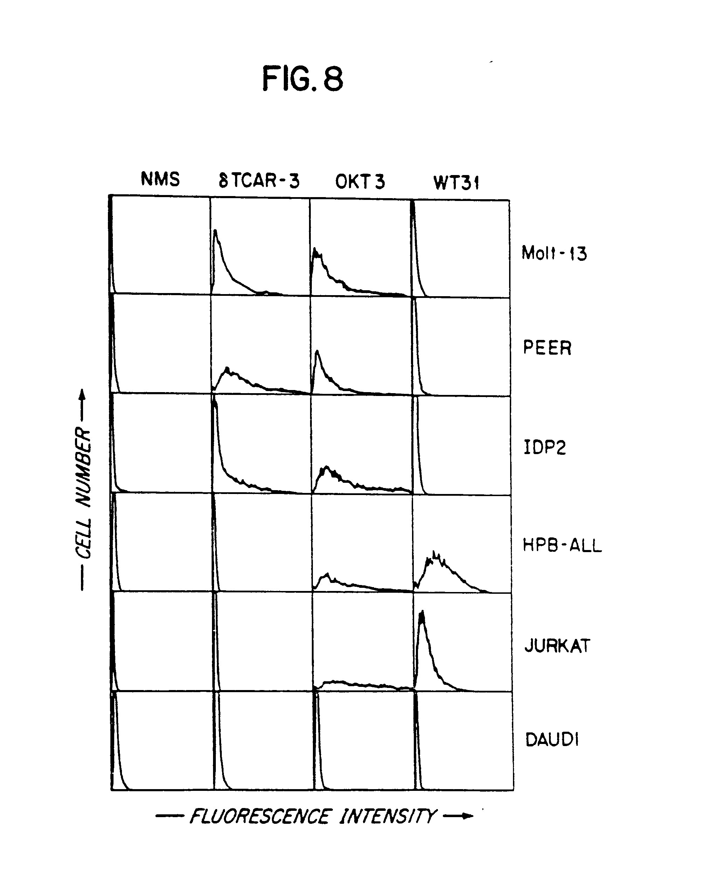

Figure 8. Analysis of cell surface staining by flow cytometry. γ,δTCR-positive cells (MOLT-13) PEER, IDP2) and α,βTCR-positive cells (HPB-ALL, Jurkat) were incubated with δTCAR-3, OKT3, WT31 and normal mouse serum (NMS) antibodies and analyzed by flow cytometry. The B cell line, Daudi, was the negative control.

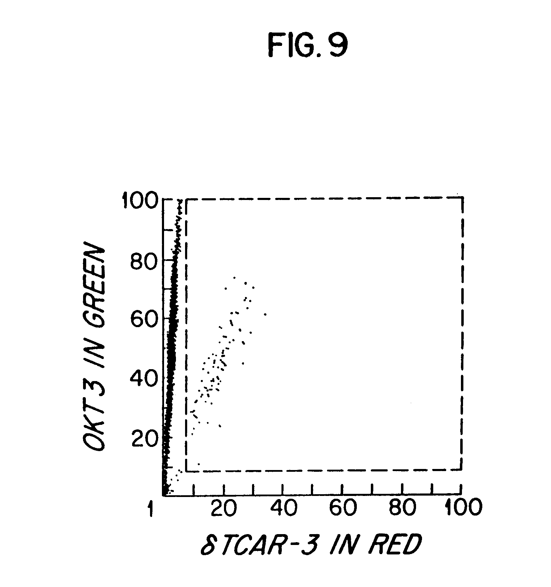

Figure 9. Two color cytofluorographic analysis of δTCAR-3⁺ and OKT3⁺ peripheral blood lymphocytes. The fluorescein isothiocyanate (FITC) fluorescence is depicted on the Y axis and phycoerythrin (PE) fluorescence on the X axis. The CD3⁺ γ,δTCR⁺ cells in this sample represent 2.4% of CD3⁺ lymphocytes.

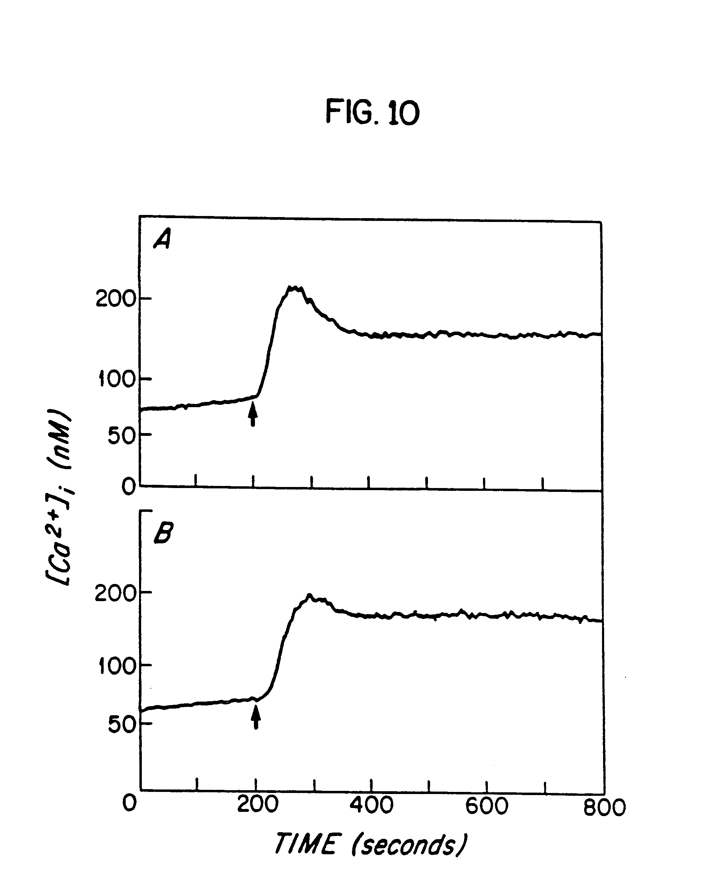

Figure 10. Measurement of intracytoplasmic Ca2+ concentration ([Ca²⁺]ᵢ) versus time. Top panel: δTCAR-3. Bottom panel: Anti-Leu antibody. Arrows indicate the time of addition of antibody.

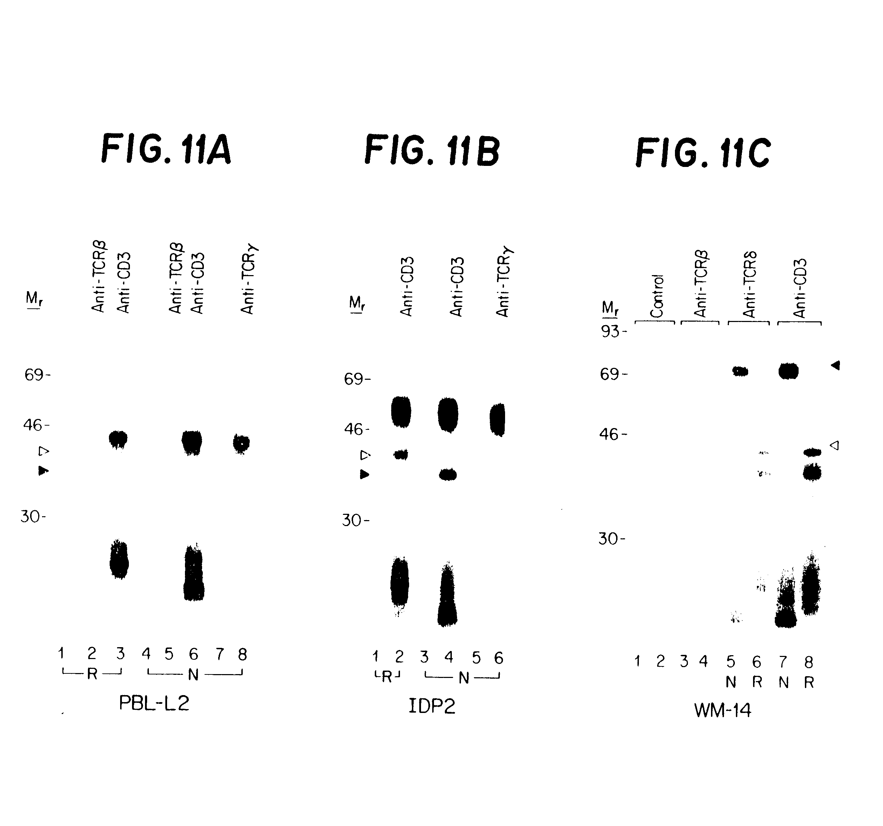

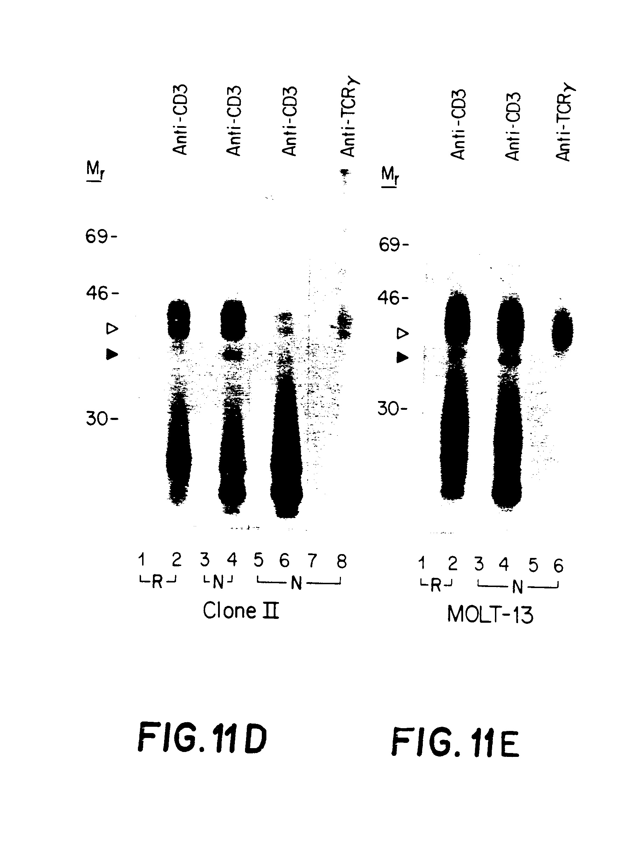

Figure 11. Immunoprecipitation of the three forms of γ,δTCR. For parts A-E, the antibodies used for immunoprecipitation are anti-Leu4 (anti-CD3), βF1 (anti-TCRβ, anti-81TCR (anti-8TCR), anti-Cyb serum (anti-yTCR) and P3 (unlabelled lanes, control). Immunoprecipitations from ¹²⁵1-labelled cell lysates were analyzed by SDS-PAGE (10% polyacrylamide) under reducing (R) or nonreducing (N) conditions. An open arrow (▷) indicates the position of TCR δ under reducing conditions, whereas the solid arrow (▷) denotes the position of δTCR under nonreducing conditions. Size markers, Mᵣ in thousands, are shown on the left.
   A) Nondisulfide-linked yTCR (40 kD) on PBL-L2. In lanes 1-6 the radiolabelled cells were solubilized in 0.3% CHAPS detergent which preserves the TCR-CD3 association, whereas in lanes 7 and 8, immunoprecipitations were performed after chain separation (see methods).
   B) Nondisulfide-linked yTCR (55 kD) on IDP2 cells. In lanes 1-4 radiolabelled cells were solubilized in 0.3% CHAPS detergent, whereas in lanes 5 and 6 imunoprecipitations were carried out after chain separation.
   C) Disulfide-linked yTCR (40 kD) on WM-14 cells. All lanes correspond to immunoprecipitations from 1% digitonin solubilized radiolabelled cells.
   D) Nondisulfide-linked yTCR (40 kD) on thymic Clone II cells. Radiolabelled cells were solubilized in 1% digitonin (lanes 1-4) or in 0.1% Triton X-100 (lanes 5 and 6), whereas in lanes 7 and 8 immunoprecipitations were carried out after chain separation.
   E) Nondisulfide-linked yTCR (40 kD) on MOLT-13 leukemia T cells. In lanes 1-4 immunoprecipitations were carried out after solubilization of cells in 0.3% CHAPS detergent, whereas in lanes 5 and 6 immunoprecipitations were carried out after chain separation.

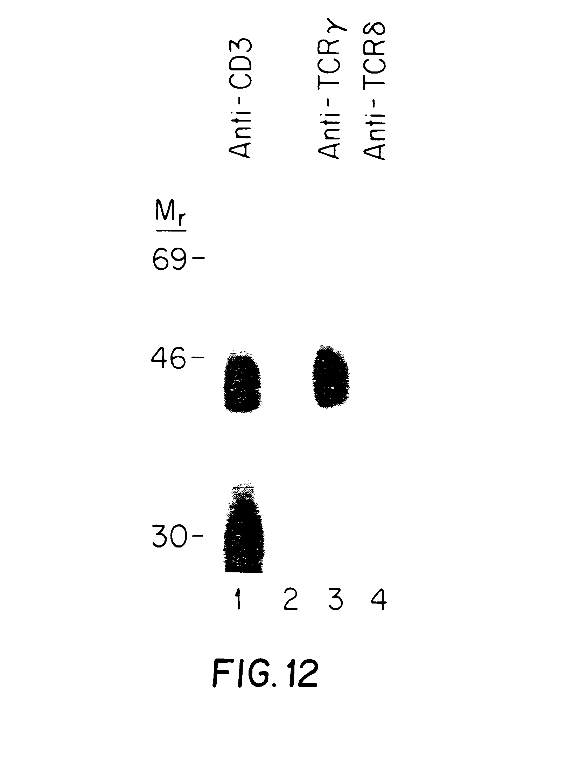

Figure 12. Immunoprecipitation of yTCR and δTCR chain by anti-Cym1 antibody and anti-TCR81 antibody, respectively. Cell surface radiolabelled MOLT-13 cells were solubilized in 0.3% CHAPS detergent and the y, δTCR-CD3 complex was isolated with anti-CD3 monoclonal antibody. Immunoprecipitates were analyzed by 10% SDS-PAGE under reducing conditions.
   Lane 1: Immunoprecipitation with anti-Leu4 (anti-CD3) mAb
   Lane 3: Immunoprecipitation with anti-Cyml (anti-TCRy) mAb after separating chains of isolated γ,δTCR-CD3 complexes.
   Lane 4: Immunoprecipitation with anti-TCR81 (anti-TCR8) mAb after separating chains of isolated γ,δTCR-CD3 complexes.

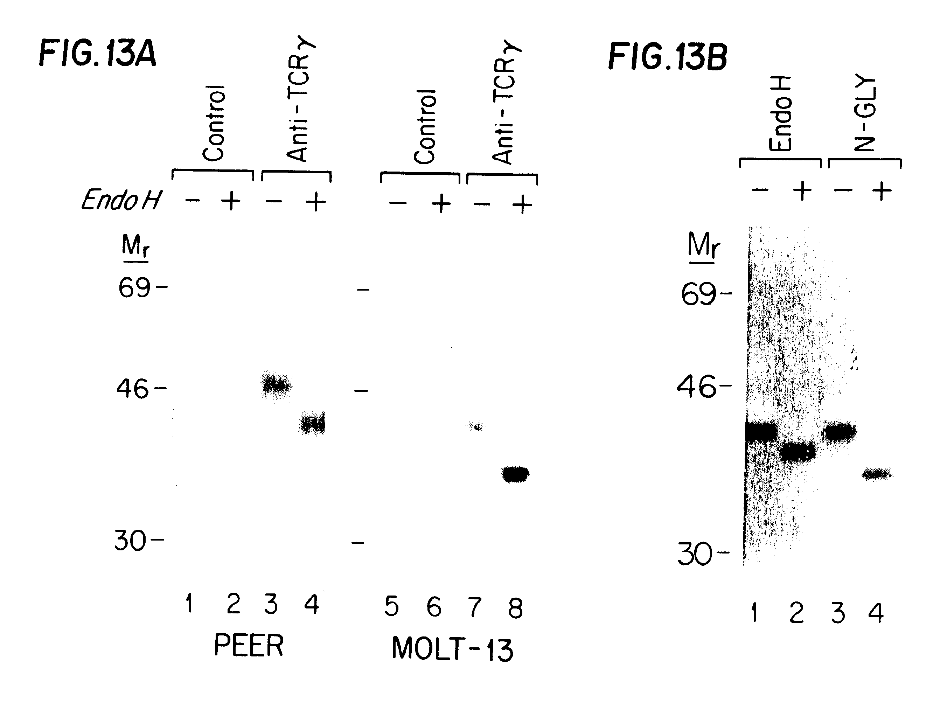

Figure 13. Determination of peptide backbone sizes and glycosylation of y and δTCRs from PEER and MOLT-13 cells. Monoclonal antibodies used for immunoprecipitation are anti-Cyml (anti-TCRy), anti-TCR81 (anti-TCR8) and P3 (labelled control) as shown at the top of each lane. The labelled cell lines used are shown at the bottom of each 10% SDS-PAGE autoradiograph or fluorograph. All samples were resolved under reducing conditions. Size markers, Mᵣ in the thousands.
   A) Peptide backbone sizes of yTCR from PEER and MOLT-13 cells. Cells were biosynthetically labelled with ³⁵S-cysteine and ³⁵S-methionine for 15 minutes. Samples were either treated with Endo H (+) or mock treated (-). Immunoprecipitation with anti-Cyml shows the positions of immature yTCR of PEER cells (lane
   3) and of MOLT-13 cells (lane 7), while the corresponding polypeptide backbone sizes are visualized after treatment with endo H (lanes 4 and 8).
   B) Glycosylation of TCR δ from MOLT-13 cells. ¹²⁵1-labelled cells were immunoprecipitated with anti-CD3 mAb and the δTCR polypeptides were gel purified (see methods) before incubation with N-glycanase (lane 4), endo H (lane 2), or mock treated (lanes 1, and 3).
Figure 14. Nucleotide sequence of MOLT-13 yTCR (Form 2bc). Part A: Sequencing strategy of clone M13k. A partial restriction map of the 1.1 kb cDNA clone M13k is shown. Part B: Nucleotide and deduced amino acid sequence of clone M13k. Signal sequence (S), variable (V), N-region (N), joining (J) and constant (Cl, Cllb, Cllc and CIII) region gene segments are indicated by arrows and were identified by comparison to genomic sequences, described by Lefranc et al., (1986, Cell 45:237-246) (for S and V), Lefranc et al., (1986, Nature, 319:420-422) and Quertermous et al., (1987, Immunol. 138:2687-2690) (for J) and Lefranc et al., (1986, Proc. Natl. Acad. Sci. U.S.A. 83:9596-9600) and Pellicci et al., (1987, Science 237:1051-1055) (for C). The deduced amino acid sequence beginning at the initiator methionine is presented below the nucleotide sequence. Extracellular cysteines are highlighted by boxes, and potential N-linked carbohydrate attachment sites (N-X-S or N-X-T; Marshall, 1977, Ann. Rev. Biochem. 41:673-702) are indicated by brackets.

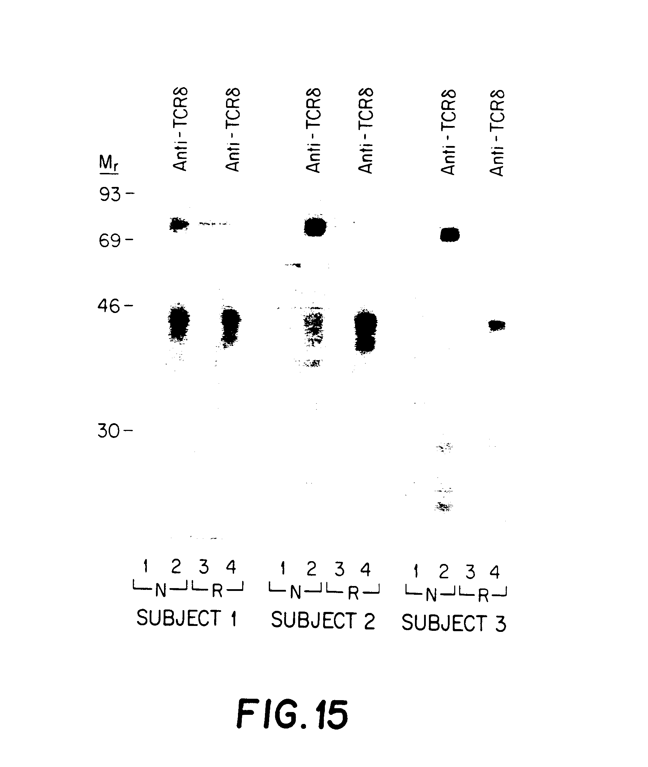

Figure 15. Preferential use of γ,δTCR Form 1. Freshly isolated peripheral blood mononuclear cells from three healthy donors were ¹²⁵1-labelled and solubilized in 1% Triton X-100. Immunoprecipitates with P3 (control, lanes 1 and 3), and anti-TCR81 (anti-TCRδ, lanes 2 and 4), were analyzed under nonreducing (N) and reducing (R) conditions. Mᵣ markers in the thousands are shown on the left.

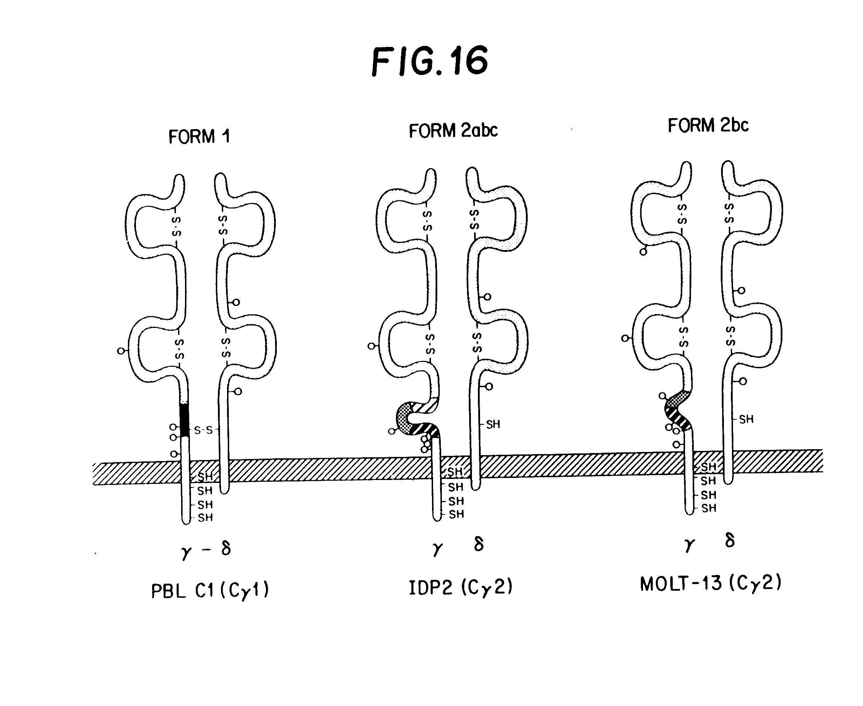

Figure 16. Schematic representation of the three γ,δTCR forms in man. The CII exon encoded connector peptides are highlighted by filled areas ( 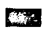 ) as Cy1 CII exon encoded peptide; 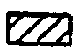 , 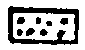 , 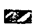 , as Cy2 CII exon copy a, copy b, and copy c encoded peptides, respectively). Potential N-linked glycan attachment sites (o), and sulfhydryl groups (-SH) and putative disulfide bridges (-S-S-) are indicated.

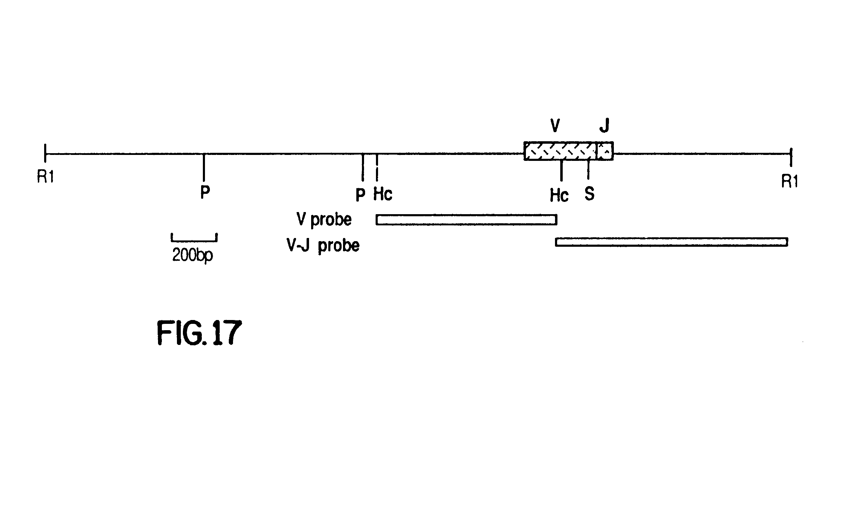

Figure 17. Map of the rearranged δTCR gene. A map of r119δ1 including EcoRI (RI), Hinc II (Hc), Scal (S) and Pvull (P) sites and probes used in Southern blot analysis is shown.
Figure 18A. Schematic representation of the yTCR chains used for transfection into MOLT-13 cell line. The schematic is based on reported analyses (Brenner, M.B., et al., 1986, Nature 322:145-149; Brenner, M.B., et al., 1987, Nature 325:689-694; Krangel, M.S., et al., 1987, Science 237:64-67). Pred., predicted; Obs., observed. The predicted glycosylated polypeptide size assumes that all available N-linked glycosylation sites (shown as lollipops), each containing 3 kD of attached carbohydrate, are used, and that no significant size differences are introduced by other post-translational modifications. The intra-chain disulfide linkages typical of Ig-like molecules are shown. Note that a cysteine residue (cys) is encoded by the CII exon in PBL C1 yTCR, but such a cysteine is absent from all the copies of CII exon used in the two other _{γ}TCR chains. yTCR constant region in the y,8 T leukemia cell line PEER (Littman, D.R., et al., 1987, Nature 326:85-88) that also expresses a 55 kD yTCR protein (Brenner, M.B., et al., 1987, Nature 325:689-694; Weiss, A, et al., 1986, Proc. Natl. Acad. Sci. USA 83:6998-7002) is identical to that of IDP2.

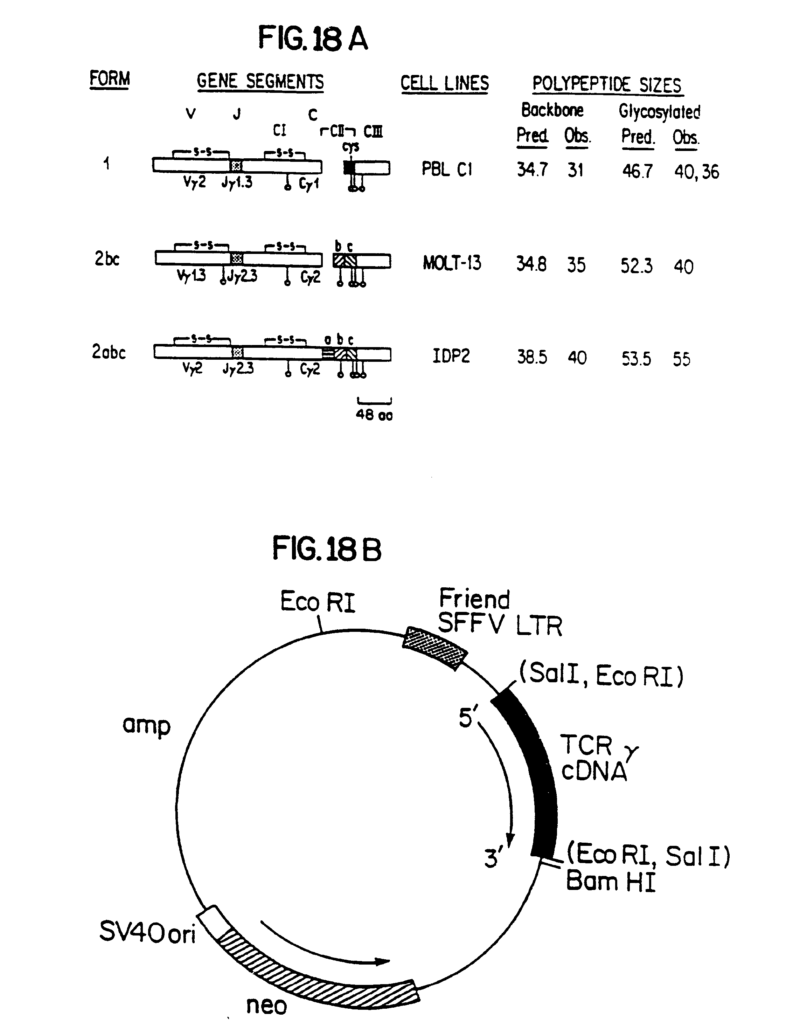

Figure 18B. The expression plasmid constructs pFneo.PBL C1y and pFneo.IDP2y were used to introduce yTCR clones into the MOLT-13 cell line. PBL C1 yTCR cDNAclone (PBL C1.15) and repaired IDP2 yTCRcDNA clone (IDP2.11 r) (Krangel, M.S., et al., 1987, Science 237:64-67) were cleaved from their parent plasmid vector (pUC 18) by EcoRl digestion, the ends were made blunt with Klenow fragment of DNA polymerase I, and the cDNAs were then ligated into a Sail-cut, and Klenow-treated pFneo mammalian expression vector. Clones containing the cDNA inserts in appropriate orientation with respect to the spleen focus forming virus (SFFV) LTR were selected based on restriction mapping. pFneo (Saito, T., et al., 1987, Nature 325:125-130) is a derivative of pTβFneo (Ohashi, P., et al., 1985, Nature 316:606-609) obtained by BamHl digestion, to delete the murine βTCR cDNA insert, followed by ligation with T4 DNAligase. As shown, this vector contains a bacterial neomycin resistance gene (neor) under the control of SV40 promoter, thus conferring resistance to the antibiotic G418 on the mammalian recipient cells. The restriction sites within parentheses were destroyed during construction.

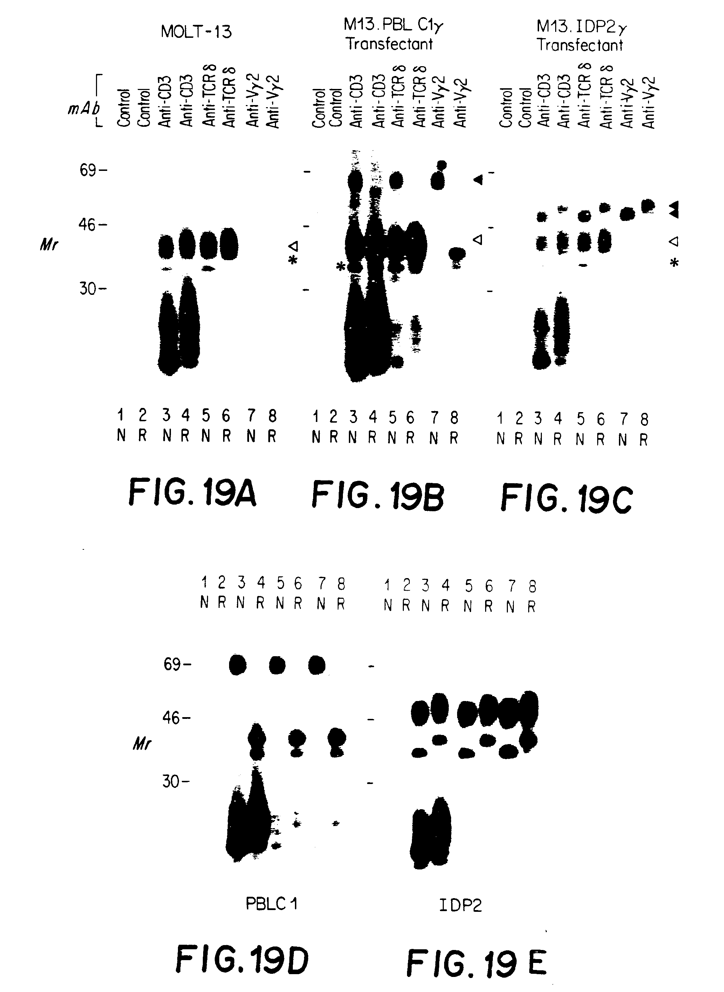

Figure 19. Immunoprecipitation analysis of γ,δTCR on Molt-13 yTCR transfectants. Surface ¹²⁵I-labeled cells were solubilized in 0.3% CHAPS detergent to preserve the chain association, immunoprecipitated with mAb P3 (control), anti-leu-4 (anti-CD3), anti-TCRδ1 (anti-δTCR), or anti-Ti-yA (anti-Vy2, and were then resolved by SDS-PAGE under nonreducing (N) or reducing (R) conditions and visualized by autoradiography as described earlier (Brenner, M.B., et al., 1986, Nature 322:145-149; Brenner, M.B., et al., 1987, Nature 325:689-694). Anti-Ti-yA mAb shows a pattern of reactivity on different T cell clones consistent with its recognition of Vy2 segment. M13.PBL C1y: MOLT-13 cells transfected with the PBL C1-derived yTCR cDNA; Clone #7 was used for this analysis. M13.IDP2_{γ}: MOLT-13 cells transfected with the IDP2-derived yTCR cDNA; Clone #10 was used for this analysis. Size markers, Mᵣ (molecular weight) in thousands of daltons. Open arrow, resident MOLT-13 yTCR chain; solid arrow, transfected (PBL C1- or IDP2-derived) yTCR cDNA; asterisk, MOLT-13 δTCR chain under nonreducing conditions. Upon reduction, the δTCR chain undergoes a mobility shift and comigrates with the 40 kD yTCR chain. However, δTCR chain is distinctly visualized as a 40 kD band under reducing conditions when the 40 kD yTCR protein is not coimmunoprecipitated, as is seen in anti-Vy2 immunoprecipitates of M13.IDP2y (Fig. 19, lane 8).

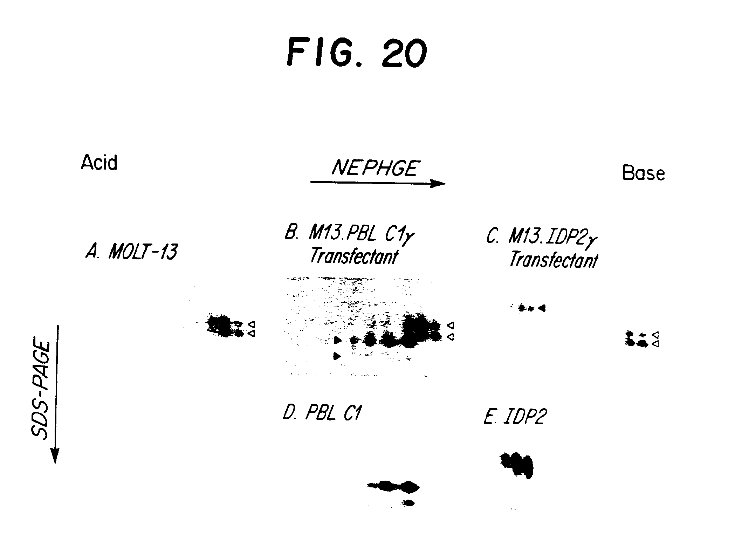

Figure 20. Two-dimensional gel analysis of yTCR polypeptides of transfectants. 2x10⁷ cells were surface ¹²⁵1 labeled, treated with neuraminidase (150 units in 1.5 ml PBS with 1 mg/ml each of glucose and bovine serum albumin for 1.5 hours at 23°C), solubilized in 0.3% CHAPS detergent, immunoprecipitated with 1 µg anti-leu-4 mAb, and subjected to 2D gel analysis under reducing conditions. Non-equilibrium pH gradient gel eletrophoresis (NEPHGE) was carried out using pH 3.5 to 10 Ampholines (LKB, Sweden) followed by SDS- polyacrylamide gel electrophoresis on a 10.5% acrylamide gel (Brenner, M.B., et al., 1987, Nature 325:689-694). Positions of the CD3 components (not shown) were used to identify and compare the yTCR species expressed in different cell lines. M13.PBL C1y transfectant clone #10 and M13IDP2γ transfectant clone#10 were used. Open arrows, MOLT-13 yTCR species; solid arrows, IDP2 yTCR species; asterisk, PBL C1 yTCR species.

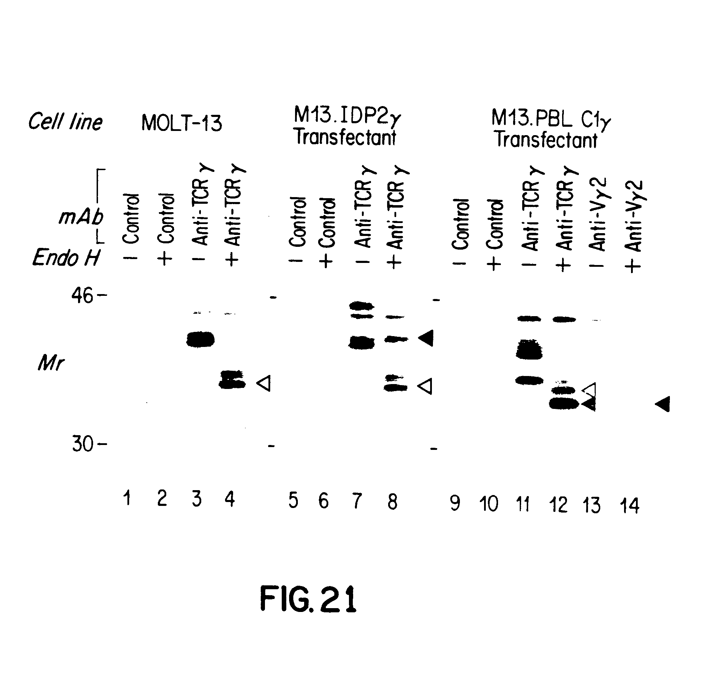

Figure 21. Analysis of backbone polypeptide sizes of yTCR chains of transfectants. Cells were pulse labeled with ³⁵S-methionine and ³⁵S-cysteine for 15 minutes and immunoprecipitated with P3 (control), anti-Cym1 (anti-yTCR), oranti-Ti-yA(anti-Vy2) mAb, as indicated. Immunoprecipitates were treated with endoglycosidase-H (Endo-H, +), or were mock-incubated (-), resolved by SDS-PAGE, and visualized by fluorography. All samples were run reduced. Mᵣ, molecular weight markers in thousands of daltons. The 43 kD contaminating actin band serves as an additional internal marker. M13.IDP2y: MOLT-13 cells transfected with the IDP2 yTCR cDNA, clone #10; M13.PBL Cly: MOLT-13 cells transfected with the PBL CI yTCR cDNA, clone #10.

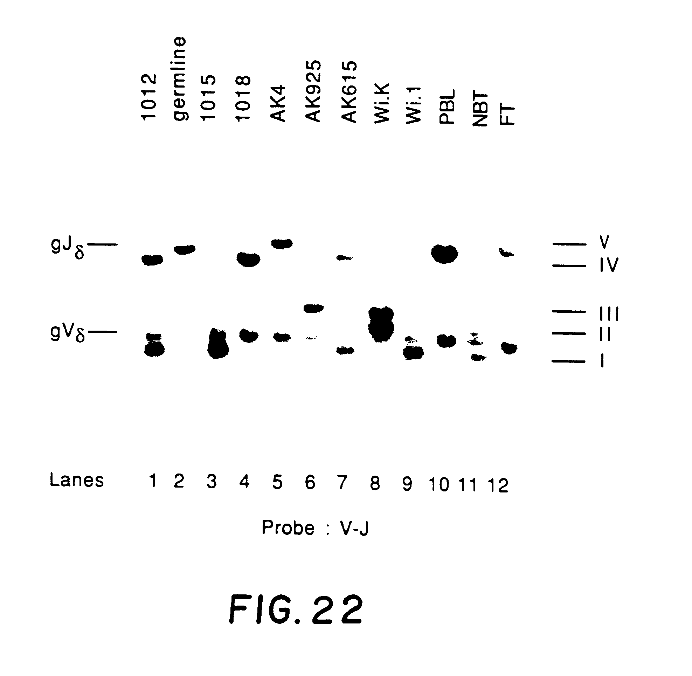

Figure 22. Southern blot analysis of y6 T cell clones and polyclonal human T cell populations. Genomic DNA was digested with EcoRl and probed with the V-J probe. DNA sources are: PBL T-cell clones, (lanes 1, 3-9), PBL (lane 10), newborn thymocytes (NBT-lane 11), fetal thymocytes (FT-lane 12), and B cells (germline- lane 2). The germline 3 kb V8 and 6.7 kb J8 fragment are indicated on the left of the blot, while the 5 common rearrangements, numbered I-V are indicated on the right. The sizes of the rearrangements from I-V are 2.9 kb, 3.5 kb, 4.2 kb, 6.2 kb and 7.1 kb respectively.

### 5. DETAILED DESCRIPTION OF THE INVENTION

### 5.1. THE yTCR FORM 2bc POLYPEPTIDE AND NUCLEIC ACIDS

The invention is directed to a form of the human yTCR polypeptide termed Form 2bc (detailed infra in Sections 8 and 9). The invention is also directed to nucleic acids encoding y Form 2bc, such as DNA and RNA, and their complementary nucleic acids.

Form 1 and Form 2abc yTCR polypeptides are previously reported forms of the human yTCR (see PCT International Publication No. WO 88/00209, Published January 14, 1988). The Form 1 yTCR polypeptide has a molecular weight of about 40,000 daltons. The Form 2abc yTCR polypeptide has a molecular weight of about 55,000 daltons. Form 2abc yTCR chain has a slightly larger peptide backbone and contains one extra potential N-linked glycan than Form 1. In contrast, the yTCR chain of the invention, Form 2bc, has a molecular weight of about 40,000 daltons. Furthermore, the Form 2bc yTCR polypeptide possesses a slightly smaller peptide backbone and 2-3 less N-linked glycans.

yTCR chain Form 2bc differs in size by more than 15 kD (40 kD versus 55 kD) compared to the previously described Form 2abc. This difference is accounted for by a 5 kD smaller polypeptide backbone size (35 kD versus 40 kD) and by a reduction in the amount of carbohydrates (5 kD versus 15 kD). The approximately 35 kD polypeptide backbone size of Form 2bc also serves to distinguish it from Form 1; Form 1 has a 40 kD backbone size.

yTCR polypeptide Form 2bc also differs from Form 1 and Form 2abc in constant region (Cy) gene segment usage. Form 1 yTCR chains have a constant region encoded by the Cy1 gene segment (Krangel et al., 1987, Science 237:64-67) containing a single CII exon. The Form 2abc y polypeptide is encoded by Cy2 gene segments containing three CII exon copies, namely copy a, copy b and copy c (Krangel et al., 1987, Science 237:64-67; Littman et al., 1987, Nature 326:85-88). In contrast, Form 2bc lacks one copy of the sequence encoded by the Cy2 second exon that is present in the cDNAof Form 2abc. This, Form 2bc contains two Cy2 CII exon copies, namely copy b and copy c. Copy a of CII, which is missing in Form 2bc, encodes a part of a connector region between the membrane spanning region and the extracellular constant domain.

Six potential N-linked carbohydrate attachment sites exist on the Form 2bc polypeptide. Since the biochemical data suggest that only 2-3 N-linked glycans are attached to the polypeptide chain, it indicates that not all potential sites are used.

In specific embodiments, yTCR polypeptide Form 2bc can be obtained from cells of the MOLT-13 (Loh et al., 1987, Nature 330:569-572) T cell line or thymus-derived Clone II (Bank et al., 1986, Nature 322:179-181). yTCR chain Form 2bc can also be obtained from T lymphocytes of a human subject which express that yTCR form.

Form 2bc yTCR polypeptide comprises the primary amino acid sequence of the yTCR polypeptide shown in Figure 14, or any portion thereof comprising a constant region consisting of copy b and copy c of Cy2 CII.

The present invention also provides a nucleic acid molecule encoding a yTCR Form 2bc polypeptide having a molecular weight of about 40,000 daltons. The constant region of yTCR Form 2bc polypeptide results from translation of a nucleic acid sequence which has only two of the three Cy2 cll exons. The invention is also directed to nucleic acid sequences comprising a Cy2 constant region having only two cll exons. The nucleic acid can be a DNA, cDNA, RNA, and complementary nucleic acids and derivatives thereof. In a specific embodiment of the invention, the DNA molecule comprises at least a portion of the nucleic acid sequence shown in Figure 14.

In an example to be discussed in Section 8, the 2bc yTCR polypeptide and its encoding nucleic acid sequence are described. In an example to be discussed in Section 9, it is shown that the ability of the yTCR polypeptide to form disulfide bonds or be glycosylated is determined by its constant region primary sequence.

### 5.2. POLYPEPTIDE COMPLEXES CONTAINING yTCR FORM 2bc

The present invention also relates to polypeptide complexes which comprise the yTCR chain Form 2bc. In a specific embodiment, the polypeptide complex consists of a T cell antigen receptor dimer. In particular, such a dimer can be a heterodimer (including but not limited to a y,8 heterodimer, a γ,β heterodimer, and a a,y heterodimer, or a y,y' heterodimer in which y' can be yTCR polypeptide Form 1, 2abc, or 2bc), or a homodimer.

In a particular embodiment of the invention, the polypeptide complex comprising yTCR Form 2bc is a γδTCR heterodimer. Thus, a purified complex which comprises at least a portion of a 8TCR polypeptide and yTCR Form 2bc polypeptide is provided by the present invention. The δ polypeptide may have at least one intrachain, covalent, disulphide bridge. Additionally, the polypeptide may comprise a 8TCR polypeptide having a molecular weight of about 40,000 daltons.

As detailed in the examples infra, the yTCR Form 2bc chain is noncovalently associated in a complex with the δTCR chain. Thus, y Form 2bc forms a nondisulfide-linked TCR complex. yTCR chain Form 2abc also forms a nondisulfide-linked complex with a δTCR chain (e.g., on IDP2 cells), while yTCR chain Form 1 forms a disulfide-linked complex with a δTCR polypeptide.

As shown in the example of Section 9, infra, yTCR constant region CII exon usage (and thus the primary sequence of the yTCR chain) determines not only the presence or absence of disulfide linkage between TCR y and 8, but also the amount of carbohydrate attached to yTCR, which is largely responsible for the differences in size of the cell surface yTCR proteins. Thus, the present invention also provides a method for producing expression of γδTCR heterodimers of defined intermolecular linkage (disulfide or nondisulfide-linked) and extent of yTCR glycosylation, which comprises introducing a yTCR gene encoding a particular y polypeptide form into a cell capable of expressing the y gene, which cell expresses the δTCR chain.

The present invention further provides a purified complex which comprises a yTCR Form 2bc polypeptide of the present invention associated with another yTCR polypeptide (e.g., Form 1, 2abc, or 2bc). In one embodiment of the invention, the two yTCR polypeptides are associated with each other through at least one in- terchain, covalent, disulfide linkage. In another embodiment of the invention, the two yTCR polypeptides are noncovalently associated with each other. In still another embodiment of the invention, the two yTCR polypeptides have the same constant domain. In yet a further embodiment of the invention, the two yTCR polypeptides have different constant domains.

### 5.3. MONOCLONAL ANTIBODIES REACTIVE WITH THE yoTCR POLYPEPTIDES

A monoclonal antibody (mAb) to an epitope of the y or δ T cell antigen receptor can be prepared by using any technique which provides for the production of antibody molecules by continuous cell lines in culture. These include but are not limited to the hybridoma technique originally described by Kohler and Milstein (1975, Nature 256:495-497), and the more recent human B cell hybridoma technique (Kozbor et al., 1983, Immunology Today 4:72) and EBV-hybridoma technique (Cole et al., 1985, Monoclonal Antibodies and Cancer Therapy, Alan R. Liss, Inc., pp. 77-96).

In one embodiment, the monoclonal antibodies may be human monoclonal antibodes or chimeric human- mouse (or other species) monoclonal antibodies. Human monoclonal antibodies may be made by any of numerous techniques known in the art (eg., Teng et al., 1983, Proc. Natl. Acad. Sci. U.S.A. 80:7308-7312; Kozbor et al., 1983, Immunology Today 4:72-79; Olsson et al., 1982, Meth. Enzymol. 92:3-16). Chimeric antibody molecules may be prepared containing a mouse (or rat, or other species) antigen-binding domain with human constant regions (Morrison et al., 1984, Proc. Natl. Acad. Sci. U.S.A. 81:6851; Takeda etal., 1985, Nature 314:452).

The invention is also directed to a method of identifying a monoclonal antibody reactive with a T cell antigen receptor. Such a mAb can be identified by detecting its ability to comodulate the CD3 antigen upon binding of the mAb to a cell which expresses both a T cell antigen receptor and CD3 complex. The CD3 comodulation can be detected, for example, by measuring the amount of labeled anti-CD3 antibody which is bound by the cell. This method is illustrated by way of example in Section 7.1.1, infra, in which it is used to identify hybridomas secreting anti-V_{δ} mAb 8TCAR-3.

A molecular clone of an antibody to an epitope of a y or δTCR polypeptide can be prepared by known techniques. Recombinant DNA methodology (see e.g., Maniatis et al., 1982, Molecular Cloning, A Laboratory Manual, Cold Spring Harbor Laboratory, Cold Spring Harbor, New York) may be used to construct nucleic acid sequences which encode a monoclonal antibody molecule, or antigen binding region thereof.

Antibody molecules may be purified by known techniques, e.g., immunoabsorption or immunoaffinity chromatography, chromatographic methods such as HPLC (high performance liquid chromatography), or a combination thereof, etc.

Antibody fragments which contain the idiotype of the molecule can be generated by known techniques. For example, such fragments include but are not limited to: the F(ab')₂ fragment which can be produced by pepsin digestion of the antibody molecule; the Fab' fragments which can be generated by reducing the disulfide bridges of the F(ab')₂ fragment, and the Fab fragments which can be generated by treating the antibody molecule with papain and a reducing agent.

One embodiment of the invention is directed to monoclonal antibodies reactive with the variable region of the δTCR chain. Such an antibody is δTCAR-3 (aka TCS81) (see Section 7, infra), which recognizes an epitope expressed from a specific δ gene rearrangement. As described in Section 7.2, infra, mAb δTCAR-3 is capable of stimulating the proliferation of a y,8⁺ T lymphocyte. Monoclonal antibody δTCAR-3 is also able to stimulate a rise in cytoplasmic free calcium ion concentration of y,8⁺ T lymphocytes.

In another embodiment, the invention relates to antibodies reactive with the constant region of the y or δTCR polypeptide. In a specific embodiment, the invention is directed to mAb TCR81 which is reactive with the constant region of the δTCR chain (see Section 6, infra). In another specific embodiment, the invention relates to mAb anti-Cyml, which is reactive with the constant region of the yTCR chain (see Section 8.1.7, infra).

### 6. GENERATION OF MONOCLONAL ANTIBODY ANTI-TCRδ1 SPECIFICALLY REACTIVE WITH THE TCR DELTA SUBUNIT CONSTANT REGION

### 6.1. EXPERIMENTAL PROCEDURES

### 6.1.1. CYTOFLUOROGRAPHIC ANALYSIS OF T CELL LINES WITH ANTI-TCR81

The anti-TCR81 mAb, which is specifically reactive with the δTCR chain constant region, was made as follows: One gram of PEER cells were solubilized in 50 ml of 0.3% CHAPS (3-[3-cholamidopropyl)dimethylammonio]1-propanesulfonate) detergent and were immunoprecipitated with 1 µl of UCHT1 (Beverley, P.C., and Callard, 1981, Eur. J. Immunol. 11:329-334) ascites, 500 µl of mAb 187.1 culture supernatant and Staphylococcus aureus Cowan I strain (SACI). Four intraperitoneal injections at six week intervals were carried out, followed by a final boost of γδTCR (without CD3) isolated by selective elution of γδTCR from the immune complexes using 2% Triton X-100. The eluted material was administered both intravenously and intraperitoneally; four days after this boost, the mice were sacrificed and fusion carried out as previously described (Brenner, M.B., et al., 1987, J. Immunol. 138:1502-1509).

y8TCR cell lines PEER and IDP2 or αβTCR cell lines HPB-MLT and JURKAT were stained with 50 µl of anti-TCR81 culture supernatant followed by staining with FITC-conjugated goat anti-mouse Ig F(ab)'₂ fragments with analysis on an Ortho cytofluorograph (see Fig. 1). Control was the mAb secreted by P3X63.Ag8 hybridoma (P3) and anti-CD3 mAb was anti-Leu 4 (Ledbetter, J.A., et al., 1981, J. Exp. Med. 153:310).

### 6.1.2. IMMUNOCHEMICAL ANALYSIS OF THE SPECIFICITY OF mAb anti-TCR81

Surface ¹²⁵1-labeled IDP2 cells were solubilized and their proteins immunoprecipitated using control mAb P3, anti-Leu 4, anti-TCR81, or anti-Cy serum. Precipitated samples were analyzed by SDS-PAGE followed by autoradiography. In CHAPS detergent, y8TCR and CD3 remained associated and were immunoprecipitated as a complex by anti-Leu 4 (Fig. 2, lanes 3 and 4). However, after solubilization in 2% Triton X-100 detergent, anti-TCR81 immunoprecipitated γδTCR as a dimeric complex without CD3 (land 6) and anti-Leu 4 immunoprecipitated CD3 as a trimeric complex without γδTCR (lane 5). After separation of the y8TCR-CD3 component chains, anti-TCR81 immunoprecipitated TCRδ alone (lane 7 and 8), while anti-Cy serum immunoprecipitated TCR γ alone (lane 9). For chain separation experiments (lane 7-9), anti-Leu 4 immunoprecipitates from CHAPS solubilized IDP2 cells were boiled in 1% SDS and were then diluted with 4 volumes of 2% Triton X-100 followed by immunoprecipitation with anti-TCR81 or anti-Cy serum. This follows procedures used previously (Brenner, M.B., et al., 1986, Nature 322:145).

### 6.1.3. N-LINKED GLYCOSYLATION OF THE TCR δ POLYPEPTIDE

¹²⁵l-labeled IDP2 cells were solubilized in 0.3% CHAPS, immunoprecipitated with anti-Leu 4 and resolved by SDS-PAGE (Fig. 3). Control lane is mock-digested IDP2 δTCR polypeptide. N-glycanase digestion of δTCR polypeptide was performed as follows: δTCR was eluted from a gel slice followed by N-glycanase (Genzyme Corp.) digestion (10 U/mi) carried out in 30 µl 0.17% SDS, 1.25% Nonidet P-40, 0.2 M sodium phosphate buffer pH 8.6 for 16 hours at 37°C (Tarentino, A.L., et al., 1985, Biochemistry 24:4665). The digested or mock-incubated δTCR samples were analyzed by SDS-PAGE and visualized by autoradiography.

### 6.1.4. RECOGNITION OF IN VITRO TRANSLATION PRODUCTS OF cDNA CLONE IDP20-240/38 BY mAb ANTI-TCR81

A plasmid designated pGEM3-O-240/38 was constructed as follows and used for in vitro transcription-translation (Fig. 4). The IDP2 O-240/38 (δTCR) cDNAclone 1.5 kb insert begins within codon 7 of the composite Group O sequence and includes the remaining coding region and most of the 3' untranslated region. This insert was cleaved as a single EcoRl fragment from λgt10 arms by partial EcoRl digestion (to prevent cleavage of the internal EcoRl site). This fragment was subcloned into a Bluescript+ vector (Stratagene). The insert was then removed from the vector as a single BamHI-Sall fragment (ends are from the Bluescript vector polylinker) facilitating directional cloning into pGEM-3 (Promega Biotech) downstream of the T7 promoter. The resultant pGEM3-O-240/38 plasmid was linearized with Sail and capped transcripts synthesized using T7 RNA polymerase (Krangel, M.S., et al., 1987, Science 237:64). Integrity and size of the transcripts were monitored via an aliquot of the reaction mixture containing ³²P-ATP. A single RNA species of 1.5 kb was observed. In vitro translation in the presence of³⁵S-methionine was performed in a rabbit reticulocyte lystate. After in vitro translation, the samples were boiled in 1% SDS with 2 mM dithiothreitol followed by the addition of 10 volumes of 2% Triton X-100 in Tris buffered saline pH 7.5. Samples were immunoprecipitated with control mAb P3 (Fig. 5, lanes 1 and 3) or with anti-TCR81 mAb (lanes 2 and 4) and analyzed by SDS-PAGE followed by fluorography (Bonner, W.J. and Laskey, R.A., 1974, Eur. J. Biochem. 46:83-88).

### 6.2. EXPERIMENTAL RESULTS

We have generated a monoclonal antibody (mAb), anti-TCR81, that is specifically reactive with the δTCR constant region.

The y8TCR-CD3 complex from the PEER cell line (Weiss, A., et al., 1986, Proc. Natl. Acad. Sci. U.S.A. 83:6998-7002; Brenner, M.B., et al., 1987, Nature 325:689-694) was used as immunogen in the production of antibody-secreting hybridoma cell lines. Hybridomas were screened both by cell surface binding (cytofluorographic analysis) and by immunoprecipitation of PEER cell proteins followed by SDS-PAGE analysis. Two hybridoma supernatants (5A6 and 4A1) bound to the surface of PEER cells. After subcloning, one mAb (5A6.E9) was characterized further. This mAb bound to the surface of γδTCR lymphocytes (PEER, IDP2) but failed to react with αβTCR cells (HPB-MLT, JURKAT) or with non-T leukocytes (Fig. 1 and data not shown). Although the immunogen was composed of a complex of γδTCR and CD3, the greater affinity of the mAb for y8TCR cell lines suggested the mAb was not directed against CD3 determinants.

The specificity of the mAb was determined in immunoprecipitation studies using various detergents which affect the association of the proteins comprising the receptor complex. After ¹²⁵1-labeled IDP2 cells were solubilized in CHAPS detergent, TCR_{γ} and 8, and CD3 y,8, and s subunits remained part of an associated complex immunoprecipitated by anti-CD3 antibody (Fig. 2, lanes 3, 4). However, if radiolabeled IDP2 cells were solubilized in 2% Triton X-100 detergent, γδBTCR and CD3 became largely dissociated, and the use of anti-CD3 mAb resulted in selective precipitation of CD3 (Fig. 2, lane 5). Under these latter conditions, mAb 5A6.E9 immunoprecipitated γδTCR as a heterodimer without associated CD3 (Fig. 2, lane 6). This observation provided the first direct evidence that TCRy and TCR δ exist as a non-disulfide-linked heterodimer. To determine whether mAb 5A6.E9 reacts with a yTCR chain, δTCR chain or a combinatorial determinant, immunoprecipitation of separated polypeptide chains was performed. An anti-Leu 4 immunoprecipitate from radiolabeled, CHAPS- solubilized IDP2 cells was boiled in 1% SDS to dissociate the yTCR, δTCR, and CD3 proteins. After dilution with four volumes of 2% Triton X-100, mAb 5A6.E9 specifically immunoprecipitated the 40 kD (δTCR) species (Fig. 2, lane 7). When an aliquot of the same immunoprecipitate was analyzed under reducing conditions (Fig. 2, lane 8), a dramatic shift in SDS-PAGE mobility was observed. This phenomenon is characteristic of δTCR from the IDP2 and PEER cell lines (Brenner, M.B., et al., 1987, Nature 325: 689-694). In contrast, when the separated chains were immunoprecipitated with the anti-Cy sera, the 55 kD species (yTCR), but not the 40 kD species (δTCR) was immunoprecipitated (Fig. 2, lane 9). Based on these biochemical and surface binding studies, mAb 5A6.E9 is referred to as anti-TCR81.

In addition to PEER and IDP2, anti-TCR81 also immunoprecipitated TCR δ from other γδTCR cell lines including MOLT-13 and PBL line 2. Further experiments have shown that anti-TCRδ1 reacts with a determinant encoded by a TCR δ constant (C) gene segment.

We have isolated cDNA clones from the IDP2 cell line (e.g., IDP2 O-240/38) by the subtractive approach representing a gene which encodes the TCR δ subunit. Genes to which IDP2 group O cDNA clones hybridize in Southern blotting experiments are expressed and rearranged in γδTCR lymphocytes but are typically not expressed (and are often deleted) in αβTCR cells. By sequence comparison with other TCR genes, these cDNA clones appear to be composed of novel V, D (?), J, and C gene segments. The IDP2 Group 0 composite DNA sequence contains a long open reading frame predicting a polypeptide with two potential asparagine-linked glycosylation sites and a molecular weight of 31.3 kilodaltons. To determine the molecular weight of the unglycosylated δTCR protein and the number of asparagine-linked carbohydrates that are present on the mature IDP2 δTCR polypeptide, gel purified δTCR was either treated with N-glycanase or mock-incubated and analyzed by SDS-PAGE (Fig. 3). Removal of N-linked carbohydrates resulted in a 5 kD decrease in apparent mo- lecularweight (40 kD to 35 kD), suggesting the presence of two (2.5-3 kD) N-linked glycans on the IDP2 δTCR. This correlates well with the number of N-linked glycans predicted by the translated amino acid sequence in Figure 5. The apparent molecular weight of the protein is in general agreement, differing from that predicted by 3.7 kD.

Given the reactivity ofanti-TCR81 on IDP2 cells, the specificity for the δTCR polypeptide, and the recognition of partially denatured (SDS boiled) δTCR, we tested whether this mAb would recognize directly polypeptide encoded by the δTCR cDNA clone. Thus, the insert from cDNA clone IDP2 O-240/38 was subcloned into the pGEM-3 expression vector downstream of the T7 promoter (Fig. 4). Transcripts generated in vitro with T7 RNA polymerase were then used in a rabbit reticulocyte lysate system to direct the synthesis of protein in the presence of ³⁵S-methionine. Following in vitro transcription-translation, the reaction mixtures were boiled in 1% SDS, diluted with ten volumes of 2% Triton X-100, and then immunoprecipitated with either an isotype- matched control mAb or with anti-TCR81. Anti-TCR81 mAb specifically immunoprecipitated a predominant species (34 kD) (Fig. 5, lane 4). No such band was observed in immunoprecipitates when control mAbs were used (lane 3), when RNAtranscripts were omitted (lanes 1 and 2), or when yTCR constructs were used. Thus, the radiolabeled species immunoprecipitated by mAb anti-TCR81 corresponds to a δ polypeptide whose synthesis was specifically directed by the IDP2 O-240/38 cDNA clone. This polypeptide (34 kD) is very similar in size to the N-glycanase treated IDP2 δTCR chain (35 kD). The IDP2 O-240/38 clone lacks a natural ATG initiation codon as well as the leader sequence. There are two potential internal ATG codons (at residues 12 and 44) within the V region of this clone (Fig. 5). Use of these codons to initiate synthesis could result in more than one polypeptide species possibly accounting for the minor species noted (Fig. 5, lane 4). Thus, there is direct serological recognition by mAb anti-TCR81 of the IDP2 δTCR subunit encoded by clone IDP2 O-240/38.

### 7. GENERATION OF MONOCLONAL ANTIBODY δTCAR-3 SPECIFICALLY REACTIVE WITH THE TCR DELTA SUBUNIT VARIABLE REGION

### 7.1. EXPERIMENTAL PROCEDURES

### 7.1.1. IMMUNOPRECIPITATION AND SDS-PAGE ANALYSIS OF T CELL ANTIGEN RECEPTOR

The δTCAR-3 mAb, specifically reactive with the variable region of the δTCR chain, was generated as follows: One mouse was immunized with 2 x 10⁷ Molt-13 cells by intraperitoneal injection. One month later, the mouse was boosted with 1 x 10⁷ Molt-13 cells by intravenous injection each day for 3 sequential days, and then immune splenocytes were fused with mouse myeloma P3x63Ag8.653 cells in the presence of 50% polyethylene glycol 1500. The hybridomas were screened by analyzing the CD3 co-modulation with flow cytometry. The analysis of CD3 co-modulation was based on the observation that antibody to α,β T cell antigen receptor, when incubated with the cells, caused the internalization of the CD3 complex (Lanier, L.L., et al., 1986, J. Immunol. 137:2286; Meuer, S.C., et al., 1983, J. Exp. Med. 157:705).

Molt-13, PEER, and HPB-ALL cell lines were iodinated using the lactoperoxidase technique. The ¹²⁵l-βF1 labeled cells were solubilized in Tris-buffered saline (pH 8) containing 1% Triton X-100. Lysates were immunoprecipitated using δTCAR-3 antibody or βF1 antibody. βF1 is a framework monoclonal antibody to the βTCR chain and is described elsewhere (Brenner, M.B., et al., 1987, J. Immunol. 138:1502-1509). All samples were analyzed by SDS-PAGE under reducing or non-reducing conditions (Fig. 6). Molt-13 and PEER are both CD3⁺4-8-WT31-. HPB is CD3⁺4⁺8⁺WT31⁺.

As shown in Figure 6, δTCAR-3 immunoprecipitated non-disulfide-linked y and δ chains from Molt-13 and PEER cells, while βF1 immunoprecipitated disulfide-linked a and β chains from HPB-ALL cells. The difference in autoradiographic intensity between the bands corresponding to the δ and y chains represent differences in the extent of iodination of these two proteins.

### 7.1.2. IMMUNOPRECIPITATION OF δTCR CHAIN BY δTCAR-3 ANTIBODY

Figure 7 shows ¹²⁵1-labeled Molt-13 cells solubilized in Tris-buffered saline (pH 8) containing 0.3% CHAPS (3-[(3-cholamidopropyl)dimethylammoni]1-propanesulfonate) or in 1% Triton X-100. In 1% Triton X-100, the γBTCR dissociates from the CD3 complex, while in 0.3% CHAPS, the γδTCR remains associated with the CD3 complex. Prior to immunoprecipitation, the ¹²⁵1-labeled lysates used in lanes 3, 4, and 7 of Figure 7 were denatured by adding SDS to a final concentration of 1% followed by heating for 5 minutes at 68°C. After cooling, iodoacetamide was added to a final concentration of 20 mM. The mixture was then diluted with 4 volumes of 1.5% Triton X-100 in Tris-buffered saline (pH 8). This denaturing process completely dissociates y chain, δ chain, and CD3 proteins from one another. All samples were analyzed by SDS-PAGE under non-reducing conditions (N) except for the sample in lane 4 which is under reducing conditions (R). Note the difference in mobility of δ chain under reducing and non-reducing conditions. The anti-Cy antiserum was generated by immunizing a rabbit with a 20 amino acid synthetic peptide from the y constant region (residues 117-136).

### 7.1.3. ANALYSIS OF CELL SURFACE STAINING BY FLOW CYTOMETRY

5 X 10⁵ cells were incubated with the appropriate antibodies (NMS (normal mouse serum), δTCAR-3, OKT3, or WT31) at 4°C for 30 minutes and then washed two times with 0.2% BSA in PBS (pH 7.4). Following incubation with fluorescein-conjugated goat anti-mouse IgG for 30 minutes at 4°C, cells were analyzed on an Ortho cytofluorograph (Fig. 8).

### 7.1.4. TWO COLOR CYTOFLUOROGRAPHIC ANALYSIS OF δTCAR-3⁺ AND OKT3⁺PERIPHERAL BLOOD LYMPHOCYTES

The peripheral blood lymphocytes were first incubated with δTCAR-3 at 4°C for 30 minutes. Afterwashing, cells were incubated with phycoerythrin (PE)-conjugated goat anti-mouse IgG for an additional 30 minutes at 4°C. After washing, the cells were incubated with fluorescein (FITC)-conjugated OKT3 for 30 minutes at 4°C and then cells were analyzed on an Ortho cytofluorograph (Fig. 9).

### 7.1.5. MEASUREMENT OF INTRACYTOPLASMIC Ca2+ CONCENTRATION ([Ca²⁺]i) VERSUS TIME

Molt-13 cells were labeled with the acetoxymethyl esterform of the Ca2+⁻sensitive probe fura-2 (2 µM from a 1 mM stock in dimethyl sulfoxide, Molecular Probes, Eugene, Oregon) at a concentration of 10⁷ cells/ml in RPMI 1640 plus 10% fetal bovine serum for 30 minutes at 37°C. Cells were then washed and resuspended at 10⁷ cells/ml in Hanks balanced salt solution (HBSS) plus 5% fetal bovine serum and kept in the dark at room temperature until use. Immediately prior to fluorescent measurement, 2 x 10⁶ cells were centrifuged then resuspended in 2 ml of fresh HBSS and placed in a quartz cuvette at 37°C and constantly stirred. Fluorescence was measured on the cell suspension in a SPF-500C fluorometer (SLM Aminco, Urbana, Illinois), the excitation wavelength alternating between 340 (±2) and 380 (±2) nm and emission was detected at 510 (±5) nm. The ratio of 350/380 was automatically calculated (1 ratio every 2 seconds), plotted, and stored in an IBM PC AT. Quantitation of [Ca²⁺]ᵢ from the fluorescence ratio was performed as described by Grynkiewicz, et al. (1985), J. Biol. Chem. 260:3440). Addition of irrelevant antibodies did not alter [Ca²⁺]ᵢ, while cell lysis resulted in [Ca²⁺]ᵢ of 1 µM.

### 7.2. RESULTS

We have generated a monoclonal antibody, δTCAR-3, that is directed against a variable region of the TCR δ chain and which can be used to characterize the δ polypeptide. This monoclonal antibody binds to T cells bearing the y8TCR and also elicits a fura-2 Ca2+ signal upon binding to Molt-13 cells.

The δTCAR-3 monoclonal antibody was generated by immunizing a mouse with the Molt-13 cell line which has a CD3⁺4⁻8⁻WT31⁻ phenotype. The hybridomas were first screened by CD3 co-modulation. The positive clones were further screened by immunoprecipitation. δTCAR-3 immunoprecipitation of γδTCR heterodimer from ¹²⁵1-labeled Molt-13 and PEER lysates is shown in Figure 6. δTCAR-3 does not immunoprecipitate any polypeptide from HPB-ALL (Fig.6). In contrast, βF1, a framework monoclonal antibody specific to the β chain (Brenner, M.B., et al., 1987, J. Immunol. 138:1502-1509), immunoprecipitates the αβ heterodimer from the HPB-ALL cell line (Fig. 6, lanes 10 and 12). The immunoprecipitated y8 receptor from both Molt-13 and PEER cells, when analyzed under either reducing or non-reducing conditions, displays a heterodimeric structure indicating a non-disulfide-linked γδTCR in these two cell lines. There is a slight shift in mobility of the δ chain under reducing conditions relative to that observed under non-reducing conditions (Fig. 6, lanes 1 and 3, 5 and 7), a phenomenon which has been noted previously in IDP2 and PEER cell lines (Brenner, M.B., et al., 1987, Nature 325:689-694), suggesting the existence of intrachain disulfide linkages. In order to demonstrate that the δTCAR-3 antibody recognizes a CD3-associated γδTCR, immunoprecipitations were performed using ¹²⁵l-labeled Molt-13 cell lysates solubilized in 0.3% CHAPS detergent (Fig. 7, lane 1). Under these conditions, the CD3 complex remains associated with the receptor, and both y8 heterodimer and the CD3 complex are immunoprecipitated by δTCAR-3. However, when ¹²⁵1-labeled lysates were solubilized in 1% Triton X-100 detergent which largely dissociates the CD3 complex from the y8 receptor, only y8 heterodimer is immunoprecipitated by δTCAR-3 (Fig. 7, lane 2). As a control, the anti-CD3 antibody, UCHT-1 (Beverley, P.C. and Callard, R.E., 1981, Eur. J. Immunol. 11:329-334) immunoprecipitates only the CD3 complex, but not the y8 heterodimer (Fig. 7, lane 5).

The specificity of δTCAR-3 was further analyzed by using immunoprecipitations of denatured, ¹²⁵1-labeled Molt-13 lysates in which y,8TCR and CD3 proteins were completely dissociated. δTCAR-3 specifically immunoprecipitated the δ chain which has an apparent molecular weight of 38 kD under non-reducing conditions (Fig. 7, lane 3) and 40 kD under reducing conditions (lane 4). The anti-Cy antiserum immunoprecipitated the y chain with molecular weight 42 kD under reducing conditions (Fig. 7, lane 7). These data indicate that δTCAR-3 is δ chain specific.

δTCAR-3 not only immunoprecipitates y,8TCR heterodimer from the PEER and Molt-13 cell lines, it also binds to the surface of these cell lines and to the IDP2 clone (Brenner, M., et al., 1987, Nature 325:689-694). It does not bind to the αβTCR-bearing HPB-ALL and Jurkat cell lines (Fig. 8). In contrast, WT31 (Tax, W.J.M., et al., 1983, Nature 304:445-447), a framework monoclonal antibody to the αβTCR, reacts with αβTCR- positive HPB-ALL and Jurkat cell lines, but not with y8TCR-positive Molt-13, PEER, and IDP2 cells (Fig. 8). When normal peripheral blood lymphocytes (PBL) were examined, a subpopulation (0.9-2.4%) of CD3⁺ lymphocytes were positive with δTCAR-3 (Fig. 9). When δTCAR-3, immobilized on tissue culture plates was used for culture of normal human PBL, it selectively stimulated the proliferation of the γδTCR-positive subpopulation. After 45 days in culture, the y8TCR subpopulation represented 96% of the total cell count.

Antibodies to the αβ T cell antigen receptor stimulate a rise in the cytoplasmic free calcium ion concentration [Ca²⁺]ᵢ (Weiss, A., et al., 1986, Ann. Rev. Immunol. 4:593). Incubation of Molt-13 cells with δTCAR-3 elicited a rapid increase in [Ca²⁺]ᵢ similar to the response induced by anti-T3 antibodies (Fig. 10). Moreover, δTCAR-3 similarly stimulated a Ca²⁺flux in PEER cells and in the y8TCR-positive cell line generated from PBL as described above. We have also observed that incubation of Molt-13, PEER, and IDP2 cells with δTCAR-3 causes the co-modulation of the CD3 protein complex.

Further characterization of the epitope specificity of mAb δTCAR-3 (also termed mAb TCS81) is presented in Section 11.2.2, infra.

### 8. THREE FORMS OF THE HUMAN T CELL RECEPTOR y8: PREFERENTIAL USE OF ONE FORM IN SELECTED HEALTHY INDIVIDUALS

In the examples herein, the structure of a new form of the human T cell receptor y8 (y8TCR), consisting of a 40 kD TCR y glycoprotein noncovalently associated with a TCR 8 chain, is presented. The newly identified yTCR glycoprotein, termed Form 2bc, differs in size by more than 15 kD (40 kD versus 55 kD) compared to the previously described nondisulfide-linked TCR y form (Form 2abc). This difference is accounted for by a 5 kD smaller polypeptide backbone size (35 kD versus 40 kD) and by a reduction in the amount of carbohydrates (5 kD versus 15 kD). Nucleotide sequence analysis of cDNA clones corresponding to Form 2bc revealed that Form 2bc cDNA clones lacked one copy of the constant region (Cy2) second exon that is present in the cDNA of the other nondisulfide-linked TCR y subunit (Form 2abc). This CII exon copy encodes part of a connector region between the membrane spanning region and the extracellular constant domain. Since the number and localization of the potential N-linked carbohydrate attachment sites is the same in both nondisulfide-linked forms, we conclude that the connector region influences the amount of attached carbohydrates, probably by affecting the conformation of the protein. In contrast, the δTCR subunits of these γδTCR forms show little variability in peptide backbone sizes or peptide mapping analyses.

We also examined the usage of the three forms of the γδBTCR complex in peripheral blood. Nearly exclusive use of the disulfide-linked form, Form 1, was observed in certain healthy subjects. In some individuals, Form 1 was expressed together with Form 2bc. Form 2abc was not identified in the subjects tested.

### 8.1. EXPERIMENTAL PROCEDURES

### 8.1.1. ANTIBODIES

Monoclonal antibodies used were anti-Leu4 (anti-CD3) (Ledbetter et al., 1981, J. Exp. Med. 153:310-323), βF1 (anti-βTCR) (Brenneretal., 1987, J. Immunol. 138:1502-1509), anti-TCR81 (anti-δTCR) (described in Section 6, supra; reactive with the δTCR chain constant region), P3 (control) (secreted by P3X63.Ag8; Koehler and Milstein, 1975, Nature 256: 495-497), 187.1 (rat anti-mouse _{K} light chain) (Yelton et al., 1981, Hybridoma 1:5-11), and WT31 (stains αβTCR lymphocytes brightly) (Spits et al., 1985, J. Immunol. 135:1922-1928). Anti-Cyb peptide serum (anti-yTCR) was generated against a 22 amino acid synthetic peptide (Gln-Leu-Asp-Ala-Asp-Val-Ser-Pro-Lys-Pro-Thr-Ile-Phe-Leu-Pro-Ser-Ile-Ala-Glu-Thr-Lys-Cys) (PCT International Publication No. WO 88/00209, published January 14, 1988).

### 8.1.2 CELL LINES

PEER (Weiss et al.,. 1986, Proc. Natl. Acad. Sci. U.S.A. 83:6998-7002) and Molt-13 (isolated by J. Mino- wada, Loh et al., 1987, Nature 330:569-572) are T leukemic cell lines. Umbilical cord blood derived clone WM-14 (Alarcon et al., 1987, Proc. Natl. Acad. Sci. U.S.A. 84:3861-3865) and peripheral blood derived cell line IDP2 (Brenneret al., 1986, Nature 322:145-149; PCT International Publication No. WO 88/00209, published January 14, 1988) and thymus-derived Clone II (Bank et al., 1986, Nature 322:179-181) were cultured as described earlier. Peripheral blood derived cell line 2 (PBL-L2) was isolated by sorting peripheral blood isolated lymphocytes that did not stain with mAb WT31. The isolated cells were then expanded in vitro in RPMI 1640 medium supplemented with 10% (v/v) conditioned medium containing IL-2 and 10% (v/v) human serum, and stimulated every 3 weeks with irradiated autologous feeder cells.

### 8.1.3 IODINATION AND IMMUNOPRECIPITATION

2 x 10⁷ cells were isolated by Ficoll-diatrizoate (Organon Teknika Corp.) centrifugation and iodinated on ice in 0.5 ml of phosphate-buffered saline, pH 7.4 (PBS) containing 1 mM MgC1₂, 5 mM glucose by adding 100 µg of lactoperoxidase (80-100 U/mg, Sigma) and 1 mCi of Na¹²⁵1 (New England Nuclear). Ten µl of a 0.03% hydrogen peroxide solution was added at 5 minute intervals over a reaction period of 30 minutes. Cells were solubilized overnight in detergent supplemented TBS (50 mM Tris-Base pH 7.6, 140 mM NaCI) containing 1 mM phenylmethylsulfonyl fluoride (PMSF, Sigma) and 8 mM iodoacetamide (IAA, Sigma). As indicated, different detergents used in this study were 0.3% (w/v) 3-[(3-cholamidopropyl) dimethylammonio] 1-propane-sulfonate (CHAPS, Signma). 1% (w/v) digitonin (Aldrich) and Triton X-100 (TX-100, Sigma). After 20 minutes of centrifugation at 10,000 x g to remove insoluble material, detergent lysates were precleared by a 30 minute incubation with 4 µl of normal rabbit serum (NRS) and 400 µl of 187.1 hybridoma culture medium, followed by addition of 200 µl of a 10% (w/v) cell suspension of fixed Staphylococcus aureus Cowan I (Pansorbin, Calbio- chem). After a one-hour incubation, Pansorbin was removed by centrifugation. Specific precipitations were carried out by adding 0.25 µl βF1 ascites, 1 µl 1 mg/ml anti-Leu4 or 0.25 µl P3 ascites, together with 150 µl of 187.1 culture supernatant to each sample, followed by a one-hour incubation. 100 µl of 10% (v/v) Protein A-Sepharose (Pharmacia) was added and the mixture was rocked for 1 hour at 4°C. Immunoprecipitates were washed five times with 0.1% (v/v) Triton X-100 containing TBS and analyzed by sodium dodecyl sulfate-polyacrylamide gel electrophoresis (SDS-PAGE) (Laemmli, 1970, Nature 227:680-685).

For immunoprecipitations with the anti-Cyb peptide serum, iodinated cells were solubilized in 1% (w/v) sodium dodecyl sulfate (SDS) containing TBS and then boiled for 3 minutes. After cooling, 5 volumes of 2% (v/v) Triton X-100 in TBS containing PMSF and IAA was added, together with 200 µl of a mixture of 1 mg/mi deoxyribonuclease (DNAse) and 0.5 mg/ml RNAse in 50 mM MgCl₂. Preclearing and immunoprecipitations were performed as described above, omitting the addition of 187.1 mAb. Immunoprecipitates were washed in TBS containing 0.5% (v/v) TritonX-100, 0.5% (w/v) deoxycholate (DOC), 0.05% (w/v) SDS. Triton and Sepharose are trade marks.

### 8.1.4. BIOSYNTHETIC LABELLING

4 x 10⁷ exponentially growing cells were resuspended in 4 ml of methionine and cysteine-free RPMI 1640 (Select-Amine kit, Gibco) supplemented with 10% dialyzed fetal calf serum (FCS) and 20 mM Hepes. After a 30 minute starvation period at 37°C, 1 mCi of ³⁵S-methionine and 1 mCi of ³⁵S-cysteine were added, allowing a 15 minute labelling period. Cells were harvested and solubilized in 2% (v/v) Triton X-100, TBS. Preclearing and immunoprecipitations were performed as described above. The immunoprecipitates were washed four times in 0.5% (v/v) Triton X-100, 0.5% (w/v) deoxycholic acid, 0.05% (w/v) SDS, TBS followed by three washes in 0.5% (v/v) Triton X-100, 0.5 M NaCI, 5 mM EDTA, 50 mM Tris, pH 7.6. The samples were analyzed by SDS-PAGE and visualized by standard fluorography procedures (Bonner and Laskey, 1974, Eur. J. Biochem. 46:83-88).

### 8.1.5. GEL PURIFICATION OF δTCR PROTEINS

Surface iodinated cells were solubilized in 0.3% (w/v) CHAPS-TBS and immunoprecipitated using 50 µl of anti-Leu4-coupled Sepharose beads. The immunoprecipitated species were resolved by SDS-PAGE under nonreducing conditions and the wet gel was exposed for 24 hours at 4°C on XAR-5 film (Kodak) to visualize radiolabelled 8TCR proteins. The gel regions corresponding to 8TCR were excised, incubated in 5% (v/v) 2-mercaptoethanol containing sample buffer and resolved a second time by SDS-PAGE. Because of the characteristic SDS-PAGE mobility shift upon reduction, δTCR protein could be separated and then purified from contaminants. TCR proteins were eluted from gel slices by overnight incubation in 0.05% (w/v) SDS, 50 mM ammonium bicarbonate buffer at 37°C and lyophilized.

### 8.1.6. ENDOGLYCOSIDASE DIGESTION

Forendoglycosidase H (Endo H) digestions, immunoprecipitated material orgel purified protein was boiled for 3 minutes in a 40 µl of 1% (w/v) SDS solution containing 0.14 M 2-mercaptoethanol. After cooling, the mixture was diluted with 360 µl of 0.15 M acetate buffer, pH 5.5 containing 1 mM PMSF. Five µl Endo H (1 U/ml-Endo-β-N-acetylglucosaminidase H, Genzyme) was incubated with half of the above solution for 14 hours at 37°C, while the other half was mock treated.

For N-glycanase (N-GLY) digestion, gel purified material was boiled for 3 minutes in 35 µl of 0.5% (w/v) SDS, 0.10 M 2-mercaptoethanol. Then, 100 µl of 0.2 M sodium phosphate (pH 8.6), 1.25% (v/v) Triton X-100 was added. Half of the mixture was incubated with 1 µl of N-Glycanase (250 U/ml, peptide-N-[N-acetyl-β-glu- cosaminyl]asparagine amidase; Genzyme) and incubated for 16 hours at 37°C, while the other half was mock treated.

After digestion, 10 µg bovine serum albumin was added as carrier and samples were recovered by trichloroacetic acid precipitation. Protein pellets were taken up in sample buffer containing 5% (v/v) 2-mercaptoethanol.

### 8.1.7. PRODUCTION OF MONOCLONAL ANTIBODY anti-Cym1

Part of the Cy CI and CII exons of HPB-MLT pTy-1 was isolated using the BamHl and Pstl sites at nucleotide positions 571 and 848 (Dialynas et al., 1986, Proc. Natl. Acad. Sci. USA 83:2619-2623) and was cloned into expression vector pRIT2T (Pharmacia). The resulting Protein A fusion protein was expressed in E. coli N4830. Bacteria were lysed with lysozyme and the fusion protein was isolated by purification over a IgG Sepharose column. Mice were injected intraperitoneally with 100 µg of fusion protein in Freund's adjuvant at days 0, 7 and 28. Twenty-eight days later 100 µg of fusion protein in PBS was injected intravenously. After three days, splenocytes were isolated and fused with the hybridoma P3X63Ag8.653 as described (Brenner et al., 1987, J. Immunol. 138:1502-1509). Hybridomas were screened by enzyme-linked immunoabsorbent assay (ELISA). Ninety six-well flat bottom plates (LINBRO, Flow Laboratories) were incubated overnight with 0.4 µg of fusion protein or nonfused protein in PBS. Nonspecific binding sites were blocked at 23°C with 0.25 mg/ml normal rabbit IgG (Sigma) in PBS containing 50% (v/v) FCS. 50 µl of hybridoma supernatant was added for 1 hour at 4°C, followed by a similar incubation in 50 µl of a 5 µg/ml solution of peroxidase-conjugated anti-mouse IgG (Cappel). All described incubations were interspersed with washing steps, using 10% (v/v) FCS, 0.1% (w/v) BSA, PBS. The ELISAwas developed with 0.08% (w/v) O-phenylenediamine (Sigma) in 0.012% (w/v) hydrogen peroxide containing phosphate-citrate buffer, pH 5.0.

Although anti-Cyml (IgG₁) does not recognize the native y8TCR/CD3 complex in cytofluorographic analysis nor the y8TCR heterodimer from Triton X-100 solubilized cells in immunoprecipitation, it does recognize biosynthetically labelled yTCR precursor and mature yTCR proteins after separation of CD3/y8TCR proteins into individual chains. In this way, anti-Cyml was shown to recognize the yTCR protein after separating CD-3/y8TCR complexes into individual chains by boiling anti-CD3 immunoprecipitates in 1 % (w/v) SDS in TBS (Fig. 12, lane 3).

### 8.1.8. ISOLATION AND SEQUENCING OF A MOLT-13 yTCR cDNA CLONE

Poly (A)+ RNA was prepared from MOLT-13 cells by urea/lithium chloride precipitation followed by oligo (dT) cellulose affinity chromatography. A λgt 10 cDNA library was prepared from poly(A)⁺ RNA by the method of Huynh et al., 1985 (DNA Cloning, Glover, D.M. ed. IRL Press, Oxford, 1:49-78) using Mung Bean Nuclease for the hairpin loop cleavage (McCutcham et al., 1984, Science 225:626-628). The cDNA library was amplified on the E. coli strain C600 Hf1 and screened by plaque filter hybridization with ³²P-labelled PTyl (Dialynas et al., 1986, Proc. Natl. Acad. Sci. U.S.A. 83:2619-2623). Positive clones were analyzed for size and restriction enzyme map, and cDNA clone M13k was selected for sequencing. The cDNA of M13k was excised from λgt 10 phage with the endonuclease EcoRl and further digested with appropriate restriction enzymes. The fragments were subcloned into M13 vectors and sequenced by the dideoxy chain termination method (Sanger et al., 1977, Proc. Natl. Acad. Sci. U.S.A. 74:5463-5467) using the modified T7 polymerase (Sequenase, United States Biochemical Corp.).

Clone M13k corresponds to a full length, in frame, yTCR transcript, including 36 nucleotides of 5' untranslated region and 72 nucleotides of 3' noncoding region (Fig. 14). The nucleotide sequence of the V region is identical to the genomic Vy1.3 sequence (nomenclature Lefranc et al., 1986a, Cell 45:237-246; Strauss et al., 1987, Science 237:1217-1219), except for a C to T (lie to Val) change of nucleotide 53 in the putative signal sequence. The J region is identical to the Jy2.3 sequence (nomenclature based on Lefranc et al, 1986b, Nature 319:420-422; Quertermous et al, 1987, J. Immunol. 138:2687-2690). Interestingly, 8 nucleotides occur at the V-J junction which do not appear to be encoded by the genomic V or J sequences and presumably represents an N-region. The C region sequences match the corresponding genomic sequence (Lefranc et al., 1986c, Proc. Natl. Acad. Sci. U.S.A. 83:9596-9600), with the exception of nucleotide 559 (G to C; Val to lie) and nucleotide 908 (T to C; Met to Thr).

### 8.2. RESULTS

### 8.2.1. NOVEL γδTCR PROTEIN COMPLEX

Preliminary studies of peripheral blood γδTCR lymphocytes revealed the presence of a CD3-associated complex that was different from the known human γδTCR forms. In an attempt to delineate this form, we produced and characterized a number of cell lines derived from normal human donors. Peripheral blood lymphocytes were stained with monoclonal antibody (mAb) WR31, which brightly stains resisting αβTCR lymphocytes. Cells that did not stain were isolated by cell sorting and then expanded in vitro in IL-2 containing medium. Peripheral blood lymphocyte line 2 (PBS-L2) obtained in this way, proved to be homogeneously CD3⁺CD4⁻CD8⁻, a cell surface phenotype characteristic of γδTCR lymphocytes.

To visualize γδTCR complexes on PBL-L2 cells, immunoprecipitations with an anti-CD3 mAb were carried out from cell surface ¹²⁵1-labelled cells solubilized in CHAPS or digitonin. In these detergents, the physical association between the CD3 complex and γδTCR subunits is preserved. SDS-PAGE of anti-CD3 immunoprecipitates from PBL-L2 cells resolved 40 kD and 44 kD proteins (referred to as 40 kD) that were identified as yTCR subunits by anti-Cyb serum, an antiserum directed against a yTCR constant region peptide (Fig. 11A; see methods section).

These yTCR proteins on PBL-L2 are noncovalently associated with a δTCR subunit, which is visible as a weakly iodinated protein in the anti-CD3 immunoprecipitate analyzed under nonreducing conditions (Fig. 11A, lane 6, closed arrow). This weakly iodinated protein represents the δTCR subunit on PBL-L2 cells, since it is not recognized by anti-Cyb serum (Fig. 11A, lane 8). In addition, it displays the same SDS-mobility shift comparing analysis under nonreducing and reducing conditions as was noted for the δTCR proteins on IDP2 and PEER cells (see infra; see also PCT International Publication No. WO 88/00209, published January 14,1988). The δTCR protein could not be visualized after reduction (Fig. 11A, lane 3), because it migrated with a mobility of 40 kD (see infra) and then was obscured by the similar sized yTCR protein (open arrow).

This y8TCR form is not only present on normal peripheral blood T lymphocytes, but is also observed on thymus-derived Clone II cells (Fig. 11D) and on the T-leukemic cell line MOLT-13 (Fig. 11 E). These three cell lines possess yTCR species that display differential glycosylation resulting in a yTCR protein doublet observed on PBL-L2 (40 kD and 44 kD; Fig. 11A, lane 8) and Clone II cells (40 kD and 44 kD. 11 D, lane 8) or a diffusely labelled yTCR protein band observed on MOLT-13 cells (40 to 46 kD; Fig. 11 E, lane 6). Two-dimensional gel analysis [nonequilibrium pH gradient electrophoresis (NEPHGE) followed by SDS-PAGE] of the MOLT-13 yTCR protein band resolved two parallel yTCR species (40 kD and 44 kD), of which the 44 kD yTCR species contained an additional high mannose (or hybrid) N-linked glycan compared to the 40 kD yTCR species. Thus, the yTCR subunits of this receptor complex isolated from three different cell sources (peripheral blood, thymus, and leukemia), revealed cell surface species of 40 kD that are noncovalently associated with yTCR partner chains.

For comparison to the γδTCR form on PBL-L2, Clone II and MOLT-13 cells, we examined the previously known forms on the IDP2 and WM-14 cell lines. The IDP2 cell line (see PCT International Publication No. WO 88/00209, published January 14, 1988; Brenner et al., 1986, Nature 322:145-149) contains a larger, 55-60 kD yTCR protein (referred to as 55 kD), which is recognized by anti-Cyb serum (Fig. 11 B). When the anti-CD3 immunoprecipitate is examined under nonreducing conditions, it is evident that the IDP2 yTCR protein is associated noncovalently with its yTCR partner chain (Fig. 11 B, lane 4, solid arrow). Upon reduction, the 8TCR protein displays a decrease in SDS-PAGE mobility to a relative molecular mass of 40 kD (compare Fig. 11 B, lane 4, closed arrow, with Fig. 11 B, lane 2, open arrow).

In contrast to the noncovalently associated γδTCR forms, the peripheral blood-derived T cell clone, WM-14, bears a disulfide-linked TCR dimer of 70 kD (Fig. 11C, lane 7), that was recognized by anti-Cy serum (Alarcon et al., 1987, Proc. Natl. Acad. Sci. U.S.A. 84:3861-3865). This dimer is also recognized by anti-TCR81, a mAb directed against the 8TCR subunit (Fig. 11 C, lane 5), and therefore represents a γδTCR heterodimer. Analysis under reducing conditions reveals three yTCR proteins of 36 kD, 40 kD and 43 kD (referred to as 40 kD).

Thus, the CD3-associated complex on PBL-L2, Clone II and MOLT-13 cells constitutes a novel γδTCR heterodimer compared to the previously known forms, since its TCR y subunit is 40 kD (similar in size to the disulfide-linked Cyl encoded yTCR protein on WM-14 cells), yet it is not disulfide-linked to its partner chain (similar to the 55 kD, Cy2 encoded yTCR protein on IDP2 cells). To understand the molecular basis of this complex, more detailed structural analysis of its yTCR and 8TCR subunits was carried out as described infra, using the MOLT-13 cell line as an example.

### 8.2.2. CORE POLYPEPTIDE SIZE OF MOLT-13 yTCR SUBUNIT

To determine the size of the yTCR core polypeptide of MOLT-13 cells (40 kD yTCR glycoprotein), and compare it with that of PEER cells (55 kD yTCR glycoprotein), both cell lines were biosynthetically labelled for 15 minutes in the presence of³⁵S-methionine and ³⁵S-cysteine, solubilized in Triton X-100 and then immunoprecipitated with anti-Cyml, a monoclonal antibody that specifically recognizes the yTCR chain (Fig. 13A, see methods section). Immunoprecipitated material was subsequently digested with endoglycosidase H (Endo H) to remove the immature N-linked glycans. The MOLT-13 yTCR polypeptide backbone has a relative molecular mass of 35 kD (Fig. 13A, lane 8), which is 5 kD smaller than the PEER yTCR core polypeptide (40 kD; Fig. 13A, lane 4) or the IDP2 yTCR core polypeptide (40 kD; See PCT International Publication No. WO 88/00209, published January 14, 1988). It now can be concluded that MOLT-13 cells express a yTCR core polypeptide that is distinct from the IDP2 and PEER yTCR core polypeptides based on its being 5 kD smaller in size. In addition, only 5-11 kD of size on the mature MOLT-13 yTCR cell surface glycoprotein are accounted for by post-translational processes (40-46 kD surface size minus 35 kD core size), where 15-20 kD of relative molecular mass can be accounted for by post-translational processes on the PEER and IDP2 yTCR glycoproteins (55-60 kD surface size minus 40 kD core size). Assuming that all post-translational processes are N-linked glycans and that each glycan chain accounts for approximately 3 kD of relative molecular mass, we predict that 2 to 3 N-linked glycans are attached to the MOLT-13 yTCR protein, while 5 N-linked glycans are added to the polypeptides on PEER and IDP2 cells. Experiments using N-glycanase to remove N-linked carbohydrates from cell surface yTCR proteins showed that the majority of the post-translational processes that are added to the core polypeptide are indeed N-linked glycans.

### 8.2.3. PRIMARY SEQUENCE OF MOLT-13 yTCR

To understand the structure of the constant region gene segment encoding the MOLT-13 yTCR subunit, the sequence of a cDNAclone representing the MOLT-13 yTCR transcript was determined. Aλgt10 library from MOLT-13 derived poly-A+ RNAwas constructed and probed with a human yTCR cDNA clone, pTy-1 (Dialynas et al., 1986, Proc. Natl. Acad. Sci. U.S.A. 83:2619-23). Based on size and limited restriction enzyme mapping, one clone, M13k, was selected and its nucleotide sequence determined (Fig. 14). Clone M13k represents a full length, in-frame yTCR transcript, using a Vy1.3 gene segment joined to a Jy2.3 gene segment (Lefranc et al., 1986, Cell 45:237-246; Lefranc et al., 1986, Nature 319:420-422; nomenclature based on Strauss et al., 1987, Science 237:1217-1219; Quertermous et al., 1987, J. Immunol. 138:2687-2690). The constant region sequence was found to be nearly identical to a recently reported non-functional yTCR (Pellici et al., 1987, Science 287:1051-1055) and to the Cy2 genomic sequence containing two CII exon copies b and c (Lefranc et al., 1986, Proc. Natl. Acad. Sci. U.S.A. 83:9596-9600) (see methods section for detailed account). This represents the first in-frame transcript encoding a yTCR protein expressed on the cell surface that utilizes a Cy2 gene segment with two CII exon copies.

The deduced amino acid sequence of this cDNA clone predicts a polypeptide backbone size of 34.8 kD which is in good agreement with biochemical data described above. Surprisingly, six potential N-linked carbohydrate attachment sites are encoded by this transcript. Since the biochemical data suggest that only 2 to 3 N-linked glycans are attached to the polypeptide chain, it indicates that not all potential sites are used.

To reflect Cy gene segment usage, we have denoted the disulfide-linked γδTCR form expressed by PBL-C1 and WM-14 as "Form 1", since such disulfide-linked yTCR chains utilize the Cy1 gene segment (Krangel et al., 1987, Science 237:64-67). The large (55 kD), nondisulfide-linked yTCR subunit of the γδTCR form expressed on IDP2 and PEER cells is encoded by Cy2 gene segments containing three CII exon copies, namely copy a, copy b and copy c (Krangel et al., 1987, Science 237:64-67; Littman et al., 1987, Nature 326:85-88) and therefore this γδTCR form is called herein "Form 2abc". In concordance, the form characterized on MOLT-13 cells is referred to as "Form 2bc".

### 8.2.4. PREFERENTIAL Cy GENE SEGMENT USAGE

To determine the presence of these three γ,δTCR forms in freshly isolated peripheral blood we analyzed the mononuclear cells from ten healthy subjects, using biochemical analysis with mAb anti-TCR81 (described in Section 6, supra; reactive with the 8TCR constant region). This antibody reacts with the great majority, if not all γ,δTCR lymphocytes. Representative results from this panel are shown in Figure 15. In subject 1, anti-TCR81 immunoprecipitates (analyzed under nonreducing conditions) demonstrate the presence of both disulfide-linked γ,δTCR complexes as a 70 kD protein band (Form 1) and nondisulfide-linked γ,δTCR complexes as a broad 40 kD protein band (Form 2bc) (Fig. 15, lane 2). This indicates that the Cy1 and Cy2 constant regions are both used by the expressed γ,δTCR of this individual. However, the amount of Form 2bc varied among individuals. Note the smaller fraction of Form 2bc in subject 2 compared to subject 1 by comparing the intensity of the 40 kD protein bands in both individuals (compare lane 2 of subject 2 with lane 2 of subject 1). Even more strikingly, only disulfide-linked γ,δTCR complexes could be detected on the mononuclear cells of three of the ten individuals examined, even after long exposure of the autoradiographs (see subject 3). None of the analyzed individuals revealed the 55 kD, nondisulfide-linked γ,δTCR complex (Form 2abc) in peripheral blood.

### 8.2.5. CHARACTERIZATION OF THE 8TCR SUBUNIT

In contrast to the striking structural differences in size and glycosylation of the yTCR proteins, 8TCR subunits from different cell sources proved to be markedly similar. The relative molecular mass of the 8TCR glycoprotein on MOLT-13 cells was directly determined to be 40 kD using the anti-8TCR mAb (Fig. 12, lane 4), confirming that it is similar in size to the 8TCR glycoprotein on IDP2 cells (Fig. 11 B, lane 2, open arrow).

To also compare δTCR polypeptide backbone sizes, cell surface ¹²⁵1-labelled δTCR protein from MOLT-13 cells was digested with N-glycanase to remove asparagine-linked glycans (of the high mannose, hybrid, and complex-type; Tarentino et al., 1985, Biochem. 24:4665-4671; Hirani et al., 1987, Anal. Biochem. 162:485-492). The δTCR core polypeptides of MOLT-13 cells has a relative molecular mass of 35 kD (Fig. 13B, lane 4), which is similar to that of the δTCR backbone of IDP2 cells (35 Kd) (Band et al., 1987, Science 238:682-684).

In addition, digestion of cell surface ¹²⁵1-labelled MOLT-13 δTCR protein with endoglycosidase H (Endo H, removing only high mannose and certain hybrid N-glycans; Tarentino et al., 1974, J. Biol. Chem. 249:811-817; Trimble and Maley, 1984, Anal. Biochem. 141:514-522) caused a decrease in relative molecular mass of 2.5 kD, (Fig. 13B, lane 2) consistent with the presence of one carbohydrate moiety, leaving a relative mass of 2.5 kD of Endo H resistant carbohydrates attached to the polypeptide. Since there are two potential N-glycan attachment sites present in the δTCR constant domain (Hata, S., et al., 1987, Science 238:678-682; Loh et al., 1987, Nature 330:569-572), these data show that both are used, but that their N-glycans are processed differently, namely one as a high mannose N-glycan (Endo H-sensitive) and the other as a complex N-glycan (Endo H-resistant, but N-glycanase sensitive). In contrast to the different amounts of attached N-linked carbohydrate on yTCR polypeptide chains, the δTCR subunits expressed on PEER, IDP2 and MOLT-13 cells all revealed the same peptide core sizes and the presence of two N-linked glycans (Fig. 13B and data not shown).

### 8.3. DISCUSSION

In this example, three protein forms of the human yTCR glycoprotein are compared, namely the disulfide-linked 40 kD yTCR protein (Form 1), the nondisulfide-linked 55 kD yTCR protein (Form 2abc) and the nondisulfide-linked 40 kD yTCR protein (Form 2bc). All three forms are shown to be associated with a δTCR subunit. Complementary DNAsequences representing the first two γ TCR forms have been reported previously (Krangel et al., 1987, Science 237:64-67; Littman et al., 1987, Nature 326:85-88. The constant region of yTCR polypeptide Form 1 (on PBL-C1) is encoded by the Cy1 gene segment containing a single CII exon, while yTCR polypeptide Form 2abc (on IDP2 and PEER cells) utilizes the Cy2 gene segment containing CII exon copy a, copy b and copy c. The cDNA sequence corresponding to a yTCR chain of Form 2bc was shown to contain a Cy2 gene segment utilizing only two CII exon copies, namely copy b and copy c. Similarly, it seems likely that the gene structure of the yTCR connector region of Clone II and PBL-L2 (nondisulfide-linked, 40 kD yTCR protein) will also be like the MOLT-13 structure determined here, namely of Form 2bc. Since the δTCR constant region used is the same for all these forms (Hata, S., et al., 1987, Science 238:678-682; Loh, E.Y., et al., 1987, Nature 330:569-572), a complete comparison of the structures of the three γδTCR forms in man now can be made (Fig. 16).

Two Cy2 polymorphic genomic forms exist in man (Lefranc et al., Nature 319:420-422; Pellici et al., 1987, Science 237:1051-1055). The two transcript forms (Form 2abc and Form 2bc) are probably the product of these different allelic types. To date, no allelic form of yTCR polypeptides have been found in mice. We conclude that the dramatic difference in yTCR cell surface protein size between Form 2abc (55 kD) and Form 2bc (40 kD) is largely determined by the amount of attached N-link carbohydrates, most likely reflecting the number of N-linked glycans. Backbone sizes of IDP2 yTCR (Form 2abc) and MOLT-13 yTCR (Form 2bc) proteins have been measured to be 40 kD and 35 kD respectively, on the basis of SDS-PAGE, which correlates well with their predicted molecular masses of 36.6 kD and 34.8 kD respectively, calculated on the basis of cDNA sequences. It is clear that this small difference in backbone size (5 kD in SDS-PAGE), accounted for mainly by one CII exon encoded peptide of 16 amino acids, contributed to, but could not solely explain the observed difference in molecular mass between the 55 kD and 40 kD nondisulfide-linked yTCR surface forms. Form 2abc yTCR polypeptides possess 5 potential N-linked glycan attachment sites that are probably all used, in contrast to the MOLT-13 yTCR polypeptide which bears one additional potential attachment site, while carrying only 2 to 3 N-linked glycans. The reason for this limited use of potential attachment sites is unknown, but may result from the influence of the CII exon encoded peptides on the conformation of the yTCR protein. The CII exon encoded peptides and their neighboring amino acids make up a connector region between the plasma membrane and the immunoglobulin-like constant domain. This region contains most of the N-linked glycan attachment sites (Fig. 17). We conclude that the CII exon copies appear to determine the protein form not only by determining polypeptide backbone size, and by creating the ability to disulfide-link chains, but also by influencing the amount of attached carbohydrates.

δTCR complementary DNAs of IDP2 (Hata et al., 1987, Science 238:678-682), PEER (Loh et al., 1987, Nature 330:569-572) and MOLT-13 cells have been sequenced and were found to be identical, except for the diversity/N-region interspacing the variable and constant region gene segments. The δTCR protein on WM-14 cells has a relative molecular mass of 43 kD, which is similar to the δTCR protein described previously (Borst et al., 1987, Nature 325:683-688; Lanier et al., 1987, J. Exp. Med. 165:1076-1094) but is 3 kD larger than the other δTCR chains. These 43 kD δTCR proteins might indicate the presence of an additional N-linked glycosylation site in a different δ variable domain.

Structural differences comparable to those described for yTCR constant region segments have not been observed for aTCR and βTCR genes (Yoshikai et al., 1985, Nature 316:837-840; Toyonaga et al., 1985, Proc. Natl. Acad. Sci. U.S.A. 82:8624-8628; Royer et al., 1984, J. Exp. Med. 160:947-952; Kronenberg et al., 1985, Nature 313:647-653). There is possible structural similarity in the number of human CII exon repeats with the length in murine Cy regions, of which the Cy1, Cy2 and Cy4 constant regions encode for 15, 10 and 33 amino acid connector region respectively (Garman et al, 1986, Cell 45:733-742; Iwamoto et al., 1986, J. Exp. Med. 163:1203-1212). The connector regions in mouse, however, reflect a difference in the size of the relevant exon, not the multiple use of exons as is seen in Form 2abc yTCR and Form 2bc yTCR in humans. Also, the murine yTCR only exist in disulfide linked forms in contrast to the two nondisulfide linked human forms.

Importantly, the human y,8TCR forms do not appear to be used equally. In some individuals (selected for high percentages of γ,δTCR lymphocytes), a single form (Form 1) predominates, suggesting that either positive selection occurs for this form or that there is selection against other γ,δTCR forms.

### 9. THREE T CELL RECEPTOR y,8 ISOTYPIC FORMS RECONSTITUTED BY PAIRING OF DISTINCT TRANSFECTED yTCR CHAINS WITH A SINGLE δTCR SUBUNIT

As described in the example herein, the role of the yTCR polypeptide in the formation of the y8 heterodimer was explored. We examined by transfection the γδTCR complexes formed by the association of yTCR chains corresponding to the three γδTCR forms (Forms 1, 2abc, and 2bc) with a single resident δTCR chain. yTCR DNAencoding either Form 1 or Form 2abc of the yTCR polypeptide was transfected into the MOLT-13 cell line, which constitutively expresses a y8 heterodimer comprised of form 2bc yTCR polypeptide noncovalently associated with δTCR polypeptide. Transfected cells were capable of expressing, together with the γδTCR characteristic of the MOLT-13 cell line, y8 heterodimers comprised of either Form 2abc yTCR noncovalently associated with δTCR or Form 1 yTCR covalently linked to δTCR. Furthermore, the glycosylation of the transfected yTCR gene products was identical to the glycosylation of these genes in their native cell lines. Thus, the degree of glycosylation and the ability to form disulfide linkages are properties determined by the yTCR gene. yTCR constant region CII exon usage determines not only the presence or absence of disulfide linkage between TCR y and δ polypeptides, but also the amount of carbohydrate attached to the yTCR chain, which is largely responsible for the differences in size of the cell surface yTCR proteins.

### 9.1. MATERIALS AND METHODS

### 9.1.1. CELL LINES

MOLT-13, a TCR y8⁺ T leukemia cell line (Hata, S., et al., 1987, Science 238:678-682; Loh, E.Y., et al., 1987, Nature 330:569-572), and peripheral blood derived TCRγδ⁺ cell lines PBL C1 (Brenner, M.B., et al., 1987, Nature 325:689-694) and IDP2 (Brenner, M.B., et al., 1986, Nature 322:145-149) were cultured as previously described.

### 9.1.2. ANTIBODIES

The monoclonal antibodies (mAb) used were: Anti-leu-4 (anti-human CD3; IgGI) (Ledbetter, J.A. et al., 1981, J. Exp. Med. 153:310-323), anti-TCR81 (anti-human TCR8 chain constant region; IgG1) (See Section 6; Band, H., et al., 1987, Science 238:682-684), anti-Ti-_{γ}A(anti-V_{γ}2; IgG2a) (Jitsukawa, S., et al., 1987, J. Exp. Med. 166:1192-1197), anti-Cyml (anti-human yTCR contant region; see Section 8.1.7), P3 (lgG1 secreted by the P3X63Ag8 myeloma) (Koehler, G., and Milstein, C., 1975, Nature 256:495-497), and 187.1 (rat anti-mouse _{K} light chain-specific) (Yelton, D.E., et al., 1981, Hybridoma 1:5-11).

### 9.1.3. ISOLATION AND SEQUENCING OF MOLT-13 δTCR cDNA CLONES

A complementary DNA (cDNA) library prepared from MOLT-13 poly A+ RNA in the vector λgt10 (Huynh, et al., 1985, in DNA Cloning, ed. Glover, D.M. (IRL Press, Oxford), Volume 1, pp. 49-78) was screened by hybridization with ³²P-labeled human δTCR cDNA clone IDP2 O-240/38 (Hata et al., 1987, Science 238:678-682). Clones were selected for detailed analysis on the basis of size and limited restriction enzyme mapping. Nucleotide sequence was determined in M13 vectors by dideoxy chain termination method (Sanger et al., 1977, Proc. Natl. Acad. Sci. U.S.A. 74:5463-5467) using the modified T7 polymerase (Sequenace, United States Biochemical Corp.) (Potter et al., 1984, Proc. Natl. Acad. Sci. U.S.A. 81:7161-7165).

### 9.1.4. CONSTRUCTION OF EXPRESSION PLASMIDS AND TRANSFECTIONS

yTCR cDNAs (PBL C1.15 and IDP2.11r) (Krangel, M.S. et al., 1987, Science 237:64-67) were cloned into pFneo mammalian expression vector (Saito, T., et al., 1987, Nature 325:125-130; Ohashi, P., et al., 1085, Nature 316:606-609) downstream from Friend spleen focus forming virus (SFFV) long terminal repeat (LTR), as shown schematically in figure 10B. The plasmid constructs were transfected into MOLT-13 cells by electro- poration (Potter, H., et al., 1984, Proc. Natl. Acad. Sci. 81:7161-7165). Transfectants were selected and maintained in medium containing 2 mg/ml of G418 (480 µg/mg solid by bioassay; GIBCO), and cloned by limiting dilution.

### 9.1.5. IODINATION AND IMMUNOPRECIPITATION

Cell surface labeling with ¹²⁵I using lactoperoxidase, solubilization in 3-[(3-cholamidopropyl) dimethylammonio] 1-propanesulfonate (CHAPS; Sigma Chemical Co., St. Louis, MO), immunoprecipitation with various antibodies, nonequilibrium pH gradient gel electrophoresis (NEPHGE), and SDS polyacrylamide gel electrophoresis (SDS-PAGE) were performed as described (See Section 8.1.3; Brenner, M.B., et al., 1986, Nature 322:145-149; Brenner, M.B., et al., 1987, Nature 325:689-694). Specific immunoprecipitations were carried out with 1 µg anti-leu-4, 0.1 µl anti-TCR81 ascites, or 1 µl P3 ascites, together with 150 µl of 187.1 culture supernatant. For anti-T9-yA, 1 µl of ascites was used without 187.1.

### 9.1.6. BIOSYNTHETIC LABELING

Exponentially growing cells were incubated for 30 minutes in methionine- and cysteine-free medium followed by a 15 minute pulse labeling at 37°C with ³⁵S-methionine and ³⁵S-cysteine, and immunoprecipitations were carried out as described in Section 8.1.3, supra. Immunoprecipitates were either treated with endoglycosidase-H (endo-H) or were mock-incubated, separated by SDS-PAGE, and visualized by fluorography (Bonner, W.M. and Laskey, R.A., 1974, Eur. J. Biochem. 46:83-88).

### 9.2. RESULTS

We investigated the products resulting from association of structurally distinct yTCR gene products with a single δTCR protein in order to demonstrate the role of the yTCR gene, and in particular the TCR CII exons, in determining the structural differences between various yTCR isotypes. For this purpose, MOLT-13, a T leukemia cell line that expresses the 40 kD nondisulfide-linked yTCR polypeptide (Form 2bc), was used as a recipient for yTCR chain cDNA clones corresponding to the other two forms of the receptor (Forms 1 and 2abc). Complete sequences of the cDNA clones representing these yTCR chains are described in Figure 14 (Form 2bc), and in Krangel et al. (1987, Science 237:64-67) (Forms 1 and 2abc) and they are schematically represented in Figure 18A.

### 9.2.1. A SINGLE FUNCTIONAL δTCR CHAIN IS PRESENT IN THE MOLT-13 CELL LINE

δTCR gene rearrangement studies of the MOLT-13 cell line (Hata, S., et al., 1987, Science 238:678-682) suggested that only a single functional δTCR gene product was expressed in this cell line. However, to demonstrate directly that a single functional transcript for δTCR is made in MOLT-13 cells, cDNA clones cross- hybridizing with a δTCR cDNA probe (Hata, S., et al., 1987, Science 238:678-682) were isolated from a MOLT-13 cDNA library prepared in λgt10 and the sequence of selected cDNA clones was determined. This analysis revealed that MOLT-13 cells express transcripts corresponding to one functionally rearranged and one aber- rantly rearranged δTCR gene. The cDNA clone corresponding to the functionally rearranged δTCR gene has the same V(V81), J (Jδ1), and C gene segments described earlier for the IDP2 cell line (Hata, S., et al., 1987, Science 238:678-682). The MOLT-13 δTCR cDNA clone, however, possesses a distinct nucleotide sequence between the V and J gene segments arising from D segment utilization (MOLT-13 probably uses only D82), imprecise joining, and N-region diversity at the V-D and D-J junctions (Hata, S., et al., 1988, Science 240:1541-1544). The MOLT-13 δTCR cDNA also predicts a cysteine residue in the membrane proximal connector region of the constant gene segment that would be available for disulfide linkage to yTCR gene products that utilize the Cy1 gene segment. Although the MOLT-13 cell line expresses a nondisulfide-linked y,8TCR receptor, the presence of a cysteine residue in the membrane proximal connector region of its δTCR chain leaves open the possibility that this 8TCR subunit might be capable of participating in either a nondisulfide-linked or a disulfide-linked complex.

### 9.2.2. yTCR GENE PRODUCT DETERMINES THE FORM OF THE RECEPTOR

The MOLT-13 cells transfected with yTCR cDNA constructs were abbreviated as M13.PBL C1y (for MOLT-13 cells transfected with PBL C1-derived yTCR cDNA) and M13.IDP2y (for MOLT-13 cells transfected with IDP2-derived yTCR cDNA). The bulk transfectant cell lines and representative subclones derived from these lines were analyzed by Northern blot analysis with yTCR (VJC) or Vy2-specific cDNA probes. In addition to the resident 1.6 kb MOLT-13 yTCR transcript, a second yTCR transcript of about 1.8 kb (expected size for a yTCR transcript initiating in the SFFV LTR of the expression plasmid) was observed in transfectant lines and their clones. The 1.8 kb transcript specifically hybridized with a Vy2 probe that does not cross-hybridize with the Vy1.3 present in the resident MOLT-13 yTCR transcript, and thus the 1.8 kb transcript represents the transcript of the transfected yTCR cDNAs (which utilize a Vy2 segment).

To biochemically characterize the yTCR protein(s) expressed on the surface of the transfectants, a representative clone derived from each line was analyzed by immunoprecipitation of surface iodinated cells with P3 (control), anti-leu-4 (anti-CD3), anti-TCR81 (anti-8TCR), or anti-Ti-yA mAbS. Anti-Ti-yA Jitsukawa, S., et al., 1987, J. Exp. Med. 166:1192-1197) appears to specifically recognize the y,8TCR cells that utilize the Vy2 gene segment as the variable portion of their yTCR chains. Untransfected (Fig. 19A) as well as the transfected MOLT-13 cells (Fig. 19B and 19C) express the expected parental yTCR (40 kD; see open arrow) and δTCR subunits (see asterisk). Note that anti-Vy2-specific mAb (anti-Ti-yA) fails to react with the resident MOLT-13 yTCR chain (Fig. 19, lanes 7 and 8). Anti-CD3 immunoprecipitates of M13.PBL C1y transfectant cells revealed an additional CD3-associated species (68 kD) when examined under nonreducing conditions (Fig. 19B, lane 3, see solid arrow). On both PBL C1 cells (Fig. 19D, lanes 3 and 4) and the M13.PBL C1y transfectant cell line (Fig. 19B lanes 3 and 4), the 68 kD complex yielded 40 and 36 kD species upon reduction (in the case of the M13.PBL C1y transfectant, these bands are clearly visualized in the anti-Vy2 immunoprecipitate, see Fig. 19B, lanes 7 and 8, solid arrows). These 40 and 36 kD species represent differentially glycosylated yTCR polypeptides (Brenner, M.B., et al., 1987, Nature 325:689-694). In these immunoprecipitates, the 8TCR chain (40 kD reduced) comigrates with the 40 kD yTCR polypeptide and is therefore not visualized (however, see below).

Importantly these experiments show that the resident 8TCR chain of the MOLT-13 cell line, normally part of a nondisulfide-linked complex, associates with the PBL C1 yTCR protein to form a disulfide-linked γ,δTCR heterodimer in the transfectant cell line. In contrast, the IDP2-derived yTCR protein (55 kD) in the M13.IDP2 transfectant cell line formed a nondisulfide-linked complex with the resident MOLT-13 yTCR chain (Fig. 19C, lanes 3 and 4). Immunoprecipitates carried out with anti-TCR81 mAb (specific for yTCR peptide) confirmed that the endogenous (Figs. 19A, D and E, lanes 5 and 6) as well as the transfected yTCR chains (Fig. 19B and C, lanes 5 and 6) from all these cell lines were associated directly with the yTCR chain. Anti-Ti-yA specifically immunoprecipitated the 68 kD disulfide-linked γ,δTCR heterodimer from the M13.PBL C1 transfectant cells (Fig. 19B, lanes 7 and 8), and the 55 kD yTCR chain, along with the 40 kD yTCR chain, from the M13.EDP2y transfectant cells (Fig. 19C, lanes 7 and 8), confirming that the yTCR chains that are part of these complexes correspond to the transfected PBL C1 and IDP2-derived yTCR cDNAs, respectively.

To further characterize the various yTCR proteins biochemically, two-dimensional (2D) gel analyses (NEPHGE followed by SDA-PAGE) of surface ¹²⁵1-labeled cells were carried out. Superimposition of the 2D patterns of resident (MOLT-13) and transfected (PBL C1 or IDP2) yTCR chains relative to the positions of the CD3 components allowed comparison of the relevant yTCR species. In the immunoprecipitates from MOLT-13 cells, the yTCR chains resolved as two discrete parallel series of iodinated species (Fig. 20A, see open arrows). MOLT-13 cells transfected with the PBL C1 or the IDP2 TCR cDNAs revealed the resident MOLT-13 yTCR polypeptide series, but in addition, showed radiolabeled species that were identical in 2D gel patterns to the yTCR polypeptides of PBL C1 (compare Fig. 20B and D; see asterisks in Fig. 20B) or IDP2 (compare Fig. 20C and D; see closed arrows in Fig. 20C) cells, respectively. Thus, the 2D gel biochemical analyses confirmed that the transfected yTCR chains were expressed and processed similarly in MOLT-13 transfectants and in the parental cell lines, PBL C1 and IDP2.

### 9.2.3. POLYPEPTIDE BACKBONE SIZES OF THE TRANSFECTED yTCR CHAIN PROTEINS

The peptide backbone sizes of the transfected yTCR chains were determined by endoglycosidase-H treatment of the material immunoprecipitated from metabolically pulse-labeled cells. Immunoprecipitates carried out with anti-CγM1 (specific foryTCR chain) identified 35.5 and 34 kD species in untransfected MOLT-13 cells (Fig. 21, lane 4) that represent endogenous MOLT-13 yTCR polypeptides. The smaller of these two polypeptides (see open arrows) corresponds to the expected polypeptide core size of the MOLT-13 yTCR polypeptide, whereas the larger polypeptide appears to represent a partially processed intermediate. In addition to these resident MOLT-13 yTCR polypeptides, a polypeptide with a deglycosylated size of 41 kD was immunoprecipitated by anti-CγM1 from the M13.IDP2y transfectant, (Fig. 21, lane 8, see solid arrow). The size of this transfectant-specific yTCR polypeptide agrees well with the deglycosylated IDP2 yTCR polypeptide core size determined earlier in IDP2 cells (Brenner, M.B., et al., 1987, Nature 325:689-694). As expected, M13.PBL C1γ transfectant cells revealed an additional yTCR protein with a deglycosylated size of 32 kD (Fig. 21, lane 12, see solid arrow) which compares well with the yTCR polypeptide backbone size reported earlier for the PBL C1 cell line (Brenner, M.B., et al., 1987, Nature 325:689-694). This 32 kD species was specifically immunoprecipitated by the Vy2-specific mAb, anti-Ti-yA (Fig. 21, lane 13, see solid arrow), thereby allowing unambiguous assignment of resident and transfected yTCR species in this cell line. Thus, the determined backbone sizes of the transfected yTCR chains, derived from IDP2 and PBL C1 cell lines, match the backbone sizes of these polypeptides in their parent cell lines. By comparing the yTCR polypeptide core sizes with those of the cell surface proteins, we infer that the MOLT-13, IDP2, and PBL C1 derived yTCR chains carry 6, 14, and 8 kD N-linked carbohydrate, respectively.

### 9.3. DISCUSSION

Three biochemically distinct forms of the human γδTCR subunit structure occur. In the present work, we show that a single 8TCR polypeptide can associate with yTCR chains representing each of the three receptor forms to reconstitute the appropriate γ,δBTCR heterodimers. The resident yTCR polypeptide of MOLT-13 (form 2bc) is 40 kD and is noncovalently associated with the 8TCR subunit. When the yTCR cDNAclones corresponding to the disulfide-linked receptor of PBL C1 (Form 1), or the 55 kD non-disulfide-linked receptor of the IDP2 cell line (Form 2abc) were transfected into the MOLT-13 cell line, the γδTCR forms corresponding to those found in the cDNA-donor cell lines were reconstituted. The present transfection studies provide direct evidence that disulfide linkage is dictated by yTCR constant segment usage, since the resident MOLT-13 8TCR chain was shown to participate in a disulfide-linked receptor complex with the PBL C1-derived yTCR chain (Form 1), and a nondisulfide-linked receptor complex with the IDP2-derived yTCR chain (Form 2abc).

We have shown that the remarkable difference in size between the 55 kD (Form 2abc) and 40 kD (Form 2bc) non-disulfide-linked yTCR polypetides is primarily due to different amounts of N-linked carbohydrate attached to the yTCR polypeptide backbone (See Section 8.2.2, supra). Thus, either 15 kD (Form 2abc on IDP2 or PEER) or only 5 kD (Form 2bc on MOLT-13) of N-linked carbohydrate is attached to these yTCR polypeptides even though the same number (five each) of N-linked glycan acceptor sites are encoded by the constant region gene segments used in both of these forms. Four of these N-linked glycosylation sites are present in or around the CII exon-encoded connector region. In the example herein, we show that the amount of N-linked carbohydrate attached to the transfected yTCR proteins is identical to that seen in their parent cell lines, based on a comparison of peptide core size and mature cell surface size of the protein products of transfected yTCRcDNA clones. Thus, the conformation of the two Cy2 encoded protein segments must differ sufficiently to result in drastic differences in glycosylation. The major difference between Cy segments of these two forms is that copy "a" of the CII exon is present in the 55 kD yTCR chain of Form 2abc and it is absent from the 40 kD yTCR chain of Form 2bc. Thus the presence or absence of this CII exon copy may be largely responsible for the glycosylation differences that account for the yTCR polypeptide sizes.

The variation in structure of human γδTCR isotypic forms is unprecedented among T cell receptors as no such parallel is observed in αβTCR.

### 10. T CELL RECEPTOR y8 COMPLEX, NOT ASSOCIATED WITH CD3, IS IDENTIFIED IN HUMAN ENDOMETRIAL GLANDULAR EPITHELIUM

In the early stages of placentation, infiltration of mononuclear cells is abundant at the proximity of spiral arteries and endometrial glands in maternal uterine tissues. These include an unusual population of T lineage cells of unknown function. Many extravillous trophoblasts express a novel type of class I MHC antigens which is different from that expressed on most somatic cells. We have tested a panel of monoclonal antibodies to TCR y8 heterodimer (γδTCR) in pregnant & non-pregnant uteri. Surprisingly, γδTCR complex was not detected in leukocytes, but was localized in the cytoplasm of the endometrial glandular epithelium from pregnant uteri. These antibodies also react with the glandular epithelium from non-pregnant uteri, and the reactivity was stronger in the secretory phase than that in the proliferative phase of the menstrual cycle. However, yoTCR was not associated with the CD3 complex, as shown by examining immunoprecipitates using three different monoclonal antibodies to CD3 (OKT3, anti-leu-4, UCHT-1). The γδTCR-positive glandular epithelial cells did not react with monoclonal antibodies to αβTCR; the cells were also CD4- and CD8-negative. Moreover, the glandular epithelial cells lose the class I MHC antigens in early pregnancy. These data suggest that these γδTCR bearing endometrial glandular cells undergo, at least, phenotypic alterations under local regulation of gene expression.

### 11. EXAMPLE: CHARACTERIZATION OF A HUMAN 8 T CELL RECEPTOR GENE AND AVₛ SPECIC MONOCLONAL ANTIBODY

We have isolated δTCR cDNA and a rearranged δTCR gene from a human y8 T cell clone, AK119. From these DNAclones, a K8 probe was obtained, and used to determine the diversity of 8TCR gene rearrangements in a panel of 13 human y,8 T cell clones and 3 y,8 human T cell tumor lines. Altogether five different rearrangements were detected, which corresponded to rearrangements using 2 to 5 different X₈ genes. One particular rearrangement was always seen in human y,8 T cells that reacted with mAb TCS81 (8TCAR-3). In addition, TCS81 immunoprecipitated with δTCR polypeptide from a human y,8 tumor cell line, Molt 13. We provide evidence that monoclonal antibody TCS81 recognizes an epitope encoded in the AK119 V8 gene or in a combination epitope of the rearranged AK119 gene V8-J8 gene.

### 11.1. MATERIALS AND METHODS

### 11.1.1. ISOLATION AND SEQUENCING OF AK119 δTCR cDNA CLONES

A cDNA library was generated from the PBL T-cell clone, AK119, by the method of Gubler and Hoffmann (Gublerand Hoffman, 1983, Gene 25:263). About 100,000 plaques of an amplified librarywere screened using a ³²P-labelled nick-translated C8 probe, isolated from a δTCR clone called O-024 (Hata, S., et al.,1987, Science 238:678). The longest hybridizing cDNA clone (1.3 kb clone C11983) was selected for sequence analysis by the dideoxy chain termination method.

### 11.1.2. CLONING A REARRANGED δTCR GENE

A 3.5 kb genomic DNA clone containing the rearranged V8 gene was obtained from AK119 cells as follows: EcoRl digested DNA was size fractionated on a preparative agarose gel, ligated into λgt10, packaged and transfected into E. coli. Recombinant phage were screened with a ³²P-labeled nick translated 550 bp EcoRl fragment derived from the cDNAclone, c11983. A rearranged clone called r11981 which contains a 0.8 kb Hincll fragment (V region specific) and a 1 kb Hincll-EcoRl fragment (V-J region) was isolated.

### 11.1.3. DNA PREPARATION

Fetal and newborn thymic tissues were collected in accordance with accepted guidelines regarding patients' rights and approval. T cell clones were obtained from peripheral blood, pleural exudate or cerebrospinal fluid by limiting dilution and were cultured in vitro (Hafler et al., 1985, Ann Neurol. 18:451; Van de Griend et al., 1987, J. Immunol. 138:1627). In all cases, DNAwas prepared by digestion with proteinase Kin 1% sodium dodecyl sulfate, followed by extraction with phenol/chloroform and ethanol precipitation.

### 11.1.4 SOUTHERN BLOT ANALYSIS

Genomic DNAwas digested with EcoRl, size fractionated on a 0.9% agarose gel and transferred to nitrocellulose. Hybridization was carried out with ³²P-nick translated probes as previously described (Maniatis, 1982, Molecular Cloning: A Laboratory Manual (Cold Spring Harbor Laboratory; Cold Spring Harbor, New York).

### 11.1.5 CYTOFLUOROMETRIC ANALYSIS

Normal peripheral blood monoclear cells (PBMC), obtained from volunteers were isolated by fractionation on a Ficoll gradient. PBMC and PBL T cell clones were stained by indirect immunofluorescence using TCS81 mAb (referred to previously as δTCAR-3; See Section 7, supra) and fluorescein-conjugated goat antimouse IgG (Becton Dickinson) and analyzed in a fluorescence activated flow cytometer.

### 11.2. RESULTS

### 11.2.1. DIVERSITY OF δTCR GENE REARRANGEMENTS

Using a C8 probe, we isolated a 1.3 kb δTCR cDNA clone, termed c11983, from a λgt10 cDNA library of the T-cell clone AK119. The 5' end of c11983 was sequenced and found to use previously identified V_{δ} and J₈ genes (Hata et al., 1987, Science 238:678; Loh et al., 1987, Nature 330:569). The sequence of the V-J junction indicated that C11983 has an in-frame V-J joint.

A 550 basepair (bp) EcoRl fragment encoding all the variable and joining region and part of the constant region (V-J-C probe) was obtained from c11983 and used in Southern blot analysis of EcoRl digested genomic DNA from AK119. This probe detects a germline 3.2 kb V₈ and a germline 1.0 kb C₈ band. AK119 showed an extra rearranged 2.5 kb band that is identical to the common δTCR rearrangement (described in Hata et al., 1987, Science 238:678; Loh et al., 1987, Nature 330:569) (rearrangement II in Fig. 22). This 3.5 kb band was cloned from a EcoRl size-fractionated λgt10 genomic library using the V-J-C cDNA probe. A partial map of the cloned rearranged δTCT gene, called r11981, is shown in Figure 17. The localization of the variable and joining region was determined using J oligonucleotide probes and variable region specific probes. From r11981, a 1 kb V-J probe was isolated by digestion with Hincll and EcoRl enzyme (see Fig. 17).

This V-J probe was used to determine the diversity of δTCR gene rearrangements in a panel of 13 human γδTCR positive T-cell clones and 3 human γ-δTCR positive tumorcell lines. As shown in Figure 22, five common rearrangements, numbered I-V, are seen in the polyclonal newborn thymocyte sample (lane 11). These rearrangements are representative of rearrangements used by the human y8 T cell clones. Only rearrangement II hybridizes to the Hincll - Hincll V₈ specific probe. Although we do not know that all these arrangements represent V-D-J rather than D-J rearrangements, some of them must represent rearrangements of new variable regions to the previously characterized J₈ gene segment because these cells express a functional δTCR polypeptide chain on their cell surfaces. We have not ruled out the possibility that these new rearrangements represent rearrangements of a single new V₈ gene to other J_{δ} genes, yet to be identified. Our data is consistent with the fact that there must be 2-5 variable region genes that can be used in δTCR gene rearrangements.

### 11.2.2. DETERMINING THE SPECIFICITY OF mAb TCS81

TCS81 previously referred to as δTCAR-3, was generated by fusing splenocytes from of mice immunized with the human tumor γδTCR cell line MOLT-13 with a mouse myeloma line. When used in fluorescent activated cell sorter analysis, TCS81 reacted only with some but not all human y8 T cells. The results are given in Table 1. There is a perfect correlation with usage of the AK119 V_{δ} gene (rearrangement II) with positive staining by TCS81. This data provides strong evidence that the epitope recognized by 8TCS1 is encoded in the AK119 V_{δ} gene or in a combinatorial epitope of the rearranged AK119 V_{δ}-J_{δ} gene.

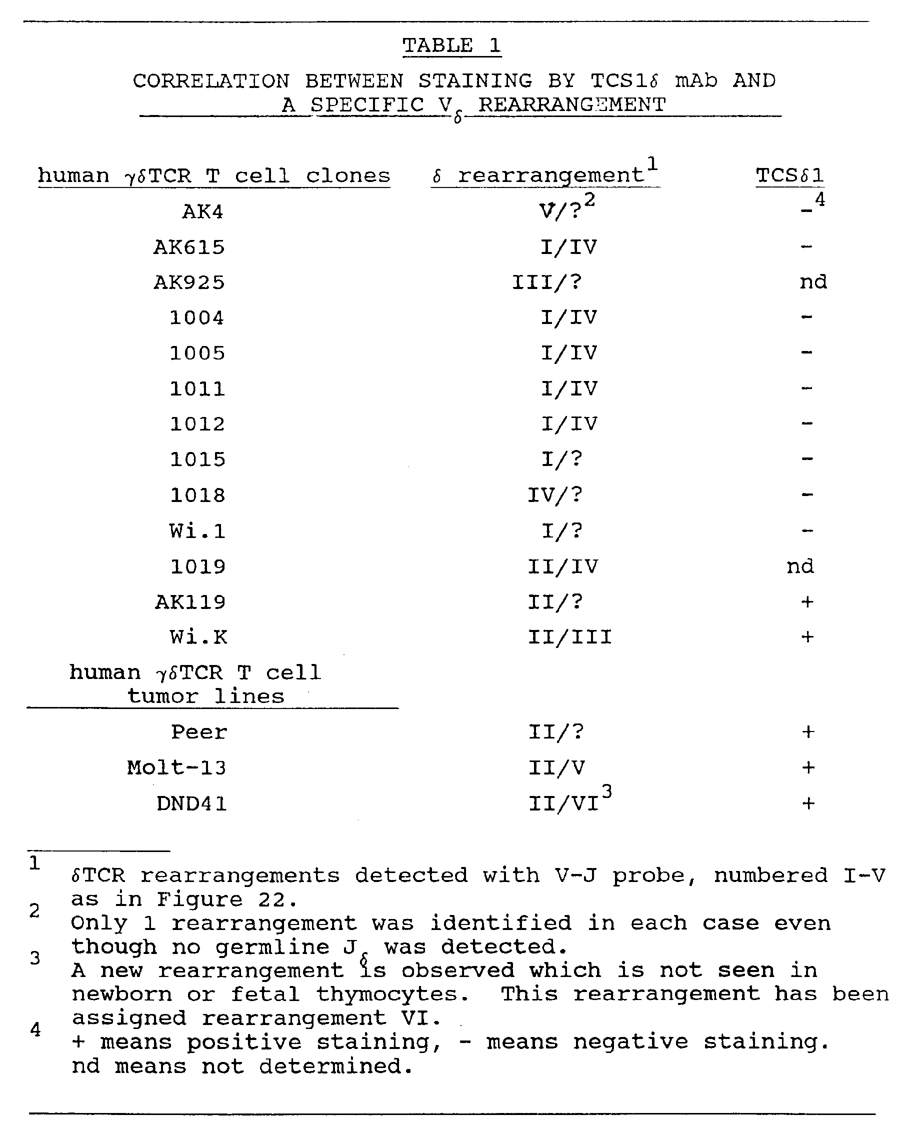

### 12. DEPOSIT OF HYBRIDOMAS

The following hybridoma cell lines, producing the indicated monoclonal antibody, have been deposited with the American Type Culture Collection (ATCC), 12310 Parklawn Drive, Rockville, Maryland 20852, U.S.A. on the indicated dates, and have been assigned the listed accession numbers:

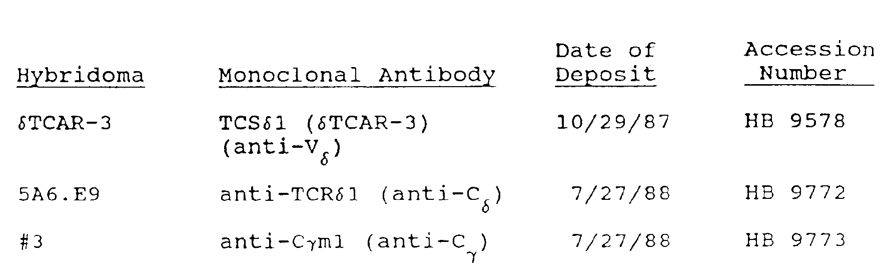

The following hybridoma cell lines have also been deposited with the American Type Culture Collection, on the indicated dates, and have been assigned the listed accession numbers:

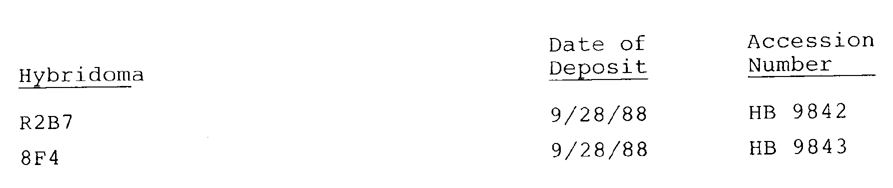

The present invention is not to be limited in scope by the cell lines deposited since the deposited embodiments are intended as single illustrations of one aspect of the invention and any cell lines which are functionally equivalent are within the scope of this invention. Indeed, various modifications of the invention in addition to those shown and described herein will become apparent to those skilled in the art from the foregoing description and accompanying drawings. Such modifications are intended to fall within the scope of the appended claims.

## Claims

1. A monoclonal antibody reactive with an epitope of a variable or rearranged variable-joining region of a δ chain of the human T cell antigen receptor.

2. The monoclonal antibody of claim 1 which comprises monoclonal antibody TCS81 (8TCAR-3) as produced by the hybridoma deposited with the ATCC and assigned accession number HB 9578.

3. A monoclonal antibody reactive with an epitope of the constant region of the δ chain of the human T cell antigen receptor.

4. The monoclonal antibody of claim 3 which comprises monoclonal antibody anti-TCR81 as produced by the hybridoma deposited with the ATCC and assigned accession number HB 9772.

5. A monoclonal antibody reactive with an epitope of the constant region of the Form 2bc gamma chain of the human T cell antigen receptor, which epitope is characteristic of the Form 2bc gamma chain.

6. A monoclonal antibody anti-Cyml as produced by the hybridoma deposited with the ATCC and assigned accession number HB 9773.

7. The Fv, Fab, Fab', or F(ab')₂ fragment of the monoclonal antibody of claim 1, 2 or 3.

8. The Fv, Fab, Fab', or F(ab')₂ fragment of the monoclonal antibody of claim 4, 5 or 6.

9. A hybridoma producing the monoclonal antibody of claim 1 or 2.

10. A hybridoma producing the monoclonal antibody of claim 3 or 4.

11. A hybridoma producing the monoclonal antibody of claim 5 or 6.

12. A method for detecting the presence of a functional rearrangement of a human δ T cell antigen receptor variable gene in a cell which comprises contacting the cell or a sample containing polypeptides of the cell with a monoclonal antibody specifically reactive with an epitope of a polypeptide encoded by the rearranged gene.

13. The method according to claim 12 in which the monoclonal antibody comprises TCS81 (8TCAR-3) as produced by the hybridoma deposited with the ATCC and assigned accession number HB 9578.

14. The monoclonal antibody of claim 1 which is characterized by the ability to co-modulate a CD3 antigen.

15. The monoclonal antibody of claim 3 which is characterized by the ability to co-modulate a CD3 antigen.

16. The monoclonal antibody of claim 5 which is characterized by the ability to co-modulate a CD3 antigen.

17. A purified polypeptide comprising an amino acid sequence of a constant region of a gamma T cell antigen receptor Form 2bc encoded by nucleotide numbers 439 through 1008 of Figure 14.

18. The purified polypeptide of claim 17, which comprises a gamma T cell antigen receptor Form 2bc, having a molecular weight of about 40,000 daltons and a sequence comprising a constant region consisting essentially of the amino acid sequence encoded by nucleotide numbers 439 through 1008 of Figure 14, and a variable region.

19. A purified polypeptide comprising a gamma T cell antigen receptor Form 2bc having a primary amino acid sequence substantially as depicted in Figure 14 for the variable, N, joining, and constant regions.

20. An isolated nucleic acid encoding the polypeptide of claim 17.

21. An isolated nucleic acid encoding the polypeptide of claim 18.

22. An isolated nucleic acid encoding the polypeptide of claim 19.

23. The nucleic acid of claim 22 which is a cDNA clone.

24. A nucleic acid vector comprising the cDNA clone of claim 23.

25. A cell containing the nucleic acid vector of claim 24.

26. The isolated nucleic acid of claim 20, 21 or 22 which comprises DNA.

27. The isolated nucleic acid of claim 20, 21 or 22 which comprises RNA.

28. A purified polypeptide complex comprising a T cell antigen receptor heterodimer consisting of the gamma T cell antigen receptor polypeptide of claim 17 and a second T cell antigen receptor polypeptide selected from the group consisting of the a, β, gamma, and δ T cell antigen receptor polypeptides.

29. The complex of claim 28 in which the second T cell antigen receptor is the δ T cell antigen receptor polypeptide.

30. A method for producing a gamma,delta T cell antigen receptor heterodimer which comprises culturing a cell (a) capable of expressing a nucleic acid encoding a δ T cell antigen receptor polypeptide, and (b) which has been transfected with a nucleic acid encoding a gamma T cell antigen receptor polypeptide, under conditions such that both the gamma T cell antigen receptor polypeptide and the δ T cell antigen receptor polypeptide are expressed by the cell and assemble to form a heterodimer.

31. The method according to claim 30 in which the gamma T cell antigen receptor polypeptide comprises an amino acid sequence of a constant region of a gamma T cell antigen receptor polypeptide Form 2bc encoded by nucleotide numbers 439 through 1008 of Figure 14.

32. The method according to claim 30 in which the gamma T cell antigen receptor polypeptide is Form 2bc having a primary amino acid sequence substantially as depicted in Figure 14 for the variable, N, joining, and constant regions.

33. The method according to claim 30 in which the gamma T cell antigen receptor polypeptide is Form 2bc having a molecular weight of about 40,000.

34. The method according to claim 30 in which the subunits of the heterodimer are disulfide-linked, and the gamma T cell antigen receptor polypeptide is Form 1 having a molecular weight of about 40,000.

35. The method according to claim 30 in which the gamma T cell antigen receptor polypeptide is Form 2abc having a molecular weight of about 55,000 daltons.

36. The method according to claim 30 in which the cell is a T cell.

37. The method according to claim 30 in which the nucleic acid encoding a gamma T cell antigen receptor polypeptide is selected from the group consisting of (i) a nucleic acid comprising a single CII exon; (ii) a nucleic acid comprising two CII exons; and (iii) a nucleic acid comprising three CII exons.

38. The method according to claim 30 in which the expressed gamma T cell antigen receptor polypeptide and the expressed δ T cell antigen receptor polypeptide are non-covalently associated.

39. The method according to claim 30 in which the expressed gamma T cell antigen receptor polypeptide and the expressed δ T cell antigen receptor polypeptide are covalently associated.

40. A method for producing at least a portion of a gamma,delta T cell antigen receptor heterodimer, which comprises culturing a transfected cell (a) capable of expressing a nucleic acid encoding at least a region of a gamma T cell antigen receptor polypeptide selected from the group consisting of a constant region, a variable region, and a joining region; and (b) capable of expressing a nucleic acid encoding at least a region of a δ T cell antigen receptor polypeptide selected from the group consisting of a constant region, a variable region, a joining region, and a diversity region; and subjecting the cell to conditions such that both nucleic acid sequences are expressed by the cell.

41. A method for expressing at least a portion of a gamma,delta T cell antigen receptor heterodimer, which comprises culturing a transfected cell (a) capable of expressing a nucleic acid encoding at least a region of a gamma T cell antigen receptor polypeptide selected from the group consisting of a variable region, a joining region, and a constant region; and (b) capable of expressing a nucleic acid encoding at least a region of a δ T cell antigen receptor polypeptide selected from the group consisting of a constant region, a variable region, a joining region, and a diversity region; and subjecting the cell to conditions such that both nucleic acid sequences are expressed by the cell, in which the nucleic acid encoding at least a region of the gamma T cell antigen receptor polypeptide was transfected into the cell.

42. The method according to claim 41 in which the gamma T cell antigen receptor polypeptide comprises Form 2bc, having a molecular weight of about 40,000 daltons and a sequence comprising a constant region consisting essentially of the amino acid sequence encoded by nucleotide numbers 439 through 1008 of Figure 14, and a variable region.

43. The method according to claim 40, 41 or 42, in which the cell is a T cell.

44. The method according to claim 40 or 41 in which the nucleic acid encoding at least a portion of the gamma T cell antigen receptor polypeptide is selected from the group consisting of (i) a nucleic acid comprising a single CII exon; (ii) a nucleic acid comprising two CII exons; and (iii) a nucleic acid comprising three CII exons.

45. The method according to claim 40 or 41 in which the portion of the gamma T cell antigen receptor polypeptide and the portion of the δ T cell antigen receptor polypeptide are non-covalently associated.

46. The method according to claim 40 or 41 in which the portion of the gamma T cell antigen receptor polypeptide and the portion of the δ T cell antigen receptor polypeptide are covalently associated.

47. Isolated cells which express at least a portion of a gamma, delta T cell antigen receptor heterodimer, which portion of a gamma,delta T cell antigen receptor comprises at least a region of a gamma T cell antigen receptor polypeptide selected from the group consisting of a constant region, a variable region, and a joining region, associated with at least a region of a δ T cell antigen receptor polypeptide selected from the group consisting of a constant region, a variable region, a joining region, and a diversity region; which heterodimer is not associated with a CD3 complex.

48. The cells of claim 47 in which the cells are endometrial glandular epithelial cells.

49. A transfected cell which expresses a gamma,delta T cell antigen receptor heterodimer, in which the gamma T cell antigen receptor polypeptide of the heterodimer is produced by expression of a nucleic acid encoding the gamma T cell antigen receptor polypeptide which was transfected into the cell.

50. The cell of claim 49 in which the gamma T cell antigen receptor polypeptide is Form 2bc having a molecular weight of about 40,000 daltons and a sequence comprising a constant region consisting essentially of the amino acid sequence encoded by nucleotide numbers 439 through 1008 of Figure 14, and a variable region.

51. A transfected cell which expresses at least a portion of a gamma,delta T cell antigen receptor heterodimer, which portion of a gamma,delta T cell antigen receptor comprises at least a region of a gamma T cell antigen receptor polypeptide selected from the group consisting of a constant region, a variable region, and a joining region, associated with at least a region of a 8 T cell antigen receptor polypeptide selected from the group consisting of a constant region, a variable region, a joining region, and a diversity region; which heterodimer is not associated with a CD3 complex, in which the portion of the gamma T cell antigen receptor polypeptide is produced by expression of a nucleic acid encoding the portion of the gamma T cell antigen receptor which was transfected into the cell.

52. A method for determining the relative usage of a gamma T cell antigen receptor polypeptide in a sample from a human subject comprising:
(a) determining in a sample containing T cells from a subject the amount of a gamma Form 2bc T cell antigen receptor polypeptide;
(b) determining in a sample from a subject the amount of a gamma T cell antigen receptor polypeptide selected from the group consisting of a Form 1 gamma polypeptide, and Form 2abc gamma polypeptide; and
(c) comparing the amount determined in step (a) with the amount determined in step (b), thereby determining the relative usage.

## Patentansprüche

1. MonoklonalerAntikörper, der gegenüber einem Epitop einer variablen oder rearrangierten variablen-verknüpfenden (joining) Region einer o-Kette des menschlichen T-Zell-Antigen-Rezeptors reaktiv ist.

2. MonoklonalerAntikörper nach Anspruch 1, umfassend den monoklonalen AntikörperTCSδ1 (äTCAR-3), wie er von dem Hybridom produziert wird, welches beim ATCC unter der Zugriffsnummer HB 9578 hinterlegt ist.

3. Monoklonaler Antikörper, der gegenüber einem Epitop der konstanten Region der o-Kette des menschlichen T-Zell-Antigen-Rezeptors reaktiv ist.

4. Monoklonaler Antikörper nach Anspruch 3, umfassend den monoklonalen Antikörper Anti-TCRδ1, wie er von dem Hybridom produziert wird, welches beim ATCC unter der Zugriffsnummer HB 9772 hinterlegt ist.

5. Monoklonaler Antikörper, der gegenüber einem Epitop der konstanten Region der Form 2bc-y-Kette des menschlichen T-Zell-Antigen-Rezeptors reaktiv ist, wobei das Epitop für die Form 2bc-y-Kette charakteristisch ist.

6. MonoklonalerAntikörperAnti-Cyml, wie er von dem Hybridom produziert wird, welches beim ATCC unter der Zugriffsnummer HB 9773 hinterlegt ist.

7. Fv-, Fab-, Fab'- oder F(ab')₂-Fragment des monoklonalen Antikörpers gemäß Anspruch 1, 2 oder 3.

8. Fv-, Fab-, Fab'- oder F(ab')₂-Fragment des monoklonalen Antikörpers gemäß Anspruch 4, 5 oder 6.

9. Hybridom, das den monoklonalen Antikörper gemäß Anspruch 1 oder 2 produziert.

10. Hybridom, das den monoklonalen Antikörper gemäß Anspruch 3 oder 4 produziert.

11. Hybridom, das den monoklonalen Antikörper gemäß Anspruch 5 oder 6 produziert.

12. Verfahren zum Nachweis des Vorliegens eines funktionellen Rearrangements eines menschlichen δ-T-Zell-Antigen-Rezeptorvariablen Gens in einerZelle, umfassend das Kontaktieren der Zelle oder einer Probe, die Polypeptide der Zelle enthält, mit einem monoklonalen Antikörper, der in spezifischer Weise gegenüber einem Epitop eines von dem rearrangierten Gen kodierten Polypeptids reaktiv ist.

13. Verfahren nach Anspruch 12, bei dem der monoklonale Anti körper TCSδ1 (6TCAR-3) umfaßt, wie er von dem Hybridom produziert wird, welches beim ATCC unter der Zugriffsnummer HB 9578 hinterlegt ist.

14. Monoklonaler Antikörper nach Anspruch 1, der durch die Fähigkeit gekennzeichnet ist, ein CD3-Antigen zu comodulieren.

15. Monoklonaler Antikörper nach Anspruch 3, der durch die Fähigkeit gekennzeichnet ist, ein CD3-Antigen zu comodulieren.

16. Monoklonaler Antikörper nach Anspruch 5, der durch die Fähigkeit gekennzeichnet ist, ein CD3-Antigen zu comodulieren.

17. Gereinigtes Polypeptid, das eine Aminosäuresequenz einer konstanten Region eines y-T-Zell-Antigen-Rezeptors Form 2bc umfaßt, die durch die Nukleotide 439 bis 1008 der Figur 14 kodiert wird.

18. Gereinigtes Polypeptid nach Anspruch 17, umfassend einen y-T-Zell-Antigen-Rezeptor Form 2bc mit einer relativen Molekülmasse von etwa 40 000 Dalton und einer Sequenz, die eine konstante Region umfaßt, welche im wesentlichen aus der Aminosäuresequenz besteht, welche durch die Nukleotide 439 bis 1008 der Figur 14 kodiert wird, und eine variable Region.

19. Gereinigtes Polypeptid, umfassend einen y-T-Zell-Antigen-Rezeptor Form 2bc mit einer primären Aminosäuresequenz im wesentlichen gemäß Darstellung in Figur 14 für die variable, N-, verknüpfende (joining) und konstante Region.

20. Isolierte Nukleinsäure, die für das Polypeptid gemäß Anspruch 17 kodiert.

21. Isolierte Nukleinsäure, die für das Polypeptid gemäß Anspruch 18 kodiert.

22. Isolierte Nukleinsäure, die für das Polypeptid gemäß Anspruch 19 kodiert.

23. Nukleinsäure nach Anspruch 22, welche ein cDNA-Klon ist.

24. Nukleinsäurevektor, umfassend den cDNA-Klon gemäß Anspruch 23.

25. Zelle, die den Nukleinsäurevektor gemäß Anspruch 24 enthält.

26. Isolierte Nukleinsäure nach Anspruch 20, 21 oder 22, die DNA umfaßt.

27. Isolierte Nukleinsäure nach Anspruch 20, 21 oder 22, die RNA umfaßt.

28. Gereinigter Polypeptidkomplex, umfassend ein T-Zell-Antigen-Rezeptor-Heterodimer, bestehend aus dem y-T-Zell-Antigen-Rezeptor-Polypeptid gemäß Anspruch 17 und einem zweiten T-Zell-Antigen-Rezeptor-Polypeptid, ausgewählt aus der Gruppe bestehend aus den a-, β-, y- und 5-T-Zell-Antigen-Rezeptor-Polypeptiden.

29. Komplex nach Anspruch 28, bei dem der zweite T-Zell-Antigen-Rezeptor das δ-T-Zell-Antigen-Rezeptor-Polypeptid ist.

30. Verfahren zur Herstellung eines y,o-T-Zell-Antigen-Rezeptor-Heterodimers, umfassend das Kultivieren einer Zelle, die (a) in der Lage ist, eine Nukleinsäure zu exprimieren, welche für ein δ-T-Zell-Antigen-Rezeptor-polypeptid kodiert, und die (b) mit einer Nukleinsäure transfiziert worden ist, welche für ein y-T-Zell-Antigen-Rezeptor-Polypeptid kodiert, unter solchen Bedingungen, daß sowohl das y-T-Zell-Antigen-Rezeptor-Polypeptid als auch das δ-T-Zell-Antigen-Rezeptor-Polypeptid von der Zelle exprimiert werden und sich zur Bildung eines Heterodimers zusammengefügen.

31. Verfahren nach Anspruch 30, bei dem das y-T-Zell-Antigen-Rezeptor-Polypeptid eine Aminosäuresequenz einer konstanten Region eines y-T-Zell-Antigen-Rezeptor-Polypeptids Form 2bc umfaßt, welche durch die Nukleotide 439 bis 1008 der Figur 14 kodiert wird.

32. Verfahren nach Anspruch 30, bei dem das y-T-Zell-Antigen-Rezeptor-Polypeptid Form 2bc ist mit einer primären Aminosäuresequenz im wesentlichen gemäß Darlegung in Figur 14 für die variable, N-, verknüpfende (joining) und konstante Region.

33. Verfahren nach Anspruch 30, bei dem das y-T-Zell-Antigen-Rezeptor-Polypeptid Form 2bc ist mit einer relativen Molekülmasse von etwa 40 000.

34. Verfahren nach Anspruch 30, bei dem die Untereinheiten des Heterodimers über Disulfidbrücken verknüpft sind, und bei dem das y-T-Zell-Antigen-Rezeptor-Polypeptid Form 1 ist mit einer relativen Molekülmasse von etwa 40 000.

35. Verfahren nach Anspruch 30, bei dem das y-T-Zell-Antigen-Rezeptor-Polypeptid Form 2abc ist mit einer relativen Molekülmasse von etwa 55 000 Dalton.

36. Verfahren nach Anspruch 30, bei dem die Zelle eine T-Zelle ist.

37. Verfahren nach Anspruch 30, bei dem die Nukleinsäure, die für ein y-T-Zell-Antigen-Rezeptor-Polypeptid kodiert, ausgewählt wird aus der Gruppe bestehend aus: (i) einer Nukleinsäure, die ein einziges CII-Exon umfaßt; (ii) einer Nukleinsäure, die zwei CII-Exons umfaßt; und (iii) einer Nukleinsäure, die drei CII-Exons umfaßt.

38. Verfahren nach Anspruch 30, bei dem das exprimierte y-T-Zell-Antigen-Rezeptor-Polypeptid und das exprimierte δ-T-Zell-Antigen-Rezeptor-Polypeptid nicht-kovalent assoziiert sind.

39. Verfahren nach Anspruch 30, bei dem das exprimierte y-T-Zell-Antigen-Rezeptor-Polypeptid und das exprimierte δ-T-Zell-Antigen-Rezeptor-Polypeptid kovalent assoziiert sind.

40. Verfahren zur Herstellung mindestens eines Teils eines γ,δ-T-Zell-Antigen-Rezeptor-Heterodimers, umfassend das Kultivieren einertransfizierten Zelle, die (a) in der Lage ist, eine Nukleinsäure zu exprimieren, welche für mindestens eine Region eines y-T-Zell-Antigen-Rezeptor-Polypeptids, ausgewählt aus der Gruppe bestehend aus einer konstanten Region, einer variablen Region, und einer verknüpfenden (joining) Region, kodiert; und die (b) in der Lage ist, eine Nukleinsäure zu exprimieren, welche für mindestens eine Region eines δ-T-Zell-Antigen-Rezeptor-Polypeptids, ausgewählt aus der Gruppe bestehend aus einer konstanten Region, einer variablen Region, einer verknüpfenden (joining) Region, und einer Verschiedenheits (diversity)-Region, kodiert; und das Zuführen der Zelle unter solche Bedingungen, daß beide Nukleinsäuresequenzen von der Zelle exprimiert werden.

41. Verfahren zur Expression mindestens eines Bereiches eines y,5-T-Zell-Antigen-Rezeptor-Heterodimers, umfassend das Kultivieren einer transfizierten Zelle, welche für (a) in der Lage ist, eine Nukleinsäure zu exprimieren, die mindestens eine Region eines y-T-Zell-Antigen-Rezeptor-Polypeptids, ausgewählt aus der Gruppe bestehend aus einer variablen Region, einer verknüpfenden (joining) Region, und einer konstanten Region, kodiert; und die (b) in der Lage ist, eine Nukleinsäure zu exprimieren, welche für mindestens eine Region eines δ-T-Zell-Antigen-Rezeptor-Polypeptids, ausgewählt aus der Gruppe bestehend aus einer konstanten Region, einer variablen Region, einer verknüpfenden (joining) Region, und einer Verschiedenheits- (diversity) Region, kodiert; und das Zuführen der Zelle unter solche Bedingungen, daß beide Nukleinsäuresequenzen von der Zelle exprimiert werden, wobei die Zelle mit der Nukleinsäure transfiziert worden ist, welche für mindestens eine Region des y-T-Zell-Antigen-Rezeptor-Polypeptids kodiert.

42. Verfahren nach Anspruch 41, bei dem das y-T-Zell-Antigen-Rezeptor-Polypeptid Form 2bc umfaßt, eine relative Molekülmasse von etwa 40 000 Dalton aufweist und eine Sequenz besitzt, die eine konstante Region, welche im wesentlichen aus der von den Nukleotiden 439 bis 1008 der Figur 14 kodierten Aminosäuresequenz besteht, und eine variable Region umfaßt.

43. Verfahren nach Anspruch 40, 41 oder 42, bei dem die Zelle eine T-Zelle ist.

44. Verfahren nach Anspruch 40 oder 41, bei dem die Nukleinsäure, welche für mindestens einen Bereich des y-T-Zell-Antigen-Rezeptor-Polypeptids kodiert, ausgewählt wird aus der Gruppe bestehend aus: (i) einer Nukleinsäure, die ein einziges CII-Exon umfaßt; (ii) einer Nukleinsäure, die zwei CII-Exons umfaßt; und (iii) einer Nukleinsäure, die drei CII-Exons umfaßt.

45. Verfahren nach Anspruch 40 oder41, bei dem der Bereich des y-T-Zell-Antigen-Rezeptor-Polypeptids und der Bereich des δ-T-Zell-Antigen-Rezeptor-Polypeptids nicht-kovalent assoziiert sind.

46. Verfahren nach Anspruch 40 oder41, bei dem der Bereich des y T-Zell-Antigen-Rezeptor-Polypeptids und der Bereich des δ-T-Zell-Antigen-Rezeptor-Polypeptids kovalent assoziiert sind.

47. Isolierte Zellen, die mindestens einen Bereich eines γ,δ-T-Zell-Antigen-Rezeptor-Heterodimers exprimieren, wobei der Bereich eines γ,δ-T-Zell-Antigen-Rezeptors mindestens eine Region eines y-T-Zell-Antigen-Rezeptor-Polypeptids, ausgewählt aus der Gruppe bestehend aus einer konstanten Region, einer variablen Region, und einer verknüpfenden (joining) Region, assoziiert mit mindestens einer Region eines δ-T-Zell-Antigen-Rezeptor-Polypeptids ausgewählt aus der Gruppe bestehend aus einer konstanten Region, einer variablen Region, einer verknüpfenden (joining) Region, und einer Verschiedenheits (diversity)-Region, umfaßt, und wobei das Heterodimer nicht mit einem CD3-Komplex assoziiert ist.

48. Zellen nach Anspruch 47, wobei die Zellen Drüsenzellen des Endometriomepithels sind.

49. Transfizierte Zelle, die ein y,5-T-Zell-Antigen-Rezeptor-Heterodimer exprimiert, wobei das y-T-Zell-Antigen-Rezeptor-Polypeptid des Heterodimers durch Expression einer Nukleinsäure produziert wird, welche für das y-T-Zell-Antigen-Rezeptor-Polypeptid kodiert und mit der die Zelle transfiziert worden ist.

50. Zelle nach Anspruch 49, in der das y-T-Zell-Antigen-Rezeptor-Polypeptid Form 2bc ist mit einer relativen Molekülmasse von etwa 40 000 Dalton und einer Sequenz, die eine konstante Region, welche im wesentlichen aus der von den Nukleotiden 439 bis 1008 der Figur 14 kodierten Aminosäuresequenz besteht, und eine variable Region umfaßt.

51. Transfizierte Zelle, die mindestens einen Bereich eines γ,δ-T-Zell-Antigen-Rezeptor-Heterodimers exprimert, wobei der Bereich eines y,5-T-Zell-Antigen-Rezeptors mindestens eine Region eines y-T-Zell-Antigen-Rezeptor-Polypeptids, ausgewählt aus der Gruppe bestehend aus einer konstanten Region, einer variablen Region, und einer verknüpfenden (joining) Region, assoziiert mit mindestens einer Region eines δ-T-Zell-Antigen-Rezeptor-Polypeptids, ausgewählt aus der Gruppe bestehend aus einer konstanten Region, einer variablen Region, einer verknüpfenden (joining) Region, und einer Verschiedenheits (diversity)-Region, umfaßt; wobei das Heterodimer nicht mit einem CD3-Komplex assoziiert ist, und wobei der Bereich des y-T-Zell-Antigen-Rezeptor-Polypeptids durch Expression einer Nukleinsäure produziert wird, welche für den Bereich des y-T-Zell-Antigen-Rezeptors kodiert und mit der die Zelle transfiziert worden ist.

52. Verfahren zur Bestimmung der relativen Verwendung eines y-T-Zell-Antigen-Rezeptor-Polypeptids in einer Probe aus einem menschlichen Individium, bei dem man:
(a) in einer Probe, die T-Zellen von einem Individium enthält, die Menge eines y-Form 2bc-T-Zell-Antigen-Rezeptor-Polypeptids bestimmt;
(b) in einer Probe eines Individiums die Menge eines y-T-Zell-Antigen-Rezeptor-Polypeptids, ausgewählt aus der Gruppe bestehend aus einem Form 1-y-Polypeptid und einem Form 2abc-γ-Polypeptid, bestimmt; und
(c) die in Stufe (a) ermittelte Menge mit der in Stufe (b) ermittelten Menge vergleicht und dadurch die relative Verwendung bestimmt.

## Revendications

1. Anticorps monoclonal réactif avec un épitope d'une région variable ou d'une région de jonction variable réarrangée d'une chaîne 0 du récepteur d'antigènes de cellules T humaines.

2. Anticorps monoclonal selon la revendication 1, comprenant un anticorps monoclonal TCSδ1 (δ TCAR-3) tel que produit par l'hybridome déposé à l'ATCC et ayant reçu le numéro d'accès HB 9578.

3. Anticorps monoclonal réactif avec un épitope de la région constante de la chaîne 0 du récepteur d'antigènes de cellules T humaines.

4. Anticorps monoclonal selon la revendication 3, comprenant un anticorps monoclonal anti-TCRâ1 tel que produit par l'hybridome déposé à l'ATCC et ayant reçu le numéro d'accès HB 9772.

5. Anticorps monoclonal réactif avec un épitope de la région constante de la chaîne gamma de Forme 2bc du récepteur d'antigènes de cellules T humaines, lequel épitope est caractéristique de la chaîne gamma de Forme 2bc.

6. Anticorps monoclonal anti-Cγm1 tel que produit par l'hybridome déposé à l'ATCC et ayant reçu le numéro d'accès HB 9773.

7. Fragment Fv, Fab, Fab' ou F(ab')₂ de l'anticorps monoclonal selon l'une quelconque des revendications 1, 2 ou 3.

8. Fragment Fv, Fab, Fab' ou F(ab')₂ de l'anticorps monoclonal selon l'une quelconque des revendications 4, 5 ou 6.

9. Hybridome produisant l'anticorps monoclonal selon l'une des revendications 1 ou 2.

10. Hybridome produisant l'anticorps monoclonal selon l'une des revendications 3 ou 4.

11. Hybridome produisant l'anticorps monoclonal selon l'une des revendications 5 ou 6.

12. Procédé pour la détection de la présence d'un réarrangement fonctionnel d'un gène variable de récepteur d'antigènes de cellules T 0 humaines dans une cellule, comprenant la mise en contact de la cellules ou d'un échantillon contenant des polypeptides de la cellule avec un anticorps monoclonal spécifiquement réactif avec un épitope d'un polypeptide codé par le gène réarrangé.

13. Procédé selon la revendication 12, dans lequel l'anticorps monoclonal comprend TCSδ1 (δ TCAR-3) tel que produit par l'hybridome déposé à l'ATCC et ayant reçu le numéro d'accès HB 9578.

14. Anticorps monoclonal selon la revendication 1, caractérisé par son aptitude à co-moduler un antigène CD3.

15. Anticorps monoclonal selon la revendication 3, caractérisé par son aptitude à co-moduler un antigène CD3.

16. Anticorps monoclonal selon la revendication 5, caractérisé par son aptitude à co-moduler un antigène CD3.

17. Polypeptide purifié comprenant une séquence d'acides aminés d'une région constante d'un récepteur d'antigènes de cellules T gamma de Forme 2bc codée par les nucléotides numéros 439 à 1008 de la figure 14.

18. Polypeptide purifié selon la revendication 17, comprenant un récepteur d'antigènes de cellules T gamma de Forme 2bc, ayant une masse moléculaire d'environ 40.000 daltons et une séquence comprenant une région constante consistant essentiellement en la séquence d'acides aminés codéée par les nucléotides numéros 439 à 1008 de la figure 14, et une région variable.

19. Polypeptide purifié comprenant un récepteur d'antigènes de cellules T gamma de Forme 2bc ayant une séquence d'acides aminés primaire substantiellement telle que décrite dans la figure 14 pour les régions variable, N, de jonction et constante.

20. Acide nucléique isolé codant pour le polypeptide selon la revendication 17.

21. Acide nucléique isolé codant pour le polypeptide selon la revendication 18.

22. Acide nucléique isolé codant pour le polypeptide selon la revendication 19.

23. Acide nucléique selon la revendication 22, qui est unclone d'ADNc.

24. Vecteur d'acide nucléique, comprenant le clone d'ADNc selon la revendication 23.

25. Cellule contenant le vecteur d'acide nucléique selon la revendication 24.

26. Acide nucléique isolé selon l'une quelconque des revendications 20, 21 ou 22, comprenant de l'ADN.

27. Acide nucléique isolé selon l'une quelconque des revendications 20, 21 ou 22, comprenant de l'ARN.

28. Complexe polypeptidique purifié, comprenant un hétérodimère de récepteur d'antigènes de cellules T consistant en le polypeptide récepteur d'antigènes de cellules T gamma selon la revendication 17 et un second polypeptide récepteur d'antigènes de cellules T choisi dans le groupe comprenant les polypeptides récepteurs d'antigènes de cellules T α,β, gamma et 8.

29. Complexe selon la revendication 28, dans lequel le second récepteur d'antigènes de cellules T est le polypeptide récepteur d'antigènes de cellules T 8.

30. Procédé pour la production d'un hétérodimère de récepteur d'antigènes de cellules T gamma,delta, comprenant la mise en culture d'une cellule (a) apte à exprimer un acide nucléique codant pour un polypeptide récepteur d'antigènes de cellules T 8, et (b) qui a été transfectée avec un acide nucléique codant pour un polypeptide récepteur d'antigènes de cellules T gamma, dans des conditions telles qu'à la fois le polypeptide récepteur d'antigènes de cellules T gamma et le polypeptide récepteur d'antigènes de cellules To sont exprimés par la cellule et s'assemblent pour former un hétérodimère.

31. Procédé selon la revendication 30, dans lequel le polypeptide récepteur d'antigènes de cellules T gamma comprend une séquence d'acides aminés d'une région constante d'un polypeptide récepteur d'antigène de cellules T gamma de Forme 2bc codé par les nucléotides numéros 439 à 1008 de la figure 14.

32. Procédé selon la revendication 30, dans lequel le polypeptide récepteur d'antigènes de cellules T gamma est la Forme 2bc ayant une séquence d'acides aminés primaire substantiellement telle que décrite dans la figure 14 pour les régions variable, N, de jonction et constante.

33. Procédé selon la revendication 30, dans lequel le polypeptide récepteur d'antigènes de cellules T gamma est la Forme 2bc ayant une masse moléculaire d'environ 40.000.

34. Procédé selon la revendication 30, dans lequel les sous-unités de l'hétérodimère sont liées par disulfure, et le polypeptide récepteur d'antigènes de cellules T gamma est la Forme 1 ayant une masse moléculaire d'environ 40.000.

35. Procédé selon la revendication 30, dans lequel le polypeptide récepteur d'antigènes de cellules T gamma est la Forme 2abc ayant une masse moléculaire d'environ 55.000 daltons.

36. Procédé selon la revendication 30, dans lequel la cellule est une cellule T.

37. Procédé selon la revendication 30, dans lequel l'acide nucléique codant pour un polypeptide récepteur d'antigènes de cellules Tgamma est choisi dans le groupe consistant en (i) un acide nucléique comprenant un unique exon CII; (ii) un acide nucléique comprenant deux exons CII; et (iii) un acide nucléique comprenant trois exons CII.

38. Procédé selon la revendication 30, dans lequel le polypeptide récepteur d'antigènes de cellules T gamma exprimé et le polypeptide récepteur d'antigènes de cellules T 0 exprimé ne sont pas associés par covalence.

39. Procédé selon la revendication 30, dans lequel le polypeptide récepteur d'antigènes de cellules T gamma exprimé et le polypeptide récepteur d'antigènes de cellules T 0 exprimé sont associés par covalence.

40. Procédé pour la production d'au moins une portion d'un hétérodimère de récepteur d'antigènes de cellules T gamma,delta, comprenant la mise en culture d'une cellule transfectée (a) apte à exprimer un acide nucléique codant pour au moins une région d'un polypeptide récepteur d'antigènes de cellules T gamma choisie dans le groupe consistant en une région constante, une région variable et une région de jonction; et (b) apte à exprimer un acide nucléique codant pour au moins une région d'un polypeptide récepteur d'antigènes de cellules Tδ, choisie dans le groupe consistant en une région constante, une région variable, une région de jonction et une région de diversité; et l'exposition de la cellule à des conditions telles que les deux séquences d'acides nucléiques sont exprimées par la cellule.

41. Procédé pour l'expression d'au moins une portion d'un hétérodimère de récepteur d'antigènes de cellules T gamma,delta, comprenant la mise en culture d'une cellule transfectée (a) apte à exprimer un acide nucléique codant pour au moins une région d'un polypeptide récepteur d'antigènes de cellules T gamma choisie dans le groupe consistant en une région variable, une région de jonction et une région constante; et (b) apte à exprimer un acide nucléique codant pour au moins une région d'un polypeptide récepteur d'antigènes de cellules T 8, choisie dans le groupe consistant en une région variable, une région de jonction et une région de diversité; et l'exposition de la cellule à des conditions telles que les deux séquences d'acides nucléiques sont exprimées par la cellule, l'acide nucléique codant pour au moins une région du polypeptide récepteur d'antigènes de cellules T gamma ayant été transfecté dans la cellule.

42. Procédé selon la revendication 41, dans lequel le polypeptide récepteur d'antigènes de cellules T gamma comprend la Forme 2bc, ayant une masse moléculaire d'environ 40.000 daltons et une séquence comprenant une région constante consistant essentiellement en la séquence d'acides aminés codée par les nucléotides numéros 439 à 1008 de la figure 14, et une région variable.

43. Procédé selon l'une quelconque des revendications 40, 41 ou 42, dans lequel la cellule est une cellule T.

44. Procédé selon l'une des revendications 40 ou 41, dans lequel l'acide nucléique codant pour au moins une portion du polypeptide récepteur d'antigènes de cellules T gamma est choisi dans le groupe consistant en (i) un acide nucléique comprenant un unique exon CII; (ii) un acide nucléique comprenant deux exons CII; et (iii) un acide nucléique comprenant trois exons CII.

45. Procédé selon l'une des revendications 40 ou 41, dans lequel la portion du polypeptide récepteur d'antigènes de cellules T gamma et la portion du polypeptide récepteur d'antigènes de cellules To ne sont pas associées par covalence.

46. Procédé selon l'une des revendications 40 ou 41, dans lequel la portion du polypeptide récepteur d'antigènes de cellules T gamma et la portion du polypeptide récepteur d'antigènes de cellules To sont associées par covalence.

47. Cellules isolées qui expriment au moins une portion d'un hétérodimère de récepteur d'antigènes de cellules T gamma,delta, laquelle portion d'un récepteur d'antigènes de cellules T gamma,delta comprend au moins une région d'un polypeptide récepteur d'antigènes de cellules T gamma choisie dans le groupe consistant en une région constante, une région variable et une région de jonction, associée avec au moins une région d'un polypeptide récepteur d'antigènes de cellules Tδ choisie dans le groupe consistant en une région constante, une région variable, une région de jonction et une région de diversité, ledit hétérodimère n'étant pas associé avec un complexe CD3.

48. Cellules selon la revendication 47, dans lesquelles les cellules sont des cellules épithéliales glandulaires endométriales.

49. Celle transfectée, qui exprime un hétérodimère de récepteur d'antigènes de cellules T gamma,delta, dans lequel le polypeptide récepteur d'antigènes de cellules T gamma de l'hétérodimère est produit par expression d'un acide nucléique codant pour le polypeptide récepteur d'antigènes de cellules T gamma qui a été transfecté dans la cellule.

50. Cellule selon la revendication 49, dans laquelle le polypeptide récepteur d'antigènes de cellules T gamma est la Forme 2bc ayant une masse moléculaire d'environ 40.000 daltons et une séquence comprenant une région constante consistant essentiellement en la séquence d'acides aminés codéée par les nucléotides numéros 439 à 1008 de la figure 14, et une région variable.

51. Cellule transfectée, qui exprime au moins une portion d'un hétérodimère récepteur d'antigènes de cellules T gamma,delta, laquelle portion d'un récepteur d'antigènes de cellules T gamma,delta comprend au moins une région d'un polypeptide récepteur d'antigènes de cellules T gamma choisie dans le groupe consistant en une région constante, une région variable et une région de jonction, associée avec au moins une région d'un polypeptide récepteur d'antigènes de cellules To choisie dans le groupe consistant en une région constante, une région variable, une région de jonction et une région de diversité; l'hétérodimère n'étant pas associé avec un complexe CD3, la portion du polypeptide récepteur d'antigènes de cellules T gamma étant produite par expression d'un acide nucléique codant pour la portion du récepteur d'antigènes de cellules T gamma qui a été transfecté dans la cellule.

52. Procédé pour la détermination de l'utilisation relative d'un polypeptide récepteur d'antigènes de cellules T gamma dans un échantillon provenant d'un sujet humain, comprenant:
(a) la détermination dans un échantillon contenant des cellules T provenant d'un sujet de la quantité d'un polypeptide récepteur d'antigènes de cellules T gamma de Forme 2bc;
(b) la détermination dans un échantillon provenant d'un sujet de la quantité d'un polypeptide récepteur d'antigènes de cellules T gamma choisi dans le groupe consistant en un polypeptide gamma de Forme 1 et un polypeptide gamma de Forme 2abc; et
(c) la comparaison de la quantité déterminée dans l'étape (a) avec la quantité déterminée dans l'étape
(b), déterminant ainsi l'utilisation relative.
